(19) Europäisches Patentamt
European Patent Office
Office européen des brevets

(11) Veröffentlichungsnummer: **0 025 495**
**B1**

# (12) EUROPÄISCHE PATENTSCHRIFT

(45) Veröffentlichungstag der Patentschrift: **20.06.84**

(51) Int. Cl.³: **C 07 H 15/04**, A 61 K 31/70

(21) Anmeldenummer: **80104395.1**

(22) Anmeldetag: **25.07.80**

(54) **Lipophile Muramylpeptide, Verfahren zu deren Herstellung und deren Verwendung.**

(30) Priorität: **25.07.79 CH 6893/79**

(43) Veröffentlichungstag der Anmeldung: **25.03.81 Patentblatt 81/12**

(45) Bekanntmachung des Hinweises auf die Patenterteilung: **20.06.84 Patentblatt 84/25**

(84) Benannte Vertragsstaaten: **AT BE CH DE FR GB IT LI LU NL SE**

(56) Entgegenhaltungen: **FR - A - 2 361 902**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Postfach, CH-4002 Basel (CH)**

(72) Erfinder: **Tarcsay, Lajos, Dr., Muttenzerstrasse 25, D-7889 Grenzach-Wyhlen (DE)**
Erfinder: **Baschang, Gerhard, Dr., Bückenweg 7, CH-4126 Bettingen (CH)**
Erfinder: **Hartmann, Albert, Dr., Steingasse 21A, D-7889 Grenzach (DE)**
Erfinder: **Stanek, Jaroslav, Dr., Florastrasse 6, CH-4127 Birsfelden (CH)**

(74) Vertreter: **Zumstein, Fritz sen., Dr. et al, Bräuhausstrasse 4, D-8000 München 2 (DE)**

**EP 0 025 495 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung sind neue lipophile Muramylpeptide, ferner Verfahren zu deren Herstellung, sowie pharmazeutische Präparate, die diese lipophilen Muramylpeptide enthalten, wie auch deren Verwendung zu Stimulierung der Immunität.

Die Erfindung betrifft insbesondere Verbindungen der Formel

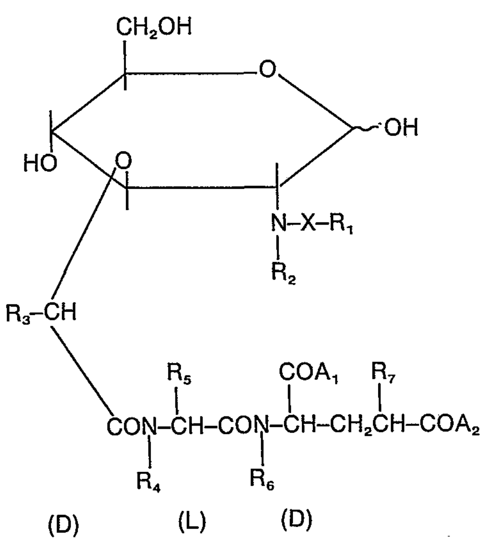

(I)

worin X Carbonyl oder Carbonyloxy, $R_1$ gegebenenfalls substituiertes Alkyl oder Aryl, $R_2$, $R_4$ und $R_6$ Wasserstoff oder Niederalkyl, $R_3$ Wasserstoff oder Niederalkyl, $R_5$ Wasserstoff, Niederalkyl, freies oder funktionell abgewandeltes Hydroxyniederalkyl, freies oder funktionell abgewandeltes Mercaptoniederalkyl, gegebenenfalls substituiertes Aminoniederalkyl, Cycloalkyl, Cycloalkylniederalkyl, gegebenenfalls substituiertes Aryl oder Aralkyl, stickstoffhaltiges Heterocyclyl oder Heterocyclyl-niederalkyl, oder $R_4$ und $R_5$ zusammen auch Alkylen mit 3–4 Kohlenstoffatomen, $R_7$ Wasserstoff oder gegebenenfalls verestertes oder amidiertes Carboxyl und einer der Reste $A_1$ und $A_2$ einen Rest der Formel

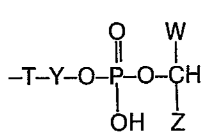

(II)

bedeutet, worin T für NH oder O steht, Y eine gegebenenfalls substituierte Alkylengruppe, die auch durch ein oder zwei Oxycarbonyl und/oder Iminocarbonyl unterbrochen sein kann, W Wasserstoff und Z eine 1,2-Dihydroxyäthyl- oder 2-Hydroxyäthylgruppe, in denen mindestens eine der Hydroxygruppen mit einer gegebenenfalls ungesättigten, aliphatischen Carbonsäure mit 12–90 C-Atomen verestert oder mit einem gegebenenfalls ungesättigten aliphatischen Alkohol mit 10–22 C-Atomen veräthert ist, oder W und Z je eine mit einer gegebenenfalls ungesättigten aliphatischen Carbonsäure mit 12–90 C-Atomen veresterte oder mit einem gegebenenfalls ungesättigten aliphatischen Alkohol mit 10–22 C-Atomen verätherte Hydroxymethylgruppe darstellen, und der andere der Reste $A_1$ und $A_2$ freies oder veräthertes Hydroxy, Amino, Niederalkylamino oder Carboxamidoniederalkyl-amino ist, und ihre Salze.

Alkyl ist geradkettiges oder verzweigtes, in beliebiger Stellung gebundenes Alkyl mit bis zu 18 Kohlenstoffatomen, in erster Linie jedoch Niederalkyl.

Als Substituenten der gegebenenfalls substituierten Alkylgruppen kommen in erster Linie freie oder funktionell abgewandelte Hydroxy- oder Mercaptogruppen, wie verätherte oder veresterte Hydroxy- oder Mercaptogruppen, z.B. Niederalkoxy- oder Niederalkylmercaptogruppen, oder Halogenatome oder freie oder funktionell abgewandelte Carboxy-, wie Carbo-niederalkoxy- oder Carbamoylgruppen in Frage. Dabei kann der substituierte Alkylrest, wie Niederalkylrest, einen, zwei oder mehrere gleiche oder verschiedene Substituenten, insbesondere freie Hydroxylgruppen oder Halogenatome tragen.

Arylreste sind insbesondere monocyclische, sowie bicyclische Arylreste, in erster Linie Phenyl, aber auch Naphthyl. Sie können gegebenenfalls, z.B. durch Niederalkylgruppen, freies, verestertes oder veräthertes Hydroxy, z.B. Niederalkoxy oder Niederalkylendioxy oder Halogenatome, und/oder Trifluoromethylgruppen, mono-, di- oder polysubstituiert sein.

Aralkyl ist insbesondere Aryl-niederalkyl, worin Aryl die oben gegebene Bedeutung hat. In erster Linie steht Arylniederalkyl für Benzyl oder Phenyläthyl, worin der Phenylkern mono-, di- oder polysubstituiert sein kann.

Gegebenenfalls substituierte Aralkylreste sind insbesondere solche Reste, die im aromatischen Kern gegebenenfalls, z.B. durch Niederalkyl, freie, verätherte oder veresterte Hydroxy- oder Mercaptogruppen, z.B. Niederalkoxy oder Niederalkylendioxy, sowie Niederalkylmercapto- oder Trifluormethylgruppen und/oder Halogenatome, mono-, di- oder polysubstituiert sind.

Cycloalkyl ist insbesondere Cycloalkyl mit 5 bis 6 Kohlenstoffatomen, wie Cyclopentyl oder Cyclohexyl, und Cycloalkyl-niederalkyl insbesondere ein solcher, worin der Cycloalkylrest 5 bis 6 Kohlenstoffatome besitzt und der Niederalkylrest in erster Linie Methyl oder Äthyl darstellt.

Stickstoffhaltiges Heterocyclyl ist insbesondere der Rest einer 5- oder 6gliedrigen, ein oder zwei Stickstoffatome im Ring enthaltenden heterocyclischen Verbindung. Er kann ungesättigt oder auch gesättigt sein, und z.B. einen ankondensierten Phenylrest enthalten. Als solche seien z.B. die Pyrrolyl-, Indolyl-, Pyridyl- oder Imidazolylreste genannt.

Im stickstoffhaltigen Heterocyclyl-niederalkyl hat der Heterocyclyl-rest die oben genannte Bedeutung und der Niederalkylrest ist in erster Linie Methyl oder Äthyl.

Der Alkylenrest, der von den Resten $R_4$ und $R_5$ gebildet sein kann, ist vorzugsweise unsubstituiert und in erster Linie der Trimethylenrest.

Eine gegebenenfalls veresterte oder amidierte Carboxylgruppe ist in erster Linie die Carboxylgruppe selbst, oder eine mit einem Niederalkanol veresterte Carboxylgruppe oder auch die Carbamoylgruppe, die am Stickstoffatom unsubstituiert, oder mit Alkyl, insbesondere Niederalkyl, Aryl, in erster Linie Phenyl, oder Aralkyl, wie Benzyl, mono- oder di-substituiert ist. Die Carbamoylgruppen kann aber auch einen Alkylen-, wie Tetraoder Pentamethylenrest tragen.

Als gegebenenfalls funktionell abgewandelte Hydroxy- oder Mercaptogruppen sind insbesondere verätherte oder veresterte Hydroxy- oder Mercaptogruppen zu nennen, wie Niederalkoxy, Niederacyloxy, z.B. Niederalkanoyloxy, oder Halogenatome, Niederalkylmercapto oder Niederacylmercapto, z.B. Niederalkanoylmercapto.

Als substituiertes Aminoniederalkyl sind insbesondere Mono--oder Diniederalkylamino-niederalkyl oder acyliertes Aminoniederalkyl, wie Methylamino-, Äthylamino-, Dimethylamino-, Diäthylamino-, Alkanoylamino-, z.B. Niederalkanoylamino-niederalkyl zu nennen.

Carboxamidoniederalkylamino ist in erster Linie 1-Carboxamido-niederalkylamino, z.B. Aminocarbonyl-methylamino. (L)-1-Aminocarbonyläthylamino, (L)-1-Aminocarbonyl-2-methyl-propylamino oder (L)-1-Aminocarbonyl-2-methylbutylamino.

Der Alkylenrest Y ist insbesondere ein Niederalkylenrest, vorzugsweise mit 2 oder 3 Kohlenstoffatomen. Der Alkylenrest Y kann aber auch ein durch oder einen Rest wie Oxycarbonyl oder N–$R_8$-iminocarbonyl unterbrochener Niederalkylenrest sein, und stellt dann insbesondere einen Rest der Formel

$$-Y_1-COO-Y_2- \qquad (IIIa)$$
$$-Y_1-OOC-Y_2- \qquad (IIIb)$$
$$-Y_1-CO\underset{R_8}{\underset{|}{N}}-Y_2- \qquad (IIIc)$$
$$\text{bzw. } -Y_1-\underset{R_8}{\underset{|}{N}}OC-Y_2- \qquad (IIId)$$

dar, worin einer der Reste $Y_1$ und $Y_2$ ein gegebenenfalls substituierter Niederalkylenrest und der andere ein gegebenenfalls substituierter Niederalkylenrest, der auch durch Oxycarbonyl oder N–$R_8$-Iminocarbonyl unterbrochen sein kann, und $Y_1$ und $Y_2$ zusammen mehr als zwei Kohlenstoffatome aufweisen, und $R_8$ Wasserstoff oder Niederalkyl sind. Als Substituenten der Reste $Y_1$ und $Y_2$ sollen insbesondere freies oder funktionell abgewandeltes Hydroxy oder Hydroxyniederalkyl, freies oder funktionell abgewandeltes Mercapto oder Mercaptoniederalkyl, freies oder mono- oder diniederalkyliertes oder acyliertes Amino-niederalkyl, Carboxamido, Alkyl, Cycloalkyl mit 5 bis 6 Kohlenstoffatomen, Aryl oder Aralkyl genannt werden, wobei die Allgemeinbegriffe die oben angegebene Bedeutung haben können.

Eine aliphatische Carbonsäure mit 12 bis 90 Kohlenstoffatomen kann auch 1 oder 2 Doppelbindungen aufweisen und gerade oder verzweigt sein. Bevorzugt sind solche mit 16 bis 22 Kohlenstoffatomen oder natürliche oder synthetische Mycolsäuren.

Ein aliphatischer Alkohol hat 10–22 Kohlenstoffatome und ist insbesondere ein Alkanol mit 10–22 Kohlenstoffatomen, das auch eine oder zwei Doppelbindungen aufweisen und gerade oder verzweigt sein kann.

Bevorzugt sind solche Alkanole, die 12–18 Kohlenstoffatome enthalten und deren Hydroxygruppe endständig ist.

Die im Zusammenhang mit der vorliegenden Beschreibung und den Patentansprüchen mit «Nieder» bezeichneten Reste und Verbindungen enthalten bis und mit 7 und in erster Linie bis und mit 4 Kohlenstoffatome.

Vorstehend wie nachfolgend können die Allgemeinbegriffe folgende Bedeutung haben:

Niederalkyl ist z.B. n-Propyl, n-Butyl, Isobutyl, sek.-Butyl oder tert.-Butyl, ferner n-Pentyl, n-Hexyl, Isohexyl oder n-Heptyl und in erster Linie Methyl oder Äthyl. In Aryl-, Cycloalkyl- oder Heterocyclylniederalkyl ist der Niederalkylrest insbesondere Methyl oder Äthyl, wobei der Aryl-, Cycloalkyl- oder Heterocyclylrest die obgenannte Bedeutung besitzt.

Niederalkoxy ist z.B. n-Propoxy, n-Butoxy, Isobutoxy, sek.-Butoxy oder tert.-Butoxy und in erster Linie Methoxy oder Äthoxy.

Niederalkylmercapto ist z.B. n-Propyl-, n-Butyl-, Isobutyl, sek.-Butyl oder tert.-Butylmercapto und in erster Linie Methylmercapto oder Äthylmercapto.

Niederalkylendioxy ist insbesondere Methylendioxy, Äthylen- oder Propylendioxy.

Halogen steht für Fluor oder Brom, vorzugsweise jedoch für Chlor.

Niederalkanoyl ist insbesondere Propionyl oder Butyryl, in erster Linie jedoch Acetyl.

Synthetische Mycolsäuren sind insbesondere α-Alkyl-β-hydroxy-alkancarbonsäuren, worin der Alkylrest in α-Stellung 1–20, in erster Linie 1–14 und die Alkancarbonsäure 20–80, insbesondere 30–34 Kohlenstoffatomen enthält. Sie können auch weitere Hydroxylgruppen, sowie Oxo-, Methylen- oder Äthylengruppen enthalten.

Natürliche Mycolsäuren sind insbesondere solche, wie sie aus lebenden Organismen, wie Bakterien, z.B. Mycobacterien, isoliert werden können.

Die neuen Verbindungen der vorliegenden Erfindung können in Form von Gemischen von Isomeren oder von reinen Isomeren vorliegen.

Die neuen lipophilen Muramylpeptide der vorliegenden Erfindung weisen eine Reihe wertvoller pharmakologischer Eigenschaften, insbesondere eine ausgeprägte immunpotenzierende Wirkung auf.

So wird durch diese Verbindungen in vivo die Antikörperbildungsfähigkeit von Mäusen erheblich gesteigert:

NMRI Mäusen werden durch intraperitoneale Injektion von 10 µg präzipitatfreiem BSA (Rinderserumalbumin) am Tag 0 immunisiert. 9, 15 und 29 Tage später werden Serumproben entnommen und auf ihren Gehalt an anti-BSA-Antikörpern mit einer passiven Haemagglutinationstechnik untersucht. In der verwendeten Dosis ist lösliches BSA

für die Empfängertiere subimmunogen, d.h. es vermag keine oder nur eine ganz geringfügige Produktion von Antikörpern auszulösen. Zusätzliche Behandlung der Mäuse mit gewissen immunpotenzierenden Stoffen vor oder nach der Antigengabe führt zu einem Anstieg der Antikörpertiter im Serum. Der Effekt der Behandlung wird durch den erreichten Scorewert, d.h. durch die Summe der $\log_2$ Titerdifferenzen an den drei Blutungstagen ausgedrückt.

In diesem Test sind die Verbindungen der Formel (I) in der Lage, bei intraperitonealer oder subkutaner Applikation von 0,5–5 mg/kg Tier an fünf aufeinander folgenden Tagen nach Immunisierung mit BSA die Antikörperproduktion gegen BSA signifikant zu steigern. Sie sind diesbezüglich den herkömmlichen hydrophilen Muramylpeptiden hoch überlegen.

Auch Manifestationen der zellvermittelten Immunität können durch die genannten Verbindungen in vivo potenziert werden:

Während Sensibilisierung von Meerschweinchen mit BSA in inkomplettem Freund'schem Adjuvans nur zu humoraler Antikörperbildung führt, induziert die Beimischung der erfindungsgemässen lipophilen Muramylpeptide in einem Dosisbereich von 5–50 µg zur Antigen-Ölemulsion Spättyp-Überempfindlichkeit gegenüber BSA: 3 Wochen nach Immunisierung führt die intrakutane Injektion von BSA bei diesen Tieren zu einer lokalen Entzündungsreaktion mit Erythem und Verdickung der Haut, die innert 24 bis 48 Stunden ihr Maximum erreicht. Diese Spättyp-Reaktionen entsprechen quantitativ und qualitativ denjenigen, die üblicherweise durch Immunisierung mit BSA in komplettem Freund'schem Adjuvans (d.h. mit Zusatz von Mykobakterien) erhalten werden. Die $ED_{50}$-Werte (benötigte µg/Tier zur Induktion einer Differenz des Reaktionsvolumens (Erythemfläche × Hautdickenzunahme) bei behandelten und unbehandelten Tieren von 200 µl, 24 Stunden nach Auslösung) betragen 10–20 µg.

Besonders hervorzuheben ist auch die Fähigkeit solcher lipophiler Muramylpeptide, durch Applikation zusammen mit BSA in Liposomen (Eilecithin : Cholesterin 4 : 1; 4 mg/Tier) und ohne die toxische Mineralöl-Komponente, in Meerschweinchen eine Spättypüberempfindlichkeit gegen BSA zu induzieren. Quantitativ und qualitativ sind diese Spättyp-Reaktionen ebenfalls identisch mit denjenigen, die durch Immunisierung mit BSA in komplettem Freund'schem Adjuvans erhalten werden. Die $ED_{50}$-Werte betragen 100–300 µg pro Tier.

Die neuen Verbindungen der Formel (I) zeigen im Vergleich zu hydrophilen Muramyldipeptiden weitere zusätzliche Qualtitäsverbesserungen:

Balb/cMäuse werden durch intraperitoneale Injektion von $2 \times 10^4$ P815 Mastocytomzellen am Tag 0 immunisiert. Am Tag 15 werden die Milzzellen der so immunisierten Tiere in vitro auf das Vorhandensein zytotoxischer, gegen P815 Mastocytom-Zellen gerichteter T-Lymphocyten untersucht. Dazu werden die P815 Zielzellen mit $^{51}Cr$ markiert und das Ausmass der zytotoxischen Reaktion durch Messen der Radioaktivität im Kulturüberstand ermittelt. In der verwendeten Dosis sind die P815 Mastocytomzellen für die Empfänger-Mäuse subimmunogen, d.h. sie induzieren keine oder nur ganz geringe Bildung zytotoxischer T-Zellen. Gleichzeitige intraperitoneale Applikation von 1 bis 50 µg der genanten Muramylpeptide der Formel I führen zu einer signifikanten Steigerung der Bildung zytotoxischer T-Zellen (Faktor 10–30 gegenüber unbehandelten Mäusen).

Die immunpotenzierenden Eigenschaften der neuen Verbindungen der Formel (I) lassen sich auch im Falle der Induktion spezifischer Immuntoleranz gegen Transplantationsantigene durch Immunisierung mit adjuvierten Autoblasten bei der Maus darstellen:

In einer gemischten Lymphocytenkultur werden Milzlymphocyten des zukünftigen Transplantat-Empfängers (C57B1/6J Mäuse) mit bestrahlten Milzzellen des zukünftigen Transplantat-Spenders (CBA/J Mäuse) inkubiert. T-Lymphocyten mit spezifischen Rezeptoren für die Histokompatibilitätsantigene des Spenders proliferieren und werden zu Blasten; diese können durch Sedimentation von den andern Zellen abgetrennt werden. Die spezifischen Blasten exprimieren die relevanten idiotypischen Spezifitäten der Membranrezeptoren und werden adjuviert mit komplettem Freund'schem Adjuvans (CFA) als Autoimmunogene zur Induktion spezifischer Toleranz gegen die betreffenden Transplantationsantigene in die prospektiven Transplantat-Empfänger (C 57 B1/6J) injiziert. Die Immunisierung erfolgt viermal in vierwöchigen Abständen mit autologen anti-CBA/J T Lymphoblasten. Adsorbate von T-Autoblasten mit den neuen Verbindungen der Formel (I) ($10^9$ Blasten werden in einer Lösung von 20 mg Substanz in 20 ml PBS suspendiert. Nach zweistündiger Inkubation werden die Zellen zentrifugiert und zweimal mit PBS gewaschen) sind in der Lage, spezifische Immuntoleranz in Abwesenheit von CFA zu induzieren, wobei die Adsorbate gleich wirksam sind wie Lymphoblasten in CFA.

Die neuen Verbindungen der Formel (I) sind ausserdem in der Lage, in Konzentrationen von 0,5–100 µg/ml in Milzzellkulturen von normalen Mäusen die Bildung antikörperproduzierender Zellen zu induzieren (Vermehrung der 19S-plaquebildenden Zellen um einen Faktor 10–30 über den Kontrollwert (in Abwesenheit der stimulierenden Substanzen): So werden in Anwesenheit der genannten Verbindungen z.B. spezifische Antikörper gegen Schaferythrocyten gebildet, ohne dass den Kulturen Schaferythrocyten zur Immunisierung zugesetzt werden. Anderseits vermögen die genannten Substanzen im selben Konzentrationsbereich auch die immunologische Reaktivität von T-zellverarmten Milzzellkulturen (von kongential athymischen nu/nu Mäusen) gegenüber einem normalerweise thymusabhängigen Antigen (Schaferythrocyten) zu steigern (Faktor 10–30 gegenüber unbehandelten Kontrollkulturen). Durch die genannten Verbindungen werden aber in vitro direkt oder indirekt nicht nur Proliferations- und

Syntheseleistungen von B-Lymphocyten (d.h. von potentiell antikörperbildenden Zellen) induziert, sondern auch Effekte auf T-Lymphocyten (zu denen regulatorisch aktive Helfer- und Suppressorzellen sowie cytotoxische Effektorzellen gehören), vermittelt. So vermögen z.B. die erwähnten Verbindungen in einem Konzentrationsbereich von 1–20 µg/ml die Reaktivität von cortisonresistenten Thymuszellen gegenüber allogenen bestrahlten Stimulatorlymphocyten erheblich (bis zu 10fach) zu potenzieren.

Die oben erwähnten Wirkungen kommen wahrscheinlich indirekt dadurch zustande, dass solche lipophilen Muramylpeptide Makrophagen aktivieren, die ihrerseits die Reaktivität von T- und B-Lymphocyten fördern. Tatsächlich kann man zeigen, dass die genannten Verbindungen bereits in geringen Konzentrationen (0,5–10 µg/ml) grosse Mengen «colony stimulating activity» (CSA) aus Maus-Makrophagen freisetzen (Induktion von bis zu 150–200 Kolonien innert 7 Tagen aus $10^5$ Mäuse-Knochenmarkzellen, nach Zugabe von 20% Überstand aus während 24 Stunden mit Substanz inkubierten Makrophagenkulturen, im Vergleich zu 0–5 Kolonien bei Zugabe von Überständen unbehandelter Makrophagenkulturen). CSA ist ein biologischer Mediator, der für die Differenzierung von Knochenmark-Stammzellen zu Makrophagen und polymorphkernigen Leucocyten notwendig ist. Damit bewirken die genannten Verbindungen einen erhöhten Nachschub von Zellen, die für die unspezifische Resistenz und für die Induktion, Amplifikation und Expression spezifischer (lymphocytenvermittelter) Immunreaktionen von zentraler Bedeutung sind.

Die immunpotenzierende Wirkung der neuen Verbindungen kann in vivo nachgewiesen werden: So führt die Injektion eines Phospholipid-Derivates von Muramylpeptid gemäss der Erfindung innert 3–9 Stunden zu einem hohen Anstieg der CSA-Konzentration im Serum (bis zu 120 Kolonien pro $10^5$ Mäuseknochenmarkzellen nach Zugabe von Chloroform extrahiertem Serum [5% Endkonzentration] im Vergleich zu 0–5 Kolonien bei unbehandelten Tieren). Dementsprechend wird durch Verabreichung derselben Verbindungen in vivo die Antikörperbildungsfähigkeit von Mäusen erheblich potenziert.

Die immunpotenzierenden Eigenschaften der neuen Verbindungen der Formel I lassen sich auch in Tumormodellen demonstrieren, so z.B. beim Ehrlich Asictes Tumor in der Maus.

Eine intraperitoneale Injektion von $10^6$ syngenen Ehrlich Ascites Tumorzellen führt bei Balb/c Mäusen durchschnittlich in 18 Tagen zum Absterben der Tiere. Injiziert man den Mäusen intraperitoneal $10^7$ (Gruppe 1). $10^6$ (Gruppe 2) und $10^5$ (Gruppe 3) Ascites Tumorzellen, die man in vitro mit den neuen Verbindungen der Formel I beladen hat ($10^9$ Ascites Tumorzellen werden in einer Lösung von 40 mg der Prüfsubstanz in 20 ml phosphat-gepufferter physiologischer Kochsalzlösung (PBS) suspendiert und nach zweistündiger Inkubation bei 37 °C werden die Zellen zentrifungiert und zweimal mit PBS gewaschen; die Zellen inkosporieren bei dieser Behandlung die Prüfverbindung in ihre Membran), so kommt es in 18 Tagen zu keinem Tumorwachstum. Am 19. Tag werden die Tiere jeweils mit $10^6$ nativen Ehrlich Ascites Tumorzellen intraperitoneal belastet. Folgende Effekte werden beobachtet:

Gruppe 1: 8 von 10 Tieren überleben den 80. Tag

Gruppe 2: 6 von 10 Tieren überleben den 80. Tag

Gruppe 3 : die Tiere sterben, wie die Kontrolltiere, nach 18 Tagen.

Die Verbindungen gemäss der vorliegenden Erfindung sind zudem wenig toxisch: Auch 5malige intraperitoneale Applikation in einer Dosis von 100 mg/kg/Tag an fünf aufeinanderfolgenden Tagen wurde von Mäusen anscheinend symptomlos vertragen. Da die für die Immunstimulation benötigten Dosen sehr gering sind, ist die therapeutische Breite der neuen Verbindung sehr gross.

Die neuen Verbindungen gemäss der vorliegenden Erfindung können somit die zelluläre und besonders die humorale Immunität erheblich steigern, und zwar sowohl in Mischung mit dem Antigen selber (Adjuvanseffekt im engeren Sinne) als auch bei zeitlich und örtlich von der Antigeninjektion getrennter Zufuhr (systemische Immunpotenzierung).

Die neuen Verbindungen gemäss der vorliegenden Erfindung können somit als Adjuvantien in Mischung mit Impfstoffen dazu benützt werden, den Impferfolg zu verbessern und den durch humorale Antikörper und/oder zelluläre Immunität vermittelten Infektionsschutz gegenüber bakteriellen, viralen oder parasitären Erregern zu verbessern.

Schliesslich eignen sich die beschriebenen Verbindungen in Mischung mit verschiedenen Antigenen als Adjuvantien bei der experimentellen und industriellen Herstellung von Antiseren für Therapie und Diagnostik und bei der Induktion von immunologisch aktivierten Lymphocyten-populationen für Zelltransferverfahren.

Darüberhinaus können die neuen Verbindungen auch ohne gleichzeitige Antigenzufuhr dazu benützt werden, bereits unterschwellig ablaufende Immunreaktionen bei Mensch und Tier zu fördern. Die Verbindungen eignen sich demnach besonders für die Stimulation der körperlichen Abwehr, z.B. bei chronischen und akuten Infektionen oder bei selektiven (antigenspezifischen) immunologischen Defekten, sowie bei angeborenen, aber auch bei erworbenen allgemeinen (d.h. nicht antigenspezifischen) immunologischen Defektzuständen, wie sie im Alter, im Verlauf schwerer Primärerkrankungen und vor allem nach Therapie mit ionisierenden Strahlen oder mit immunosuppressiv wirkenden Hormonen auftreten. Die genannten Stoffe können somit vorzugsweise auch in Kombination mit Antibiotika, Chemotherapeutika oder anderen Heilverfahren verabreicht werden, um immunologischen Schädigungen entgegenzuwirken. Schliesslich sind die beschriebenen Stoffe auch zur allgemeinen Prophylaxe von Infektionskrankheiten bei Mensch und Tier geeignet.

Die Erfindung betrifft besonders Verbindungen der Formel I, worin X Carbonyl, $R_1$ gegebenenfalls substituiertes Alkyl oder Aryl, $R_2$, $R_3$, $R_4$ und $R_6$ Wasserstoff oder Niederalkyl, $R_5$ Wasserstoff, gegebenenfalls durch Hydroxy, Niederalkoxy, Mercapto oder Niederalkylmercapto oder Halogen substituiertes Niederalkyl, Cycloalkyl oder Cycloalkylniederalkyl, worin der Cycloalkylrest 4–6 Kohlenstoffatome enthält, gegebenenfalls substituiertes Phenyl oder Phenylniederalkyl, ein oder zwei Azaatome enthaltendes Heterocyclyl oder Heterocyclylniederalkyl oder $R_4$ und $R_5$ zusammen auch Alkylen mit 3–4 Kohlenstoffatomen, $R_7$ Wasserstoff und einer der Reste $A_1$ und $A_2$ einen Rest der Formel

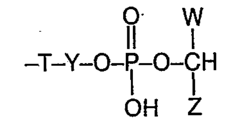

(II)

bedeuten, worin T für NH oder O steht, Y gegebenenfalls substituiertes Alkylen, das auch durch Carbonyloxy oder Carbonylimino unterbrochen sein kann, W Wasserstoff und Z eine 1,2-Dihydroxyäthyl- oder 2-Hydroxyäthylgruppe darstellen, in der mindestens eine der Hydroxygruppen in der oben angegebenen Weise verestert oder veräthert ist, und der andere der Reste $A_1$ und $A_2$ Hydroxy, Niederalkoxy, Amino, Niederalkylamino oder Carboxamidoniederalkylamino ist, und ihre Salze.

Die Erfindung betrifft insbesondere Verbindungen der Formel I, worin X Carbonyl, $R_1$ gegebenenfalls durch Hydroxy, Niederalkoxy oder Halogen substituiertes Niederalkyl oder gegebenenfalls durch Hydroxy, Niederalkoxy, Niederalkyl oder Halogen substituiertes Phenyl, $R_2$, $R_4$ und $R_6$ Wasserstoff, $R_3$ Wasserstoff oder Niederalkyl, $R_5$ Wasserstoff, gegebenenfalls durch Hydroxy, Niederalkoxy, Mercapto, Niederalkylmercapto oder Halogen substituiertes Niederalkyl mit 1–3 Kohlenstoffatomen, Cycloalkyl oder Cycloalkylniederalkyl, worin der Cycloalkylrest 4–6 Kohlenstoffatome und der Niederalkylrest 1–3 Kohlenstoffatome enthält, gegebenenfalls durch Hydroxy, Niederalkoxy oder Halogen substituiertes Phenyl oder Phenylniederalkyl mit 1–3 Kohlenstoffatomen im Niederalkylrest, ein oder zwei Azaatome enthaltendes Heterocyclyl oder Heterocyclylniederalkyl mit 5–6 Ringgliedern und 1–3 Kohlenstoffatomen im Niederalkylrest oder $R_4$ und $R_5$ zusammen auch Alkylen mit 3–4 Kohlenstoffatomen, $R_7$ Wasserstoff, und einer der Reste $A_1$ und $A_2$ einen Rest der Formel

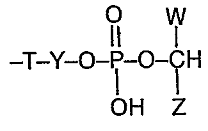

(II)

bedeutet, worin T für HN oder O steht, Y gegebenenfalls substituiertes Niederalkyl oder einen Rest der Formel

$$-Y_1-COO-Y_2- \qquad (IIIa)$$

oder

$$-Y_1-CONH-Y_2- \qquad (IVc)$$

bedeutet, worin $Y_1$ und $Y_2$ je gegebenenfalls substituiertes Niederalkylen sind, W Wasserstoff und Z eine 1,2-Dihydroxyäthyl- oder 2-Hydroxyäthylgruppe, in der mindestens eine der Hydroxygruppen mit einer gegebenenfalls ein- oder zweifach ungesättigten aliphatischen Carbonsäure mit 16–22 Kohlenstoffatomen oder mit einer natürlichen oder synthetischen Mycolsäure verestert oder mit einem gegebenenfalls ein- oder zweifach ungesättigten, aliphatischen Alkohol mit 12–18 Kohlenstoffatomen veräthert ist, darstellen, und der andere der Reste $A_1$ und $A_2$ Hydroxy, Niederalkoxy, Amino, Niederalkylamino oder Carboxamidoniederalkylamino ist, und ihre Salze.

Die Erfindung betrifft in erster Linie Verbindungen der Formel I, worin X Carbonyl, $R_1$ Niederalkyl mit 1–3 Kohlenstoffatomen oder Phenyl, $R_2$, $R_4$ und $R_6$ Wasserstoff, $R_3$ Wasserstoff oder Niederalkyl mit 1–3 Kohlenstoffatomen, $R_5$ Wasserstoff, gegebenenfalls durch Hydroxy, Methoxy, Mercapto, Methylmercapto oder Halogen substituiertes Niederalkyl mit 1–3 Kohlenstoffatomen, gegebenenfalls durch Hydroxy, Methoxy oder Halogen substituiertes Phenyl oder Phenylmethyl, ein oder zwei Azaatome enthaltendes Heterocyclyl oder Heterocyclylmethyl mit 5 Ringgliedern oder $R_4$ und $R_5$ zusammen auch Trimethylen, $R_7$ Wasserstoff und einer der Reste $A_1$ und $A_2$ einen Rest der Formel

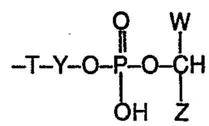

(II)

bedeuten, worin T für HN oder O steht, Y Niederalkylen mit 2–3 Kohlenstoffatomen oder einen Rest der Formel (IIIa) oder (IVc) bedeutet, worin $Y_1$ und $Y_2$ unabhängig voneinander je Niederalkylen mit 1–3 Kohlenstoffatomen bedeuten, das unsubstituiert oder durch gegebenenfalls durch Hydroxy, Niederalkoxy, Mercapto oder Niederalkylmercapto substituiertes Niederalkyl mit 1–3 Kohlenstoffatomen oder durch gegebenenfalls durch Hydroxy, Methoxy oder Halogen substituiertes Phenyl oder Phenylniederalkyl oder durch ein oder zwei Azaatome enthaltendes Heterocyclyl oder Heterocyclylniederalkyl mit 5–6 Ringgliedern und 1–3 Kohlenstoffatomen im Niederalkylrest substituiert ist, W Wasserstoff und Z eine 1,2-Dihydroxyäthyl- oder 2-Hydroxyäthylgruppe ist, in der mindestens eine der Hydroxygruppen mit einer gegebenenfalls ein- oder zweifach ungesättigten aliphatischen Carbonsäure mit 16–20 Kohlenstoffatomen verestert oder mit einem gegebenenfalls ein- oder zweifach ungesättigten aliphatischen Alkohol mit 12–18 Kohlenstoffatomen veräthert ist, und der andere der Reste $A_1$ und $A_2$ Hydroxy, Niederalkoxy, Amino, Niederalkylamino oder Carboxamidoniederalkylamino ist, und ihre Salze.

In erster Linie betrifft die Erfindung Muramylpeptide der Formel I, worin X Carbonyl oder Carbonyloxy, $R_1$ Niederalkyl mit 1–3 Kohlenstoffatomen oder Phenyl, $R_2$, $R_4$ und $R_6$ Wasserstoff, $R^3$ Wasserstoff oder Niederalkyl mit 1–3 Kohlenstoffatomen, $R^5$ Wasserstoff oder Niederalkyl, $R^7$ Was-

serstoff, $A_1$ Amino, Niederalkylamino, Hydroxy oder Niederalkoxy und $A_2$ einen Rest der in Anspruch 1 dargestellten Formel (II), worin T für NH oder O steht, Y Niederalkylen mit 2–3 Kohlenstoffatomen oder einen Rest der Formel

$$-CH_2-CO-NH-CH_2-CH_2-$$

bedeuten, W für Wasserstoff und Z für eine 1,2-Dihydroxyäthyl- oder 2-Hydroxyäthyl-Gruppe stehen, in der eine oder zwei Hydroxygruppen mit gleichen oder verschiedenen gegebenenfalls ein- oder zweifach ungesättigten Alkancarbonsäuren mit 16–22 Kohlenstoffatomen verestert oder mit einem gegebenenfalls ein- oder zweifach ungesättigten Alkanol mit 12–18 Kohlenstoffatomen veräthert sind oder worin zwei vicinale Hydroxygruppen unter Bildung eines Dioxolanringes formal mit einem Bisniederalkylketon ketalisiert oder mit einem unsubstituierten aliphatischen Aldehyd mit bis zu 20 C-Atomen acetalisiert sind, oder worin W und Z je eine Hydroxymethylgruppe darstellen, die mit einer gegebenenfalls ein- oder zweifach ungesättigten Alkancarbonsäure mit 16–22 Kohlenstoffatomen verestert oder mit einem gegebenenfalls ein- oder zweifach ungesättigten Alkanol mit 12–18 Kohlenstoffatomen veräthert ist, und ihre Salze, sowie solche Verbindungen, worin die Bedeutungen für $A_1$ und $A_2$ vertauscht sind, und ihre Salze.

Insbesondere betrifft die Erfindung die neuen, in den Beispielen beschriebenen Muramylpeptide.

Die neuen Verbindungen der Formel I können nach an sich bekannten Methoden erhalten werden.

So lassen sie sich erhalten, wenn man eine Verbindung der Formel

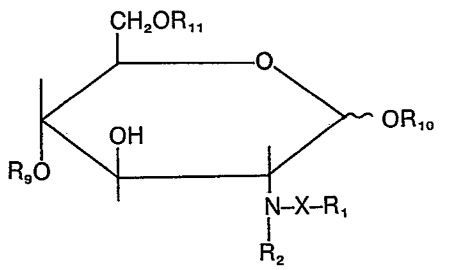

(V)

worin X, $R_1$ und $R_2$ die obgenannte Bedeutung haben, wobei gegebenenfalls darin vorhandene Hydroxygruppen mit einer leicht abspaltbaren Schutzgruppe geschützt sind, und $R_9$, $R_{10}$ und $R_{11}$ eine leicht abspaltbare Schutzgruppe bedeuten, oder eine Metallverbindung davon mit einer Verbindung der Formel

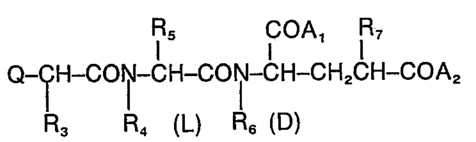

(VI)

(L)

oder (D,L)

umsetzt, worin Q eine reaktionsfähige veresterte Hydroxygruppe darstellt, $R_3$, $R_4$, $R_5$, $R_6$, $R_7$; $A_1$ und $A_2$ die oben angegebene Bedeutung haben und gegebenenfalls darin vorhandene Hydroxygruppen durch eine leicht abspaltbare Schutzgruppe geschützt sind, und vorhandene Schutzgruppen abspaltet.

Eine reaktionsfähige veresterte Hydroxygruppe ist insbesondere eine mit einer starken anorganischen oder organischen Säure veresterte Hydroxygruppe, in erster Linie eine solche, die mit Halogenwasserstoffsäure, wie Chlor-, Brom- oder insbesondere Jodwasserstoffsäure verestert ist.

Eine Metallverbindung ist insbesondere ein entsprechendes Alkalimetall-, z.B. Natrium- oder Kaliumderivat. Sie kann z.B. durch Behandeln einer Verbindung der Formel V mit einer geeigneten Base, wie einer entsprechenden Alkalimetallverbindung, wie Natriumhydrid, Natriumamid oder Butyllithium, hergestellt werden.

Leicht abspaltbare Schutzgruppen sind solche, die aus der Peptid- bzw. Zuckerchemie bekannt sind. Für Hydroxygruppen sollen insbesondere Acylreste, z.B. Niederalkanoylreste wie Acetyl, Aroylreste, wie Benzoyl und vor allem von Kohlensäurederivaten sich ableitende Reste wie Benzyloxycarbonyl oder Niederalkoxycarbonyl, oder Alkyl, insbesondere tert.-Butyl, gegebenenfalls durch Nitro, Niederalkoxy oder Halogen substituiertes Benzyl, gegebenenfalls durch Halogen oder Niederalkoxy, wie Methoxy, substituiertes Triphenylmethyl, oder Tetrahydropyranyl oder gegebenenfalls substituierte Alkylidenreste, die die Sauerstoffatome in 4- und 6-Stellung verbinden, genannt werden. Solche Alkylidenreste sind insbesondere ein Niederalkyliden-, in erster Linie der Methyliden-, Isopropyliden- oder Propylidenrest oder auch ein gegebenenfalls substituierter Benzylidenrest.

Diese Schutzgruppen können in an sich bekannter Weise abgespalten werden. So kann man sie durch saure Hydrolyse, Benzyl- oder Benzylidenreste auch hydrogenolytisch, z.B. mit Wasserstoff in Gegenwart eines Edelmetall-, wie Palladium- oder Platinkatalysators entfernen.

Die verwendeten Ausgangsstoffe sind bekannt oder lassen sich in an sich bekannter Weise herstellen.

Die neuen Verbindungen können auch erhalten werden, wenn man in an sich bekannter Weise eine Verbindung der Formel

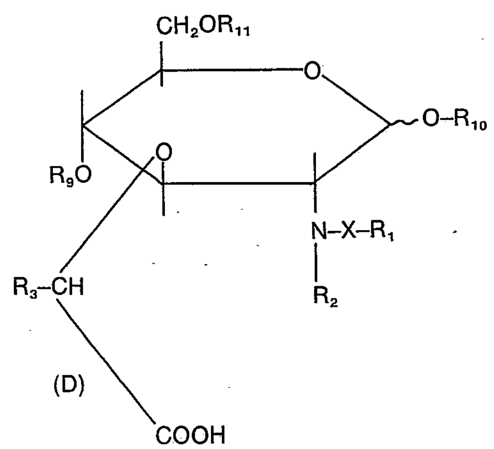

(VII)

worin X, $R_1$, $R_2$ und $R_3$ die obengenannte Bedeutung besitzen und $R_9$, $R_{10}$ und $R_{11}$ für Wasserstoff oder eine leicht abspaltbare Schutzgruppe stehen, oder ein Derivat davon, mit einer Verbindung der Formel

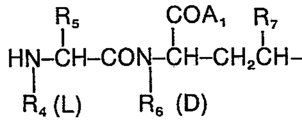
(VIII)

worin $R_4$, $R_5$, $R_6$, $R_7$, $A_1$ und $A_2$ die obgenannte Bedeutung besitzen, mit der Massgabe, dass in diesen Resten vorhandene Carboxy- und, wenn erwünscht, freie Hydroxygruppen durch leicht abspaltbare Schutzgruppen geschützt sind, oder einem Derivat davon kondensiert und vorhandene Schutzgruppen abspaltet.

Die Kondensation erfolgt dabei z.B. in der Weise, dass man die Säure (VII) in aktivierter Form mit der Aminoverbindung (VIII) umsetzt oder dass man die Säure (VII) mit der Verbindung (VIII), deren Aminogruppe in aktivierter Form vorliegt, umsetzt. Die aktivierte Carboxylgruppe kann beispielsweise ein Säureanhydrid, vorzugsweise ein gemischtes Säureanhydrid wie z.B. mit Kohlensäureniederalkylester, wie Kohlensäureäthyl- oder Isobutylester, ein Säureazid, ein Säureamid, wie ein Imidazolid, oder ein aktivierter Ester sein. Als aktivierte Ester seien insbesondere genannt:
Cyanmethylester, Carboxymethylester,
p-Nitrophenylthioester, p-Nitrophenylester,
2,4,5-Trichlorphenylester, Pentachlorphenylester
N-Hydroxy-succinimidester,
N-Hydroxy-phthalimidester,
8-Hydroxy-chinolinester,
2-Hydroxy-1,2-dihydro-1-carboäthoxy-chinolinester
N-Hydroxy-piperidinester oder Enolester,
die mit N-Äthyl-5-phenyl-isoxazolium-3'-sulfonat gebildet werden. Aktivierte Ester können auch gegebenenfalls mit einem Carbodimid unter Zusatz von N-Hydroxy-succinimid oder einem unsubstituierten oder z.B. durch Halogen, Methyl oder Methoxy substituierten 1-Hydroxybenzotriazol, 3-Hydroxy-4-oxo-3,4-dihydro-benzo[d]-1,2,3-triazin erhalten werden.

Die Aminogruppe ist beispielsweise durch Reaktion mit einem Phosphitamid aktiviert.

Unter den Methoden der Reaktion mit aktivierten Säuren sind insbesondere diejenigen mit N-Äthyl-5-phenyl-isoxyzolium-3'-sulfonat (Woodward Reagens K) oder
2-Äthoxy-1,2-dihydro-1-carboäthoxy-chinolin
oder Carbodiimid zu erwähnen.

Leicht abspaltbare Schutzgruppen sind solche, die aus der Pepti- bzw. Zuckerchemie bekannt sind. Für Carboxygruppen sollen insbesondere tertiär-Butyl, Benzyl oder Benzhydryl und für Hydroxygruppen insbesondere Acylreste, z.B. Niederalkanoylreste wie Acetyl, Aroylreste, wie Benzoyl und vor allem von Kohlensäurederivaten sich ableitende Reste, wie Benzyloxycarbonyl oder Niederalkoxycarbonyl, oder Alkyl, insbesondere tert.-Butyl, gegebenenfalls durch Nitro, Niederalkoxy oder Halogen substituiertes Benzyl, gegebenenfalls durch Halogen oder Niederalkoxy, wie Methoxy substituiertes Triphenylmethyl oder Tetrahydropyranyl oder gegebenenfalls substituierte Alkylidenreste, die die Sauerstoffatome in 4- und 6-Stellung verbinden, genannt werden. Solche Alkylidenreste sind insbesondere ein Niederalkyliden-, in erster Linie der Methyliden-, Isopropyliden oder Propylidenrest, oder auch ein gegebenenfalls substituierter, vorzugsweise in p-Stellung substituierter Benzylidenrest.

Diese Schutzgruppen können in an sich bekannter Weise abgespalten werden. So kann man sie durch saure Hydrolyse, Benzyl- oder Benzylidenreste auch hydrogenolytisch, z.B. mit Wasserstoff in Gegenwart eines Edelmetall-, wie Palladium- oder Platinkatalysators entfernen.

Die verwendeten Ausgangsstoffe sind bekannt oder lassen sich in an sich bekannter Weise herstellen.

Eine andere Verfahrensweise zur Herstellung dieser neuen Verbindungen besteht darin, dass man eine Verbindung der Formel

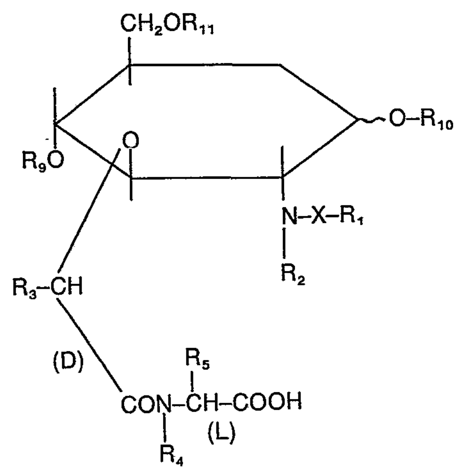
(IX)

worin X, $R_1$, $R_2$, $R_3$, $R_4$ und $R_5$ die obengenannte Bedeutung haben, mit der Massgabe, dass darin enthaltene freie Hydroxygruppen gegebenenfalls mit einer leicht abspaltbaren Schutzgruppe geschützt sind, und $R_9$, $R_{10}$ und $R_{11}$ Wasserstoff oder leicht abspaltbare Schutzgruppen darstellen, oder Derivate davon mit einer Verbindung der Formel

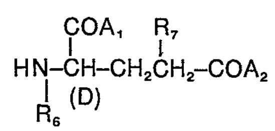
(X)

worin $R_6$, $R_7$, $A_1$ und $A_2$ die obengenannte Bedeutung haben, mit der Massgabe, dass in den Resten $R_7$, $A_1$ und $A_2$ vorhandene freie Carboxylgruppen durch leicht abspaltbare Schutzgruppen geschützt sind, kondensiert und vorhandene Schutzgruppen abspaltet.

Die Kondensation erfolgt dabei z.B. in der Weise, dass man die Säure IX in aktivierter Form mit der Aminoverbindung X umsetzt, oder dass man die Säure IX mit der Verbindung X, deren Aminogruppen in aktivierter Form vorliegt, umsetzt. Die aktivierte Carboxylgruppe kann beispielsweise

ein Säureanhydrid, vorzugsweise ein gemischtes Säureanhydrid, ein Säureamid oder ein aktivierter Ester sein. Als solche kommen insbesondere die oben genannten Säureanhydride, Amide oder Ester in Frage. Die Aminogruppe ist beispielsweise durch Reaktion mit einem Phosphitamid aktiviert.

Auch die leicht abspaltbaren Schutzgruppen entsprechen den bereits oben genannten. Sie können in an sich bekannter Weise abgespalten werden, durch saure Hydrolyse, Benzyl- oder Benzylidenreste auch hydrogenolytisch, beispielsweise mit Wasserstoff in Gegenwart eines Edelmetall-, wie Palladium- oder Platinkatalysators.

Die Ausgangsstoffe lassen sich in an sich bekannter Weise erhalten. So kann man z.B. entsprechende in 3-Stellung unsubstituierte Zucker mit einer Halogen-$R_3$-acetamido-$R_4$-essigsäure umsetzen, oder eine Verbindung der Formel VII mit einer Amino-$R_5$-essigsäure, deren Carboxylgruppe geschützt ist, in der oben gezeigten Weise umsetzen, und die Schutzgruppen abspalten.

Eine weitere Verfahrensweise zur Herstellung dieser neuen Verbindungen, worin T für NH steht, besteht darin, dass man in an sich bekannter Weise eine Verbindung der Formel

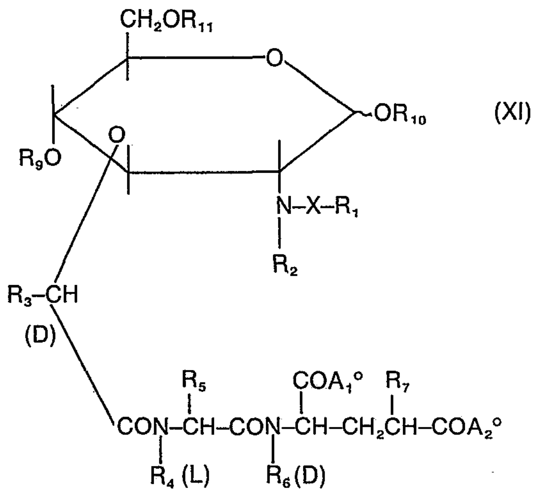

(XI)

worin X, $R_1$, $R_2$, $R_3$, $R_4$, $R_5$, $R_6$ und $R_7$ die obgenannte Bedeutung haben, $R_9$, $R_{10}$ und $R_{11}$ Wasserstoff oder eine leicht abspaltbare Schutzgruppe darstellen und einer der Reste $A_1°$ und $A_2°$ eine aktivierte Hydroxygruppe darstellt und der andere veräthertes Hydroxy, Amino. Niederalkylamino oder Carboxamidoniederalkylamino ist, mit einer Verbindung der Formel

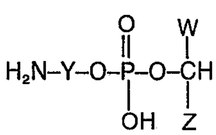

(XII)

worin Y, W und Z die obgenannte Bedeutung besitzen, kondensiert und vorhandene Schutzgruppen abspaltet.

Die aktivierte Carbonsäuregruppe $COA_1°$ bzw. $COA_2°$ kann beispielsweise ein Säureanhydrid, z.B. mit Kohlensäureniederalkylester, wie Kohlensäureäthyl- oder Isobutylester, ein Säureazid, ein Säureamid, wie Imidazolid, Isooxazolid oder ein aktivierter Ester sein. Als aktivierte Ester seine insbesondere genannt:
Cyanmethylester, Carboxymethylester,
p-Nitrophenylthioester,
Methoxyäthyltioester,
Acetylaminoäthylthioester,
p-Nitrophenylester,
2,4,5-Trichlorphenylester,
N-Hydroxy-succinimidester,
N-Hydroxyphthalimidester,
8-Hydroxy-chinolinester,
N-Hydroxy-piperidinester.
Aktivierte Ester können auch gegebenenfalls mit einem Carbodiimid unter Zusatz von N-Hydroxysuccinimid oder einem unsubstituierten oder z.B. durch Halogen, Methyl oder Methoxy substituierten 1-Hydroxy-benzotriazol oder
3-Hydroxy-4-oxo-3,4-dihydro-benzo[d]-1,2,3-triazin
erhalten werden.

Man bevorzugt als aktive Ester solche mit N-Hydroxysuccinimid oder deren C-Substitutionsprodukten, wie N-Hydroxy-methyl- oder -dimethylsuccinimid, oder die Umsetzung mit Carbodiimid, wie Carbodiimid selbst oder
1-Äthyl-3-(3-dimethylaminopropyl)-carbodiimid.

Die dazu verwendeten Ausgangsstoffe sind bekannt oder lassen sich in an sich bekannter Weise herstellen.

Steht in den neuen Verbindungen der Formel I T für O, kann man sie auch erstellen, wenn man in an sich bekannter Weise eine Verbindung der Formel

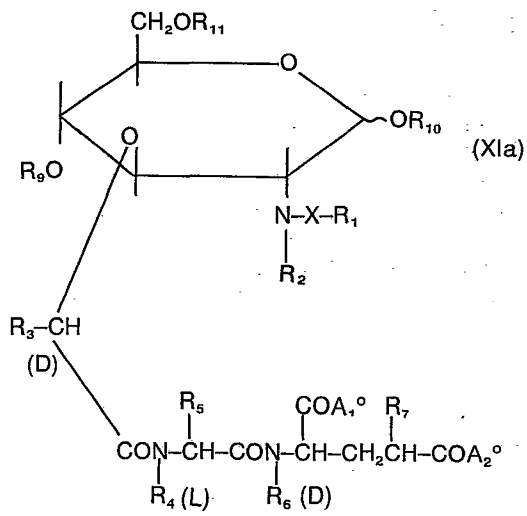

(XIa)

worin X, $R_1$, $R_2$, $R_3$, $R_4$, $R_5$, $R_6$ und $R_7$ die obengenannte Bedeutung haben, $R_9$, $R_{10}$ und $R_{11}$ Wasserstoff oder eine leicht abspaltbare Schutzgruppe darstellen und einer der Reste $A_1°$ und $A_2°$ eine Hydroxygruppe darstellt und der andere veräthertes Hydroxy, Amino, Niederalkylamino oder Carboxamidoniederalkylamino ist, mit einer Verbindung der Formel

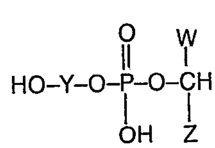

(XIIa)

worin Y, W und Z die obgenannte Bedeutung besitzen, wobei die Säure XIa oder der Alkohol XIIa in reaktionsfähiger Form vorliegt, verestert, und gegebenenfalls vorhandene Schutzgruppen abspaltet.

Diese Reaktion lässt sich so durchführen, dass man die freie Säure mit dem Alkohol in Gegenwart eines wasserabspaltenden Mittels, wie eines Carbodiimids, z.B. Dicyclohexylcarbodiimid, und eines Amins, wie Pyridin oder Dimethylaminopyridin oder eines Trialkylamins, z.B. Trimethylamin, verestert. Man kann aber auch die Carbonsäure, z.B. in Form eines Salzes, wie Natrium- oder Kaliumsalzes, mit einem reaktionsfähigen Ester des Alkohols, z.B. einem Ester mit einer starken anorganischen oder organischen Säure, wie einer Halogenwasserstoffsäure, z.B. Chlor-, Brom- oder Jodwasserstoffsäure, oder einer organischen Sulfonsäure, wie p-Toluolsulfonsäure oder Methan- oder Äthansulfonsäure umsetzen.

Ferner ist es auch möglich, den Alkohol gegebenenfalls als Salz, z.B. Natrium- oder Kaliumsalz, mit einer aktivierten Carbonsäure umzusetzen. Als aktivierte Carbonsäure sind insbesondere Anhydride, in erster Linie gemischte Säureanhydride, wie Säureazide oder Halogenide, oder aktivierte Ester zu nennen, wie
Cyanmethylester, Carboxymethylester,
p-Nitrophenylthioester,
p-Nitrophenylester,
2,4,5-Trichlorphenylester,
Pentachlorphenylester,
N-Hydroxy-succinimidester,
N-Hydroxy-phthalimidester,
8-Hydroxy-chinolinester,
2-Hydroxy-1,2-dihydro-1-carboäthoxy-chinolin-ester,
N-Hydroxy-piperidinester
oder Enolester, die mit
N-Äthyl-5-phenylisoxazolium-3'-sulfonat
gewonnen werden. Aktivierte Ester können auch gegebenenfalls mit einem Carbodiimid unter Zusatz von N-Hydroxysuccinimid oder einem unsubstituierten oder z.B. durch Halogen, Methyl oder Methoxy substituierten 1-Hydroxy-benzotriazol oder
3-Hydroxy-4-oxo-3,4-dihydro-benzo[d]-1,2,3-triazin
erhalten werden.

Leicht abspaltbare Schutzgruppen sind solche, die aus der Peptid- bzw. Zuckerchemie bekannt sind. Für Carboxygruppen sollen insbesondere tertiär-Butyl, Benzyl oder Benzhydryl und für Hydroxygruppen insbesonders Acylreste, z.B. Niederalkanoylreste, wie Acetyl, Aroylreste, wie Benzoyl, und vor allem von der Kohlensäure sich ableitende Reste wie Benzyloxycarbonyl oder Niederalkoxycarbonyl, oder Alkyl, insbesondere tert.-Butyl, gegebenenfalls durch Nitro, Niederalkoxy oder Halogen substituiertes Benzyl, gegebenenfalls durch Halogen oder Niederalkoxy, wie Methoxy substituiertes Triphenylmethyl oder Tetrahydropyranyl oder gegebenenfalls substituierte Alkylidenreste, die die Sauerstoffatome in 4- und 6-Stellung verbinden, genannt werden.

Solche Alkylidenreste sind insbesondere ein Niederalkyliden-, in erster Linie der Äthyliden-, Isopropyliden- oder Propylidenrest oder auch ein gegebenenfalls substituierter, vorzugsweise in p-Stellung substituierter Benzylidenrest.

Diese Schutzgruppen können in an sich bekannter Weise abgespalten werden. So kann man sie durch saure Hydrolyse, Benzyl oder Benzyliden auch hydrogenolytisch, z.B. mit Wasserstoff in Gegenwart eines Edelmetall-, wie Palladium- oder Platinkatalysators entfernen.

Ferner ist es auch möglich, die neuen Verbindungen der Formel I, worin X eine Carbonylgruppe und $R_2$ Wasserstoff sind, zu erhalten, wenn man in einer Verbindung der Formel

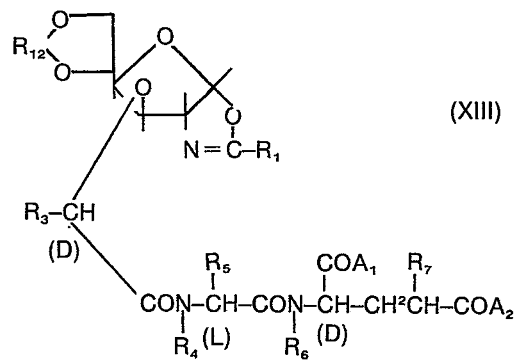

(XIII)

worin $R_1$, $R_3$, $R_4$, $R_5$, $R_6$, $R_7$, $A_1$ und $A_2$ die obengenannte Bedeutung haben und $R_{12}$ eine Alkyliden- oder Cycloalkylidengruppe ist, den Oxazolin- und den Dioxolanring sauer aufspaltet, und gegebenenfalls vorhandene Schutzgruppen abspaltet.

Alkyliden ist darin insbesondere Niederalkyliden, wie Isopropyliden, und Cycloalkyliden in erster Linie Cyclopentyliden oder Cyclohexyliden.

Diese Spaltung erfolgt ebenfalls in an sich bekannter Weise, z.B. mit einem sauren Ionenaustauscher, insbesondere solchen mit Sulfonsäuregruppen, wie Amberlite IR-120 (ein Styrolharz mit stark sauren Sulfogruppen) oder Dowex 50 (Polystyrolsulfonsäuren) oder einer starken anorganischen oder organischen Säure, wie Salzsäure, Bromwasserstoffsäure, Schwefelsäure oder einer Sulfonsäure, z.B. Methansulfonsäure, oder einer gegebenenfalls im aromatischen Ring substituierten Phenylsulfonsäure, wie p-Toluolsulfonsäure oder Trifluoressigsäure. Arbeitet man dabei in Gegenwart von Wasser, erhält man in 1-Stellung eine freie Hydroxygruppe. Ist auch eine der Carboxylgruppen $-COA_1$ bzw. $-COA_2$ und/oder $R_7$ mit einem Alkohol, insbesondere einem Niederalkanol verestert, lässt sie sich, insbesondere bei höherer Temperatur mit wässriger Säure verseifen.

In den erhaltenen Verbindungen lassen sich Schutzgruppen am Peptidrest nachträglich, z.B. durch Hydrogenolyse, wie z.B. mit katalytisch angeregtem Wasserstoff oder Hydrolyse, abspalten.

Die dabei verwendeten Ausgangsstoffe lassen sich z.B. erhalten, wenn man in ein entsprechendes Oxazolin mit einer freien Hydroxygruppe in 3-Stellung des Zuckerrestes den $R_3$-Acetamidopeptidrest in einer oder mehreren Stufen einführt.

Verbindungen der Formel I, worin $Y_1$ einen Rest der Formel IIIc oder IIId darstellt, kann man auch dadurch gewinnen, dass man eine Verbindung der Formel

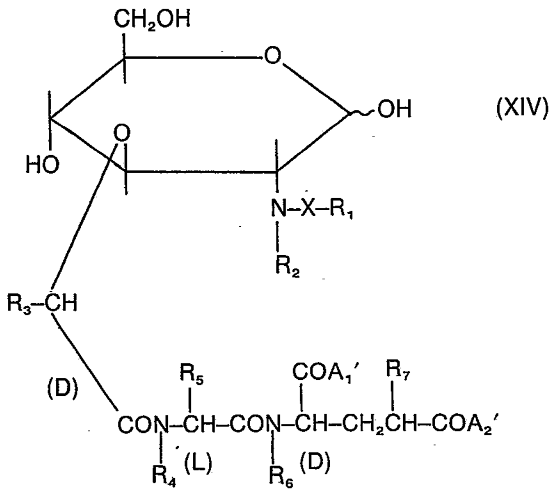

(XIV)

worin X, $R_1$, $R_2$, $R_3$, $R_4$, $R_5$, $R_6$ und $R_7$ die obengenannten Bedeutungen haben, wobei darin vorhandene Hydroxygruppen gegebenenfalls durch leicht abspaltbare Schutzgruppen geschützt sind, einer der Reste $A_1'$ und $A_2'$ einen Rest der Formel

$$-T-Y_1-M_1 \qquad (XV)$$

bedeutet, worin T die obengenannte Bedeutung hat, $Y_1$ gegebenenfalls substituiertes Alkylen, das durch eine Oxycarbonyl- oder Iminocarbonylgruppe unterbrochen sein kann, wobei darin vorhandene Hydroxygruppen gegebenenfalls durch leicht abspaltbare Schutzgruppen geschützt sind, und $M_1$ eine freie Aminogruppe, eine Carbonsäuregruppe oder ein aktiviertes Derivat dieser beiden Gruppen bedeuten, und der andere der Reste $A_1'$ und $A_2'$ veräthertes Hydroxy oder Amino, Niederalkylamino oder Carboxamidoniederalkylamino ist, mit einer Verbindung der Formel

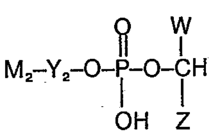

(XVI)

worin $M_2$ eine freie Aminogruppe oder ein aktiviertes Derivat davon bedeutet, wenn $M_1$ für eine Carbonsäuregruppe oder ein aktiviertes Derivat derselben steht, oder worin $M_2$ eine Carbonsäure oder ein aktiviertes Derivat derselben bedeutet, wenn $M_1$ für eine freie Aminogruppe oder ein aktiviertes Derivat davon steht, $Y_2$ gegebenenfalls substituiertes Alkylen, das durch eine Oxycarbonyl- oder Iminocarbonylgruppe unterbrochen sein kann, bedeutet, mit der Massgabe, dass die über eine Iminocarbonylgruppe miteinander verbundenen Reste $Y_1$ und $Y_2$ dem obengenannten Rest Y entsprechen, und W und Z die obengenannten Bedeutungen haben, wobei in den Resten $Y_2$, W und Z vorhandene Hydroxygruppen gegebenenfalls durch leicht abspaltbare Schutzgruppen geschützt sind, kondensiert und gegebenenfalls vorhandene Schutzgruppen abspaltet.

Leicht abspaltbare Schutzgruppen sind solche, die aus der Peptid- bzw. Zuckerchemie bekannt sind. Für Carboxygruppen sollen insbesondere tertiär-Butyl, Benzyl, gegebenenfalls durch Halogen oder Niederalkoxy, wie Methoxy, substituiertes Triphenylmethyl oder Benzhydryl und für Hydroxygruppen insbesondere Acylreste, z.B. Niederalkanoylreste, wie Acetyl, Aroylreste, wie Benzoyl, und vor allem von der Kohlensäure sich ableitende Rest wie Benzyloxycarbonyl oder Niederalkoxycarbonyl, oder Alkyl, insbesondere tert.-Butyl, gegebenenfalls durch Nitro, Niederalkoxy oder Halogen substituiertes Benzyl oder Tetrahydropyranyl oder gegebenenfalls substituierte Alkylidenreste, die die Sauerstoffatome in 4- und 6-Stellung verbinden, genannt werden. Ein solcher Alkylidenrest ist insbesondere ein Niederalkyliden-, in erster Linie der Äthyliden-, Isopropyliden- oder Propylidenrest oder auch ein gegebenenfalls substituierter, vorzugsweise in p-Stellung substituierter Benzylidenrest.

Diese Schutzgruppen können in an sich bekannter Weise abgespalten werden. So kann man sie durch saure Hydrolyse, Benzyl- oder Benzylidenreste auch hydrogenolytisch, z.B. mit Wasserstoff in Gegenwart eines Edelmetall-, wie Palladium- oder Platinkatalysators entfernen.

Die verwendeten Ausgangsstoffe sind bekannt, oder lassen sich in an sich bekannter Weise herstellen.

Die Kondensation erfolgt dabei z.B. in der Weise, dass man die Verbindung (XIV) in Form der aktivierten Carbonsäuren mit der Aminoverbindung (XVI) umsetzt oder dass man die Säure (XIV) mit der Verbindung (XVI), deren Aminogruppe in aktivierter Form vorliegt, umsetzt. Die aktivierte Carboxylgruppe kann beispielsweise ein Säureanhydrid, vorzugsweise ein gemischtes Säureanhydrid, wie z.B. mit Kohlensäureniederalkylester, wie Kohlensäureäthyl- oder Isobutylester, ein Säureazid, ein Säureamid, wie ein Imidazolid, Isoxazolid oder ein aktivierter Ester sein. Als aktivierte Ester seien insbesondere genannt:

Cyanmethylester, Carboxymethylester,
p-Nitrophenyltioester, p-Nitrophenylester,
2,4,5-Trichlorphenylester, Pentachlorphenylester,
N-Hydroxy-succinimidester,
N-Hydroxy-phthalimidester,
8-Hydroxy-chinolinester,
2-Hydroxy-1,2-dihydro-1-carboäthoxy-chinolinester,
N-Hydroxy-piperidinester oder Enolester, die mit N-Äthyl-5-phenylisoxazolium-3′-sulfonat gewonnen werden. Aktivierte Ester können auch gegebenenfalls mit einem Carbodiimid unter Zusatz von Hydroxysuccinimid oder einem unsubstituierten oder z.B. durch Halogen, Methyl oder Methoxy substituierten 1-Hydroxybenzotriazol oder 3-Hydroxy-4-oxo-3,4-dihydro-benzo[d]-1,2,3-triazin erhalten werden.

Die Aminogruppe ist beispielsweise durch Reaktion mit einem Phosphit aktiviert.

Unter den Methoden der Reaktion mit aktivierten Säuren sind insbesondere diejenigen mit N-Äthyl-5-phenyl-isoxazolium-3'-sulfonat (Woodward Reagens K) oder 2-Äthoxy-1,2-dihydro-1-carboäthoxy-chinolin oder Carbodiimid zu erwähnen.

Verbindungen der Formel I, worin Y einen Rest der Formel IIIa oder IIIb darstellt, lassen sich auch erhalten, wenn man eine Verbindung der Formel

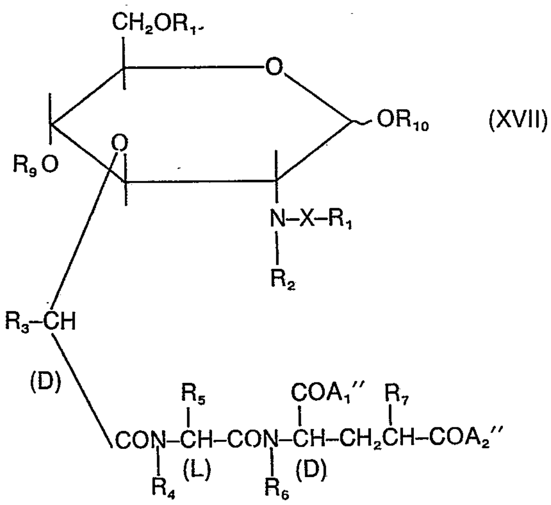

(XVII)

worin X, $R_1$, $R_2$, $R_3$, $R_4$, $R_5$, $R_6$ und $R_7$ die obengenannten Bedeutungen haben, $R_9$, $R_{10}$ und $R_{11}$ leicht abspaltbare Schutzgruppen darstellen, einer der Reste $A_1''$ und $A_2''$ einen Rest der Formel

$$-T-Y_1-M_3 \qquad \text{(XVIII)}$$

bedeutet, worin T die obengenannte Bedeutung hat, $Y_1$ gegebenenfalls substituiertes Alkylen, das durch eine Oxycarbonyl- oder Iminocarbonylgruppe unterbrochen sein kann, wobei darin vorhandene Hydroxygruppen gegebenenfalls durch leicht abspaltbare Schutzgruppen geschützt sind, und $M_3$ eine freie Hydroxy- oder Carboxylgruppe, die gegebenenfalls in reaktionsfähiger Form vorliegt, bedeuten, und der andere der Reste $A_1''$ und $A_2''$ veräthertes Hydroxy, Amino, Niederalkylamino oder Carboxamidoniederalkylamino ist, mit einer Verbindung der Formel

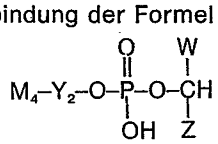

(XIX)

worin $M_4$ eine gegebenenfalls in reaktionsfähiger Form vorliegende Hydroxygruppe bedeutet, wenn $M_3$ eine gegebenenfalls in reaktionsfähiger Form vorliegende Carboxylgruppe bedeutet, oder worin $M_4$ eine gegebenenfalls in reaktionsfähiger Form vorliegende Carboxylgruppe bedeutet, wenn $M_3$ eine gegebenenfalls in reaktionsfähiger Form vorliegende Hydroxygruppe bedeutet, $Y_2$ gegebenenfalls substituiertes Alkylen, das durch eine Oxycarbonyl- oder Iminocarbonylgruppe unterbrochen sein kann, bedeutet, mit der Massgabe, dass die über eine Oxycarbonylgruppe miteinander verbundenen Reste $Y_1$ und $Y_2$ dem obengenannten Rest Y entsprechen, und W und Z die obengenannte Bedeutung haben, wobei in den Resten $Y_2$, W und Z vorhandene Hydroxygruppen gegebenenfalls durch leicht abspaltbare Schutzgruppen geschützt sind, verestert und gegebenenfalls vorhandene Schutzgruppen abspaltet.

Diese Reaktion lässt sich so durchführen, dass man die freie Säure mit Alkohol in Gegenwart eines wasserabspaltenden Mittels, wie einem Carbodiimid, z.B. Dicyclohexylcarbodiimid, und einem Amin wie Pyridin, Dimethylaminopyridin, einem Trialkylamin, z.B. Trimethylamin, verestert. Man kann aber auch die Carbonsäure, z.B. in Form eines Salzes, mit einem reaktionsfähigen Ester des Alkohols, z.B. einem Ester mit einer starken anorganischen oder organischen Säure, wie einer Halogenwasserstoffsäure, z.B. Chlor-, Brom- oder Jodwasserstoffsäure oder einer organischen Sulfonsäure, wie p-Toluolsulfonsäure oder Methan- oder Äthansulfonsäure, umsetzen.

Ferner ist es auch möglich, den Alkohol gegebenenfalls als Salz, z.B. Natrium- oder Kaliumsalz, mit einer aktivierten Carbonsäure umzusetzen. Als aktivierte Carbonsäuren sind insbesondere Anhydride, in erster Linie gemischte Säureanhydride, wie ein Säureazid oder Halogenid oder ein aktivierter Ester zu nennen, wie Cyanmethylester, Carboxymethylester, p-Nitrophenylthioester, p-Nitrophenylester, 2,4,5-Trichlorphenylester, Pentachlorphenylester, N-Hydroxysuccinimidester, N-Hydroxyphthalimidester, 8-Hydroxy-chinolinester, 2-Hydroxy-1,2-dihydro-1-carboäthoxy-chinolin-ester, N-Hydroxypiperidinester oder Enolester, die mit N-Äthyl-5-phenylisoxazolium-3'-sulfonat gewonnen werden. Aktivierte Ester können auch gegebenenfalls mit einem Carbodiimid unter Zusatz von N-Hydroxysuccinimid oder einem unsubstituierten oder z.B. durch Halogen, Methyl oder Methoxy substituierten 1-Hydroxybenzotriazol, 3-Hydroxy-4-oxo-3,4-dihydro-benzo[d]-1,2,3-triazin erhalten werden.

Leicht abspaltbare Schutzgruppen sind solche, die aus der Peptid- bzw. Zuckerchemie bekannt sind. Für Carboxygruppen sollen insbesondere tertiär-Butyl, Benzyl, gegebenenfalls durch Halogen oder Niederalkoxy, wie Methoxy substituiertes Triphenylmethyl oder Benzhydryl und für Hydroxygruppen insbesondere Acylreste, z.B. Niederalkanoylester, wie Acetyl, Aroylreste, wie Benzoyl, und vor allem von der Kohlensäure sich ableitende Rest wie Benzyloxycarbonyl oder Niederalkoxycarbonyl, oder Alkyl, insbesondere tert.-Butyl, gegebenenfalls durch Nitro, Niederalkoxy oder Halogen substituiertes Benzyl oder Tetrahydropyranyl oder gegebenenfalls substituierte Alkylidenreste, die die Sauerstoffatome in 4- und 6-Stellung verbinden, genannt werden. Ein solcher Alkylidenrest ist insbesondere ein Niederalkyliden-, in erster Linie der Äthyliden-, Isopropyliden- oder Propylidenrest oder auch ein gegebenenfalls substituierter, vorzugsweise in p-Stellung substituierter Benzylidenrest.

Diese Schutzgruppen können in an sich bekannter Weise abgespalten werden. So kann man sie durch saure Hydrolyse, Benzyl- oder Benzylidenreste auch hydrogenolytisch, z.B. mit Wasserstoff in Gegenwart eines Edelmetall-, wie Palladium- oder Platinkatalysators entfernen.

Die verwendeten Ausgangsstoffe sind bekannt und lassen sich in an sich bekannter Weise herstellen.

Eine weitere Verfahrensmethode zur Herstellung der neuen Verbindungen der Formel I besteht darin, dass man eine Verbindung der Formel

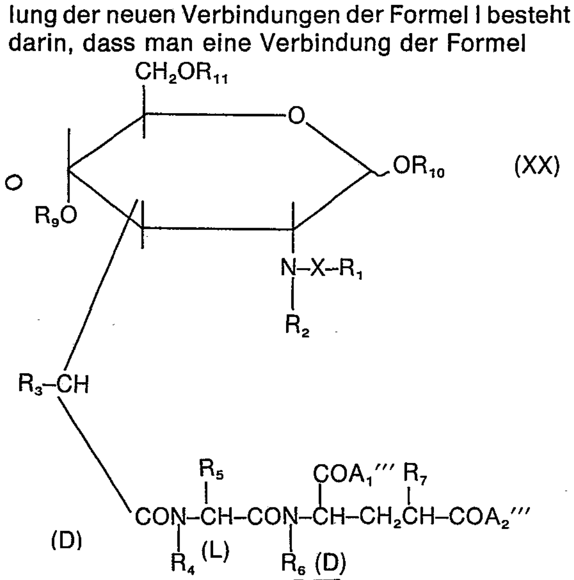

(XX)

worin X, $R_1$, $R_2$, $R_3$, $R_4$, $R_5$, $R_6$ und $R_7$ die obgenannte Bedeutung haben, wobei gegebenenfalls darin vorhandene Hydroxygruppen mit leicht abspaltbaren Schutzgruppen geschützt sind, $R_9$, $R_{10}$ und $R_{11}$ leicht abspaltbare Schutzgruppen bedeuten und einer der Reste $A_1'''$ und $A_2'''$ für –T–Y–OH steht, worin Y und T die obgenannten Bedeutungen besitzen, wobei gegebenenfalls darin vorhandene Hydroxygruppen mit leicht abspaltbaren Schutzgruppen geschützt sind, und der andere der Reste $A_1'''$ und $A_2'''$ für veräthertes Hydroxy, Amino, Niederalkylamino oder Carboxamidoniederalkylamino steht, mit einer den Rest der Formel

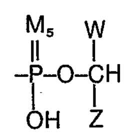

(XXI)

worin $=M_5$ ein Elektronenpaar oder Oxo bedeutet, und W und Z die obengenannten Bedeutungen haben, wobei gegebenenfalls darin vorhandene Hydroxygruppen mit leicht abspaltbaren Schutzgruppen geschützt sind, abgebenden Verbindung umsetzt, falls $=M_5$ ein Elektronenpaar ist, mit einem schwachen Oxydationsmittel oxydiert, und vorhandene Schutzgruppen abspaltet.

Als einen Rest der Formel XXI abgebende Verbindungen sollen insbesondere Verbindungen der Formel

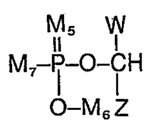

(XXII)

genannt werden, worin W, Z und $=M_5$ die obgenannte Bedeutung besitzen, $M_6$ eine leicht abspaltbare Schutzgruppe und $M_7$ gegebenenfalls in reaktionsfähiger Form vorliegendes Hydroxy, z.B. Halogen, ist.

Eine leicht abspaltbare Schutzgruppe $M_6$ ist insbesondere Niederalkyl, wie Methyl oder Äthyl, Niederalkenyl, wie Äthenyl oder 1-Methyl-propenyl, oder Benzyl.

Gegebenenfalls in reaktionsfähiger Form vorliegendes Hydroxy $M_7$ ist insbesondere freies Hydroxy oder mit einer starken anorganischen oder organischen Säure verestertes Hydroxy, wie mit einer Halogenwasserstoff-, Niederalkancarbonsäure oder Aryl- oder Alkansulfonsäure, z.B. p-Toluolsulfonsäure, Methan- oder Äthansulfonsäure, verestertes Hydroxy. $M_7$ kann aber auch für eine Phenoxy- oder Niederalkoxygruppe stehen.

Diese Reaktion wird vorzugsweise in Gegenwart eines säurebindenden Mittels, wie Pyridin, Triniederalkylamin, z.B. Triäthylamin oder Trimethylamin, eines Imidazols, oder einer anorganischen Base, wie Natrium- oder Kaliumhydroxyd, oder eines Natrium- oder Kaliumalkoholats durchgeführt, wobei man als Lösungsmittel ein aprotisches Lösungsmittel wie Dimethylsulfoxyd oder Acetonitril bevorzugt.

Ist in den erhaltenen Verbindungen $M_5$ ein Elektronenpaar, oxydiert man z.B. mit einer Persäure, wie Benzoepersäure oder einem Alkylhydroperoxyd.

Die Abspaltung einer Schutzgruppe $M_6$ geht meist gemeinsam mit der Abspaltung der übrigen Schutzgruppen. Diese können in an sich bekannter Weise, z.B. hydrogenolytisch, z.B. mit Wasserstoff in Gegenwart eines Edelmetall-, wie Palladium- oder Platinkatalysators oder durch saure Hydrolyse entfernt werden.

Die Ausgangsstoffe sind bekannt und lassen sich auf an sich bekannte Weise, z.B. nach einer der vorstehend genannten, entsprechend abgewandelten Methoden herstellen.

Im weiteren kann man die neuen Verbindungen der Formel I auch herstellen, wenn man eine Verbindung der Formel

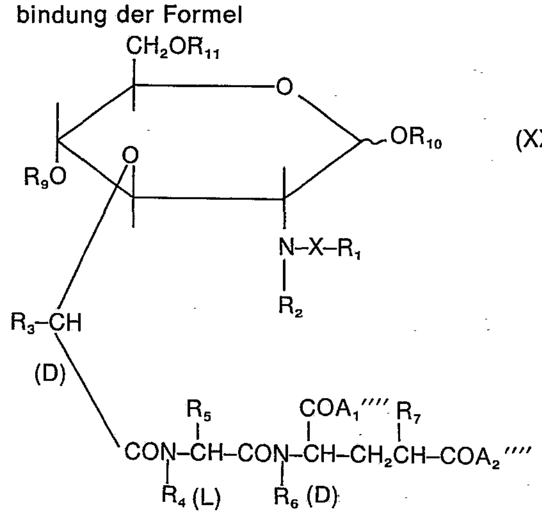

(XXIII)

worin X, $R_1$, $R_2$, $R_3$, $R_4$, $R_5$, $R_6$ und $R_7$ die obgenannten Bedeutungen besitzen, wobei gegebenenfalls darin vorhandene Hydroxygruppen mit leicht ab-

spaltbaren Schutzgruppen geschützt sind, $R_9$, $R_{10}$ und $R_{11}$ leicht abspaltbare Schutzgruppen bedeuten und einer der Reste $A_1''''$ und

$A_2''''$ für 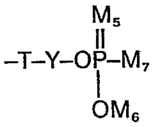 (XXIV)

worin T und Y die obengenannte Bedeutung besitzen, wobei gegebenenfalls darin vorhandene Hydroxygruppen mit leicht abspaltbaren Schutzgruppen geschützt sind, und worin = $M_5$ ein Elektronenpaar oder Oxo, $M_6$ eine leicht abspaltbare Schutzgruppe und $M_7$ freies oder in reaktionsfähiger Form vorliegendes Hydroxy bedeuten, und der andere der Reste $A_1''''$ und $A_2''''$ für freies oder veräthertes Hydroxy, Amino, Niederalkylamino oder Carboxamidoniederalkylamino steht, mit einer Verbindung der Formel

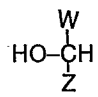 (XXV)

worin W und Z die obgenannte Bedeutung besitzen, wobei gegebenenfalls darin vorhandene Hydroxygruppen mit leicht abspaltbaren Schutzgruppen geschützt sind, umsetzt, falls $=M_5$ ein Elektronenpaar ist, mit einem schwachen Oxydationsmittel oxydiert, und vorhandene Schutzgruppen abspaltet.

Eine leicht abspaltbare Schutzgruppe $M_6$ ist insbesondere Niederalkyl, wie Methyl oder Äthyl, Niederalkenyl, wie Äthenyl oder 1-Methyl-propenyl, oder Benzyl.

Gegebenenfalls in reaktionsfähiger Form vorliegendes Hydroxy $M_7$ ist insbesondere freies Hydroxy oder mit einer starken anorganischen oder organischen Säure verestertes Hydroxy, wie mit einer Halogenwasserstoff-, Nitroalkancarbonsäure oder Aryl- oder Alkylsulfonsäure, z.B. p-Toluolsulfonsäure, Methan- oder Äthansulfonsäure, veresteres Hydroxy. $M_7$ kann aber auch für eine Phenoxy- oder Niederalkoxygruppe stehen.

Diese Reaktion wird vorzugsweise in Gegenwart eines säurebindenden Mittels, wie Pyridin, Triniederalkylamin, z.B. Triäthylamin oder Trimethylamin, eines Imidazols, oder einer anorganischen Base, wie Natrium- oder Kaliumhydroxyd, oder eines Natrium- oder Kaliumalkoholats, durchgeführt, wobei man als Lösungsmittel ein aprotisches Lösungsmittel, wie Dimethylsulfoxyd oder Acetonitril bevorzugt.

Ist in den erhaltenen Verbindungen $M_5$ ein Elektronenpaar, oxydiert man z.B. mit einer Persäure, wie Benzoesäure oder einem Alkylhydroperoxyd.

Die Abspaltung einer Schutzgruppe $M_6$ geht meist gemeinsam mit der Abspaltung der übrigen Schutzgruppen. Diese können in an sich bekannter Weise, z.B. hydrogenolytisch, z.B. mit Wasserstoff in Gegenwart eines Edelmetall-, wie Palladium- oder Platinkatalysators oder durch saure Hydrolyse entfernt werden.

Die Ausgangsstoffe sind bekannt und lassen sich auf an sich bekannte Weise, z.B. nach einer der vorstehend genannten, entsprechend abgewandelten Methoden herstellen.

Ferner kann man die Verbindungen der Formel I auch dadurch erhalten, dass man eine Verbindung der Formel

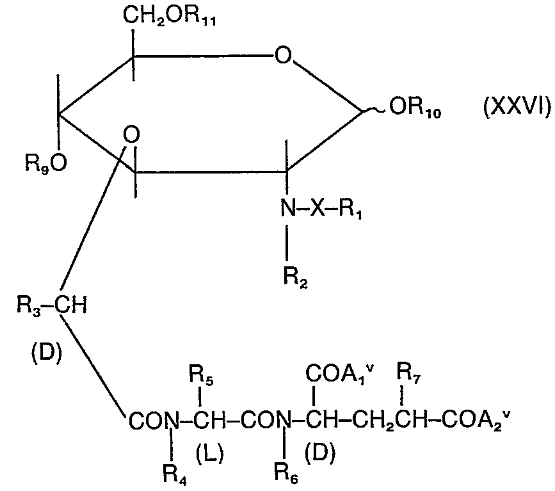 (XXVI)

worin X, $R_1$, $R_2$, $R_3$, $R_4$, $R_5$, $R_6$ und $R_7$ die obgenannte Bedeutung haben, wobei gegebenenfalls darin vorhandene Hydroxygruppen mit leicht abspaltbaren Schutzgruppen geschützt sind, $R_9$, $R_{10}$ und $R_{11}$ leicht abspaltbare Schutzgruppen bedeuten und einer der Reste $A^v_1$ und $A^v_2$ für einen Rest der Formel XXVII

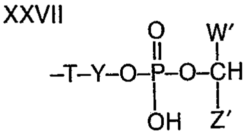 (XXVII)

steht, worin T und Y die obengenannten Bedeutungen haben, wobei in einem Rest der Formel XXVII vorhandene freie funktionelle Gruppen mit Ausnahme der an der Reaktion teilnehmenden Hydroxygruppe(n) gegebenenfalls durch leicht abspaltbare Schutzgruppen geschützt sind, und W' und Z' die obengenannte Bedeutung von W bzw. Z haben, jedoch mit der Massgabe, dass mindestens eine Hydroxygruppe in dem Rest 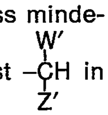 in freier Form vorliegt, und der andere der Reste $A_1^v$ und $A_2^v$ für freies oder veräthertes Hydroxy, Amino, Niederalkylamino oder Carboxamidoniederalkyl-amino steht, mit einer gegebenenfalls ungesättigten aliphatischen Carbonsäure mit 12–90 C-Atomen oder einem reaktionsfähigen Derivat davon verestert, oder mit einem gegebenenfalls ungesättigten aliphatischen Alkohol mit 10–22 C-Atomen oder einem reaktionsfähigen Derivat davon veräthert und die vorhandenen Schutzgruppen abspaltet.

Die Veresterung wie auch die Verätherung erfolgen in an sich bekannter Weise. So verwendet man vorzugsweise die aliphatische Carbonsäure und den aliphatischen Alkohol in Form eines reaktionsfähigen Derivates, wie eines Anhydrids, vorzugsweise eines gemischten Anhydrids, z.B. mit einer Halogenwasserstoffsäure, beziehungsweise einem Ester, ebenfalls z.B. mit einer Halogenwasserstoffsäure.

Die Abspaltung der Schutzgruppen, welche den vorgenannten entsprechen, lässt sich in üblicher

Weise, insbesondere hydrogenolytisch oder durch saure Hydrolyse durchführen.

Die Ausgangsstoffe lassen sich z.B. nach einer der oben genannten, entsprechend abgewandelten Methoden durchführen.

Die oben beschriebenen Verfahren werden nach an sich bekannten Methoden durchgeführt, in Abwesenheit oder vorzugsweise in Anwesenheit von Verdünnungs- oder Lösungsmitteln, wenn notwendig, unter Kühlen oder Erwärmen, unter erhöhtem Druck und/oder in einer Inertgas-, wie Stickstoffatmosphäre.

Dabei sind unter Berücksichtigung aller im Molekül befindlichen Substituenten, wenn erforderlich, insbesondere bei Anwesenheit leicht hydrolysierbarer O-Acylreste, besonders schonende Reaktionsbedingungen, wie kurze Reaktionszeiten, Verwendung von milden sauren oder basischen Mitteln in niedriger Konzentration, stöchiometrische Mengenverhältnisse, Wahl geeigneter Katalysatoren, Lösungsmittel, Temperatur- und/oder Druckbedingungen, anzuwenden.

Die Erfindung betrifft auch diejenigen Ausführungsformen des Verfahrens, bei denen man von einer auf irgendeiner Stufe des Verfahrens als Zwischenprodukt erhältlichen Verbindung ausgeht und die fehlenden Verfahrensschritte durchführt, oder das Verfahren auf irgendeiner Stufe abbricht, oder einen Ausgangsstoff unter den Reaktionsbedingungen bildet oder in Form eines reaktionsfähigen Derivats oder Salzes verwendet. Dabei geht man vorzugsweise von solchen Ausgangsstoffen aus, die verfahrensgemäss zu den oben als besonders wertvoll beschriebenen Verbindung führen.

Die vorliegende Erfindung betrifft ebenfalls pharmazeutische Präparate, welche Verbindungen der Formel I enthalten. Bei den erfindungsgemässen pharmazeutischen Präparaten handelt es sich um solche zur enteralen, wie oralen, nasalen oder rektalen, sowie parenteralen Verabreichung an Warmblüter, welche den pharmakologischen Wirkstoff allein oder zusammen mit einem pharmazeutisch anwendbaren Trägermaterial enthalten. Die Dosierung des Wirkstoffes hängt von der Warmblüter-Spezies, dem Alter und dem individuellen Zustand, sowie von der Applikationsweise ab.

Die neuen pharmazeutischen Präparate enthalten von etwa 10% bis etwa 95%, vorzugsweise von etwa 20% bis etwa 90% des Wirkstoffs. Erfindungsgemässe pharmazeutische Präparate können z.B. in Dosiseinheitsform, wie Dragées, Tabletten, Kapseln, Suppositorien oder Ampullen vorliegen.

Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren hergestellt. Ausser den auf Seite 40 erwähnten Applikationsarten können auch pharmazeutische Präparate insbesondere zur oralen Anwendung erhalten werden, indem man den Wirkstoff mit festen Trägerstoffen kombiniert, ein erhaltenes Gemisch gegebenenfalls granuliert, und das Gemisch bzw. Granulat, wenn erwünscht oder notwendig nach Zugabe von geeigneten Hilfsstoffen, zu Tabletten oder Dragée-Kernen verarbeitet. Dabei kann man sie auch in Kunststoffträger einbauen, die die Wirkstoffe dosiert abgeben oder diffundieren lassen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose, Hydroxypropyl-methylcellulose, Natriumcarboxymethylcellulose und/oder Polyvinylpyrrolidon, und/oder, wenn erwünscht Sprengmittel, wie die obgenannten Stärken, ferner Carboxymethylstärke, quervernetztes Polyvinylpyrrolidon, Agar, Alginsäure oder ein Salz davon, wie Natriumalginat, Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragées-Kerne werden mit geeigneten, gegebenenfalls magensaftresistenten Überzügen versehen, wobei man u.a. konzentrierte Zuckerlösungem, welche gegebenenfalls arabischen Gummi, Talk, Polyvinylpyrrolidon, Polyäthylenglycol und/oder Titandioxid enthalten, Lacklösungen in geeigneten organischen Lösungsmitteln oder Lösungsmittelgemischen oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Die nachfolgenden Beispiele illustrieren die oben beschriebene Erfindung; sie sollen jedoch diese in ihrem Umfang in keiner Weise einschränken. Temperaturen werden in Celsiusgraden angegeben.

Die erfindungsgemässen Verbindungen der Formel I können weder durch einen Schmelzpunkt charakterisiert werden, noch sind spektroskopische Daten wie NMR- und IR-Spektren zur einwandfreien Charakterisierung geeignet.

Ungeeignet zur Feincharakterisierung ist ferner wegen der dominierenden Natur der Lipidteile auch die Angabe von $R_f$-Werten.

Da jedoch die Struktur der Ausgangsstoffe genau bekannt ist (vgl. Deutsche Offenlegungsschrift 2 655 500; die jeweils verwendete Phospholipidkomponente ist kommerziell erhältlich) und da die Verknüpfung von Phospholipid und Muramylpeptid eindeutig ist, ist damit auch die Sequenz der Bausteine im Endprodukt und dessen Struktur eindeutig festgelegt.

In den Verbindungen der Formel I kann das über ein Sauerstoffatom an Phosphor gebundene Proton leicht mit Basen abgespalten werden. Üblicherweise liegen die Verbindungen der Formel I in Form eines Gemisches der freien Verbindungen und ihrer Salze, z.B. Natriumsalze, vor. So liegen etwa 40–55% der in den nachfolgenden Beispielen beschriebenen Muramylpeptide der Formel I als Triethylammoniumsalze vor. Diese Salze gehören zum Gegenstand der Erfindung.

Die Erfindung betrifft generell auch die Salze der Verbindungen der Formel I mit irgendwelchen anderen salzbildenden Gruppen, z.B. freien Carboxylgruppen, in erster Linie pharmazeutisch verwendbare nichttoxische Salze, z.B. Metall- oder Ammoniumsalze.

Beispiel 1

Zu einer Lösung von 14 mMol
2-(1,4-Dipalmitoyl-sn-glycero-3-hydroxyphosphoryloxy)-äthylamin
und 2,5 mMol Triäthylamin in 25 ml eines Gemisches aus Chloroform-Methanol-Wasser, 65 : 25 : 4, wird eine Lösung von 2 mMol
N-Acetylmuramyl-L-alanyl-D-isoglutamin-N-hydroxysuccinimidester
in 6,5 ml Dimethylacetamid zugetropft. Nach 18 Stunden Rühren bei 20 °C wird die Lösung bei reduziertem Druck auf ca. 15 ml eingeengt; dabei entsteht eine Emulsion. Diese wird mit 200 ml Wasser verdünnt und gefriergetrocknet. Der Rückstand wird in 30 ml Wasser suspendiert und extensiv gegen Wasser dialysiert. Das Innendialysat, das das gewünschte Produkt enthält, wird gefriergetrocknet. Das
N-Acetalymuramyl-L-alanyl-D-isoglutamin-2-(1′,2′-dipalmitoyl-sn-glycero-3′-hydroxyphosphoryloxy)-äthylamid
wird durch Chromatographie an einer Sephadex LH-20 Säure gereinigt. Elutionsgemisch: Chloroform-Methanol-Essigsäure-Wasser, 25 : 15 : 4 : 2. Die Verbindung zeigt im Dünnschichtchromatogramm auf Silicagel folgende Rf-Werte: 0,31 (in Chloroform-Methanol-Wasser, 65 : 25 : 4) bzw. 0,64 (in Chloroform-Methanol-Essigsäure-Wasser, 25 : 15 : 4 : 2).

Die neue Verbindung wird analytisch dadurch charakterisiert, dass die Bausteine – N-Acetylmuraminsäure, Palmitinsäure, Phosphat, L-Alanin und D-Glutaminsäure – quantitativ bestimmt werden. N-Acetylmuraminsäure wird mit Hilfe der Morgan-Elson-Reaktion nach der Modifikation von J.M. Ghuysen et al. [in «Methods in Enzymology» 8, 629 (1966)] spektrophotometrisch bestimmt.

Phosphat wird nach Lowry et al. [J. Biol. Chem. 207, 1 (1954)] quantitativ bestimmt.

Palmitinsäure und die Aminosäuren werden in einem Totalhydrolysat (6N HCl, 25 Std. 110 °C) gaschromatographisch bzw. mit Hilfe eines Aminosäureanalysators unter Verwendung von Pentadecansäure bzw. Norleucin als interne Standards quantitativ bestimmt.

Die gefundenen molaren Verhältnisse bezogen auf Phosphat sind wie folgt:
$PO_4'''$: N-Acetylmuraminsäure: L-Alanin : D-Glutaminsäure : Palmitinsäure =
1 : 0,92 : 0,91 : 0,95 : 2,18.

Der N-Acetymuramyl-L-alanyl-D-isoglutamin-N-hydroxy-succinimidester, der als Ausgangsstoff verwendet wird, lässt sich z.B. wie folgt herstellen:

2 mMol N-Acetylmuramyl-L-alanyl-D-isoglutamin, 2,2 mMol N-Hydroxysuccinimid und 2,2 mMol Dicyclohexylcarbodiimid werden in 6,5 ml Dimethylacetamid gelöst und 18 Stunden bei 20 °C gerührt. Der ausgefallene Dicyclohexylharnstoff wird abgetrennt und die Lösung direkt für die Kondensation mit dem Phospholipid verwendet.

Das 2-(1,2-Dipalmitoyl-sn-glycero-3-hydroxyphosphoryloxy)-äthylamin, das als Ausgangsstoff verwendet wird, ist ein kommerziell erhältliches synthetisches Präparat.

Beispiel 2

In analoger Weise zu Beispiel 1 erhält man
N-Acetyl-desmethylmuramyl-L-alanyl-D-isoglutamin-2-(1′-2′-dipalmitoyl-sn-glycero-3′-hydroxyphosphoryloxy)-äthylamin,
ausgehend von
2-(1,2-Dipalmitoyl-sn-glycero-3-hydroxyphosphoryloxy)-äthylamin
und
N-Acetyldesmethyl-muramyl-L-alanyl-D-isoglutamin-N-hydroxysuccinimidester
auf Silicagel folgende Rf-Werte:
0,29 (in Chloroform-Methanol-Wasser, 65:25:4), bzw. 0,65 (in Chloroform-Methanol-Essigsäure-Wasser, 25 : 15 : 4 : 2).

Beispiel 3

In analoger Weise zu Beispiel 1 erhält man
N-Acetyl-muramyl-L-alanyl-D-isoglutaminyl-γ-oxy-essigsäure-2-(1′,2′-dipalmitoyl-sn-glycero-3′-hydroxyphosphoryloxy)-äthylamid,
ausgehend von
2-(1,2-Dipalmitoyl-sn-glycero-3-hydroxyphosphoryloxy)-äthylamin
und von dem N-Hydroxysuccinimidester von
N-Acetylmuramyl-L-alanyl-D-isoglutamin-γ-carboxymethylester.

Die Verbindung zeigt im Dünnschichtchromatogramm auf Silicagel folgende Rf-Werte: 0,28 (in Chloroform-Methanol-Wasser, 65 : 25 : 4), bzw. 0,68 (in Chloroform-Methanol-Essigsäure-Wasser, 25 : 15 : 4 : 2).

Beispiel 4

In analoger Weise zu Beispiel 1 erhält man
N-Acetylmuramyl-L-alanyl-D-isoglutaminyl-L-alanin-2-(1′,2′-dipalmitoyl-sn-glycero-3′-hydroxyphosphoryloxy)-äthylamid,
ausgehend von
2-(1,2-Dipalmitoyl-sn-glycero-3-hydroxyphosphoryloxy)-äthylamin
und
N-Acetylmuramyl-L-alanyl-D-isoglutaminyl-L-alanin-N-hydroxysuccinimidester.
Rf-Wert im Dünnschichtchromatogramm auf Silicagel: 0,3 (im System Chloroform-Methanol-Wasser, 65 : 25 : 4).

Beispiel 5

In analoger Weise zu Beispiel 1 erhält man N-Acetyl-des-methylmuramyl-L-alanyl-D-isoglutamin-2-(1′,2′-di-hexadecyl-sn-glycero-3′-hydroxyphosphoryloxy)-äthylamid, ausgehend von 2-(1,2-Dihexadecyl-sn-glycero-3-hydroxy-phosphoryloxy)-äthylamin und N-Acetyldesmethylmuramyl-L-alanyl-D-isoglutamin-N-hydroxysuccinimidester. Rf-Wert im Dünnschichtchromatogramm auf Silicagel: 0,43 (in Chloroform-Methanol-Wasser, 65 : 25 : 4).

Das 2-(1,2-Dihexadecyl-sn-glycero-3-hydroxy-phosphoryloxy)-äthylamin, das als Ausgangsstoff verwendet wird, ist ein kommerziell erhältliches synthetisches Präparat.

Beispiel 6

In analoger Weise zu Beispiel 1 erhält man N-Acetyl-des-methylmuramyl-L-alanyl-D-isoglutamin-2-(3′-palmitoyl-rac-glycero-1′-hydroxyphosphoryloxy)-äthylamid ausgehend von 2-(3-Palmitoyl-rac-glycero-1-hydroxyphosphoryloxy)-äthylamin und N-Acetyl-desmethylmuramyl-L-alanyl-D-isoglutamin-N-hydroxysuccinimidester. Rf-Wert im Dünnschichtchromatogramm auf Silicagel: 0,47 (in Chloroform-Methanol-Wasser, 65 : 25 : 4).

Das 2-(3-Palmitoyl-rac-glycero-1-hydroxyphosphoryloxy)-äthylamin, das als Ausgangsstoff verwendet wird, ist ein kommerziell erhältliches synthetisches Präparat.

Beispiel 7

In analoger Weise zu Beispiel 1 erhält man N-Acetyl-desmethylmuramyl-L-alanyl-D-isoglutamin-2-(2′-palmitoyl-2′-oleoyl-sn-glycero-3′-hydroxyphosphoryloxy)-äthylamid, ausgehend von 2-(1-Palmitoyl-2-oleoyl-sn-glycero-3-hydroxy-phosphoryloxy)-äthylamin und N-Acetyl-desmethylmuramyl-L-alanyl-D-isoglutamin-N-hydroxysuccinimidester. Rf-Wert im Dünnschichtchromatogramm auf Silicagel: 0,33 (in Chloroform-Methanol-Wasser, 65 : 24 : 4).

Das 2-(1-Palmitoyl-2-oleoyl-sn-glycero-3-hydroxyphosphoryloxy)-äthylamin, das als Ausgangsstoff verwendet wird, ist ein kommerziell erhältliches synthetisches Präparat.

Beispiel 8

In analoger Weise zu Beispiel 1 erhält man N-Acetyl-desmethylmuramyl-L-alanyl-D-isoglutamin-2-(1′-O-palmitoyl-propandiol-3′-O-hydroxyphosphoryloxy)-äthylamid, ausgehend von 2-(1-O-Palmitoyl-propandiol-3-O-hydroxy-phosphoryloxy)-äthylamin und N-Acetyl-desmethylmuramyl-L-alanyl-D-isoglutamin-N-hydroxysuccinimidester. Rf-Wert im Dünnschichtchromatogramm auf Silicagel: 0,49 (in Chloroform-Methanol-Wasser, 65 : 25 : 4).

Das 2-(1-O-Palmitoyl-propandiol-3-O-hydroxy-phosphoryloxy)-äthylamin, das als Ausgangsstoff verwendet wird, ist ein kommerziell erhältliches synthetisches Präparat.

Beispiel 9

In analoger Weise zu den vorstehenden Beispielen erhält man N-Acetylmuramyl-L-alanyl-D-isoglutaminyl-glycerophospholid Derivate durch Kondensation von N-Acetylmuramyl-L-alanyl-D-isoglutamin mit folgenden Phospholipiden:
2-(1′,2′-Hexydecyliden-sn-glycero-3′-hydroxyphosphoryloxy)-äthylamin,
2-(1′-Palmitoyl-2′-oleoyl-sn-glycero-3′-hydroxyphosphoryloxy)-äthylamin,
2-(3′-Palmitoyl-rac-glycero-1′-hydroxy-phosphoryloxy)-äthylamin oder
2-(1′-O-Palmitoyl-propandiol-3′-O-hy-droxy-phosphoryloxy)-äthylamin.

Beispiel 10

In analoger Weise zu den vorstehenden Beispielen erhält man N-Acetylmuramyl-L-alanyl-D-isoglutaminyl-L-alanyl-glycerophospholipid Derivate durch Kondensation von N-Acetylmurymyl-L-alanyl-D-isoglutaminyl-L-alanin mit folgenden Phospholipiden:
2-(1′, 2′-Hexadecyliden-sn-glycero-3′-hydroxy-phosphoryloxy)-äthylamin,
2-(1′-Palmitoyl-2′-oleoyl-sn-glycero-3′-hydroxy-phosphoryloxy)-äthylamin,
2-(3′-Palmitoyl-rac-glycero-1′-hydroxyphosphoryloxy)-äthylamin
oder
2-(1′-O-Palmitoyl-propandiol-3′-O-hydroxyphosphoryloxy)-äthylamin.

Beispiel 11

In analoger Weise zu den vorstehenden Beispielen erhält man N-Acetyl-desmethylmuramyl-L-alanyl-D-isoglutaminyl-L-alanyl-glycerophospholipid Derivate durch Kondensation von N-Acetyl-desmethylmuramyl-L-alanyl-D-isoglutaminyl-L-alanin mit folgenden Phospholipiden:
2-(1′,2′-Dipalmitoyl-sn-glycero-3′-hy droxyphosphoryloxy)-äthylamin,
2-(1′,2′-Hexadecyliden-sn-glycero-3′-hydroxyphosphoryloxy)-äthylamin,
2-(1′-Palmitoyl-2′-oleoyl-sn-glycero-3′-hydroxyphosphoryloxy)-äthylamin,
2-(3′-Palmitoyl-rac-glycero-1′-hydroxy-phosphoryloxy)-äthylamin

oder
2-(1'-O-Palmitoyl-propandiol-3'-hydroxyphosphoryloxy)-äthylamin.

Ausgangsstoffe lassen sich wie folgt erhalten:

A) Zu einer Lösung von 6 g (10mM) α-Benzyl-N-acetyl-muramyl-L-alanyl-D-isoglutamin und 1,75 ml (11mM) Bromessigsäurebenzylester in 100 ml Dimethylformamid werden unter Ausschluss von Feuchtigkeit und gutem Rühren 1,87 ml (11mM) N,N-Diisopropyl-äthylamin, gelöst in 50 ml Dimethylformamid, innert 1¼ Stunden zugetropft. Nach Rühren über Nacht bei Raumtemperatur wird das Reaktionsgemisch im Hochvakuum vom Dimethylformamid befreit und der Rückstand mit 100 ml Wasser versetzt. Das ausfallende Öl verfestigt sich rasch und kristallisiert durch. Nach Rühren im Eisbad wird die Suspension abgesaugt, die erhaltenen Kristalle mit etwas Wasser gewaschen und über phosphorpentoxid getrocknet.

Nach Resuspendieren der Kristalle in Petroläther, Filtrieren und Trocknen (50°) verbleiben 6,2 g (85% d.Th.) farbl. Kristalle, Fp. 194; 195–98° $[\alpha]_D^{20}$+ 1° (C = 1,3; Methanol)

B) 6,5 g (8,85 mM) α-Benzyl-N-acetyl-muramyl-L-alanyl-D-isoglutamin-γ-benzyloxycarbonylmethylester werden in 200 ml Methanol/Wasser 1 : 1 in Gegenwart von 0,5 g Pd-Kohle/10%ig während 40 Stunden (40°) mit Wasserstoff behandelt.

Das Reaktionsgemisch wird auf die übliche Weise vom Katalysator befreit und das Filtrat zur Trockne verdampft.

Das anfallende Öl wird in 75 ml sek. Butanol gesättigtem Wasser gelöst, 6 × mit je 50 ml Wasser gesättigtem sek. Butanol und 1 × mit Essigsäureäthylester extrahiert. Die organischen Phasen werden mit Wasser (s.o.) rückextrahiert und die bereinigten wässrigen Phasen nach Behandlung mit Kohle (Darco G 60) zur Trockene verdampft. Der Rückstand wird 2 × mit Wasser versetzt, eingedampft und schliesslich lyophilisiert. Man erhält 4,0 g (82% d.Th.) weisses Lyophilisat $[\alpha]_D^{20}$+ 34 ± 1° (C = 0,8; Wasser)

C) 6,1 g α-Benzyl-N-acetyl-muramyl-L-alanyl-D-isoglutamin und 3,5 g L-Alaninbenzylesterhydrochlorid werden in 30 ml Dimethylformamid gelöst, die Lösung auf 0° abgekühlt und nacheinander mit 1,4 ml Triäthylamin, 1,1 g N-Hydroxysuccinimid und schliesslich mit 2,3 g Dicyclohexylcarbodiimid versetzt.

Nach 48stündigem Rühren bei Raumtemperatur wird die Suspension filtriert, der Niederschlag mit wenig Dimethylformamid gewaschen und das Filtrat im Hochvakuum zur Trockene verdampft. Der Rückstand wird in 100 ml Wasser bei 0° suspendiert, der Niederschlag abfiltriert und nach Trocknen aus Methanol/Wasser rekristallisiert.

Man erhält 5,5 g (74% d.Th.) $[\alpha]_D^{20}$+ 70 + 1° (C = 0,5; Methanol)

D) 3,4 g α-Benzyl-N-acetyl-muramyl-L-alanyl-D-isoglutaminyl-L-alaninbenzylester, gelöst in 100 ml Methanol/Wasser 2 : 1, werden in Gegenwart von Pd-Kohle/10%ig während 40 Stunden mit Wasserstoff behandelt. Der Katalysator wird abgesaugt, das Filtrat fast zur Trockene verdampft und der Rückstand nach Lösen in 40 ml mit sek.Butanol gesättigtem Wasser 3 × mit je 40 ml wassergesättigtem sek.Butanol extrahiert. Nach Rückextraktion der organischen Phase mit Wasser (3 × 40 ml, s.o.) werden die vereinigten Wasserextrakte eingedampft und lyophilisiert.

2,2 g (80% d.Th.) farbl. Pulver $[\alpha]_D^{20}$+ 9 ± 1° (C = 1,1; Wasser)

Beispiel 12

In analoger Weise werden die folgenden Verbindungen erhalten. (Ermittlung der Analysenwerte wie in Beispiel 1):

-N-Benzoyl-muramyl-L-alanyl-D-isoglutamin-2-(1',2'-dipalmitoyl-sn-glycero-3'-hydroxyphosphoryloxy)-äthylamid,

-N-Benzoyl-muramyl-L-alanyl-D-isoglutamin-2-(1'-palmitoyl-sn-glycero-3'-hydroxyphosphoryloxy)-äthylamid,

-N-Benzoyl-muramyl-L-alanyl-D-isoglutamin-2-(1'-O-palmitoyl-propandiol-3'-O-hydroxy-phosphoryloxy)-äthylamid,

-N-Benzoyl-muramyl-L-alanyl-D-isoglutamin-2-(1',2'-dihexadecyl-rac-glycero-3'-hydroxyphosphoryloxy)-äthylamid,

-N-Benzoyl-muramyl-L-alanyl-D-isoglutamin-2-(1'-hexadecyl-rac-glycero-3'-hydroxyphosphoryloxy)-äthylamid,

-N-Benzoyl-muramyl-L-alanyl-D-isoglutamin-2-(1',3'-dipalmitoyl-glycero-2'-hydroxyphosphoryloxy)-äthylamid,

-N-Benzoyl-muramyl-L-alanyl-D-isoglutaminyl-L-alanin-2-(1',2'-dipalmitoyl-sn-glycero-3'-hydroxyphosphoryloxy)-äthylamid,

-N-Benzoyl-muramyl-L-alanyl-D-isoglutaminyl-L-alanin-2-(1'-palmitoyl-sn-glycero-3'-hydroxyphosphoryloxy)-äthylamid,

-N-Benzoyl-muramyl-L-alanyl-D-isoglutaminyl-L-alanin-2-(1'-O-palmitoly-propandiol-3'-hydroxyphosphoryloxy)-äthylamid,

-N-Benzoyl-muramyl-L-alanyl-D-isoglutaminyl-L-alanin-2-(1',2'-dihexadecyl-rac-glycero-3'-hydroxyphosphoryloxy)-äthylamid,

-N-Benzoyl-muramyl-L-alanyl-D-isoglutaminyl-L-alanin-2-(1'-hexadecyl-rac-glycero-3'-hydroxyphosphoryloxy)-äthylamid,

-N-Benzoyl-muramyl-L-alanyl-D-isoglutaminyl-L-alanin-2-(1',3'-dipalmitoyl-glycero-2'-hydroxyphosphoryloxy)-äthylamid,

-N-Benzoyl-muramyl-L-alanyl-D-glutaminyl-L-alanin-2-(1',2'-dipalmitoyl-sn-glycero-3'-hydroxyphosphoryloxy)-äthylamid,

-N-Benzoyl-muramyl-L-alanyl-D-glutaminyl-L-alanin-2-(1'-pamitoyl-sn-glycero-3'-hydroxyphosphoryloxy)-äthylamid,

-N-Benzoyl-muramyl-L-alanyl-D-glutaminyl-L-alanin-2-(1'-O-palmitoyl-propandiol-3'-O-hydroxyphosphoryloxy)-äthylamid,

-N-Benzoyl-muramyl-L-alanyl-D-glutaminyl-L-alanin-2-(1',2'-dihexadecyl-rac-glycero-3'-hydroxyphosphoryloxy)-äthylamid,
-N-Benzoyl-muramyl-L-alanyl-D-glutaminyl-L-alanin-2-(1'-hexydecyl-rac-glycero-3'-hydroxyphosphoryloxy)-äthylamid,
-N-Benzoyl-muramyl-L-alanyl-D-glutaminyl-L-alanin-2-(1',3'-dipalmitoyl-glycero-2'-hydroxyphosphoryloxy)-äthylamid,
-N-Acetyl-desmethylmuramyl-L-seryl-D-isoglutamin-2-(1',2'-dipalmitoyl-sn-glycero-3'-hydroxyphosphoryloxy)-äthylamid,
-N-Acetyl-desmethylmuramyl-L-seryl-D-isoglutamin-2-(1'-palmitoyl-sn-glycero-3'-hydroxyphosphoryloxy)-äthylamid,
-N-Acetyl-desmethylmuramyl-L-seryl-D-isoglutamin-2-(1'-O-palmitoyl-propandiol-3'-O-hydroxyphosphoryloxy)-äthylamid,
-N-Acetyl-desmethylmuramyl-L-seryl-D-isoglutamin-2-(1',2'-dihexadecyl-rac-glycero-3'-hydroxyphosphoryloxy)-äthylamid,
-N-Acetyl-desmethylmuramyl-L-seryl-D-isoglutamin-2-(1'-hexadecyl-rac-glycero-3'-hydroxyphosphoryloxy)-äthylamid,
-N-Acetyl-desmethylmuramyl-L-seryl-D-isoglutamin-2-(1',3'-dipalmitoyl-glycero-2'-hydroxyphosphoryloxy)-äthylamid,
-N-Acetyl-desmethylmuramyl-L-seryl-D-isoglutaminyl-L-alanin-2-(1',2'-dipalmitoyl-sn-glycero-3'-hydroxyphosphoryloxy)-äthylamid,
-N-Acetyl-desmethylmuramyl-L-seryl-D-isoglutaminyl-L-alanin-2-(1'-palmitoyl-sn-glycero-3'-hydroxyphosphoryloxy)-äthylamid,
-N-Acetyl-desmethylmuramyl-L-seryl-D-isoglutaminyl-L-alanin-2-(1'-O-palmitoyl-propandiol-3'-O-hydroxyphosphoryloxy)-äthylamid,
-N-Acetyl-desmethylmuramyl-L-seryl-D-isoglutaminyl-L-alanin-2-(1',2'-dihexadecyl-rac-glycero-3'-hydroxyphosphoryloxy)-äthylamid,
-N-Acetyl-desmethylmuramyl-L-seryl-D-isoglutaminyl-L-alanin-2-(1'-hexadecyl-rac-glycero-3'-hydroxyphosphoryloxy)-äthylamid,
-N-Acetyl-desmethylmuramyl-L-seryl-D-isoglutaminyl-L-alanin-2-(1',3'-dipalmitoyl-glycero-2'-hydroxyphosphoryloxy)-äthylamid,

-N-Acetyl-desmethylmuramyl-L-seryl-D-glutaminyl-L-alanin-2-(1',2'-dipalmitoyl-sn-glycero-3'-hydroxyphosphoryloxy)-äthylamid,

-N-Acetyl-desmethylmuramyl-L-seryl-D-glutaminyl-L-alanin-2-(1'-palmitoyl-sn-glycero-3'-hydroxyphosphoryloxy)-äthylamid,
-N-Acetyl-desmethylmuramyl-L-seryl-D-glutaminyl-L-alanin-2-(1'-O-palmitoyl-propandiol-3'-O-hydroxyphosphoryloxy)-äthylamid,
-N-Acetyl-desmethylmuramyl-L-seryl-D-Glutaminyl-L-alanin-2-(1',2'-dihexadecyl-rac-glycero-3'-hydroxyphosphoryloxy)-äthylamid,
-N-Acetyl-desmethylmuramyl-L-seryl-D-glutaminyl-L-alanin-2-(1'-hexydecyl-rac-glycero-3'-hydroxyphosphoryloxy)-äthylamid,
-N-Acetyl-desmethylmuramyl-L-seryl-D-glutaminyl-L-alanin-2-(1',3'-dipalmitoyl-glycero-2'-hydroxyphosphoryloxy)-äthylamid,
-N-Acetyl-desmethylmuramyl-L-α-aminobutyryl-D-isoglutamin-2-(1'-2'-dipalmitoyl-sn-glycero-3'-hydroxyphosphoryloxy)-äthylamid,
-N-Acetyl-desmethylmuramyl-L-α-aminobutyryl-D-isoglutamin-2-(1'-palmitoyl-sn-glycero-3'-hydroxyphosphoryloxy)-äthylamid,
-N-Acetyl-desmethylmuramyl-L-α-aminobutyryl-D-isoglutamin-2-(1'-O-palmitoyl-propandiol-3'-O-hydroxyphosphoryloxy)-äthylamid,
-N-Acetyl-desmethylmuramyl-L-α-aminobutyryl-D-isoglutamin-2-(1',2'-dihexadecyl-rac-glycero-3'-hydroxyphosphoryloxy)-äthylamid,
-N-Acetyl-desmethylmuramyl-L-α-aminobutyryl-D-isoglutamin-2-(1'-hexadecyl-rac-glycero-3'-hydroxyphosphoryloxy)-äthylamid,
-N-Acetyl-desmethylmuramyl-L-α-aminobutyryl-D-isoglutamin-2-(1',3'-dipalmitoyl-glycero-2'-hydroxyphosphoryloxy)-äthylamid,
-N-Acetyl-desmethylmuramyl-L-α-aminobutyryl-D-isoglutaminyl-L-alanin-2-(1',2'-dipalmitoyl-sn-glycero-3'-hydroxyphosphoryloxy)-äthylamid,
-N-Acetyl-desmethylmuramyl-L-α-aminobutyryl-D-isoglutaminyl-L-alanin-2-(1'-palmitoyl-sn-glycero-3'-hydroxyphosphoryloxy)-äthylamid,
-N-Acetyl-desmethylmuramyl-L-α-aminobutyryl-D-isoglutaminyl-L-alanin-2-(1'-O-palmitoyl-propandiol-3'-O-hydroxyphosphoryloxy)-äthylamid,
-N-Acetyl-desmethylmuramyl-L-α-aminobutyryl-D-isoglutaminyl-L-alanin-2-(1',2'-dihexadecyl-rac-glycero-3'-hydroxyphosphoryloxy)-äthylamid,

-N-Acetyl-desmethylmuramyl-L-α-aminobutyryl-D-isoglutaminyl-L-alanin-2-(1'-hexadecyl-rac-glycero-3'-hydroxyphosphoryloxy)-äthylamid,

-N-Acetyl-desmethylmuramyl-L-α-aminobutyryl-D-isoglutaminyl-L-alanin-2-(1',3'-dipalmitoyl-glycero-2'-hydroxyphosphoryloxy)-äthylamid,
-N-Acetyl-desmethylmuramyl-L-α-aminobutyryl-D-glutaminyl-L-alanin-2-(1',2'-dipalmitoyl-sn-glycero-3'-hydroxyphosphoryloxy)-äthylamid,
-N-Acetyl-desmethylmuramyl-L-α-aminobutyryl-D-glutaminyl-L-alanin-2-(1'-palmitoyl-sn-glycero-3'-hydroxyphosphoryloxy)-äthylamid,
-N-Acetyl-desmethylmuramyl-L-α-aminobutyryl-D-glutaminyl-L-alanin-2-(1'-O-palmitoyl-propandiol-3'-O-hydroxyphosphoryloxy)-äthylamid,
-N-Acetyl-desmethylmuramyl-L-α-aminobutyryl-D-glutaminyl-L-alanin-2-(1',2'-dihexadecyl-rac-glycero-3'-hydroxyphosphoryloxy)-äthylamid,
-N-Acetyl-desmethylmuramyl-L-α-aminobutyryl-D-glutaminyl-L-alanin-2-(1'-hexadecyl-rac-glycero-3'-hydroxyphosphoryloxy)-äthylamid,
-N-Acetyl-desmethylmuramyl-L-α-aminobutyryl-D-glutaminyl-L-alanin-2-(1',3'-dipalmitoyl-glycero-2'-hydroxyphosphoryloxy)-äthylamid,
-N-Acetyl-muramyl-L-α-aminobutyryl-D-isoglutamin-2-(1',2'-dipalmitoyl-sn-glycero-3'-hydroxyphosphoryloxy)-äthylamid,
-N-Acetyl-muramyl-L-α-aminobutyryl-D-isoglutamin-2-(1'-palmitoyl-sn-glycero-3'-hydroxyphosphoryloxy)-äthylamid,
-N-Acetyl-muramyl-L-α-aminobutyryl-D-isoglutamin-2-(1'-O-palmitoyl-propandiol-3'-O-hydroxyphosphoryloxy)-äthylamid,
-N-Acetyl-muramyl-L-α-aminobutyryl-D-isoglutamin-2-(1',2'-dihexadecyl-rac-glycero-3'-hydroxyphosphoryloxy)-äthylamid,
-N-Acetyl-muramyl-L-α-aminobutyryl-D-isoglutamin-2-(1'-hexadexyl-rac-glycero-3'-hydroxyphosphoryloxy)-äthylamid,
-N-Acetyl-muramyl-L-α-aminobutyryl-D-isoglutamin-2-(1',3'-dipalmitoyl-glycero-2'-hydroxyphosphoryloxy)-äthylamid,
-N-Acetyl-muramyl-L-α-aminobutyryl-D-isoglutaminyl-L-alanin-2-(1',2'-dipalmitoyl-sn-glycero-3'-hydroxyphosphoryloxy)-äthylamid,
-N-Acetyl-muramyl-L-α-aminobutyryl-D-isoglutaminyl-L-alanin-2-(1'-palmitoyl-sn-glycero-3'-hydroxyphosphoryloxy)-äthylamid,
-N-Acetyl-muramyl-L-α-aminobutyryl-D-isoglutaminyl-L-alanin-2-(1'-O-palmitoyl-propandiol-3'-O-hydroxyphosphoryloxy)-äthylamid,
-N-Acetyl-muramyl-L-α-aminobutyryl-D-isoglutaminyl-L-alanin-2-(1',2'-dihexadecyl-rac-glycero-3'-hydroxyphosphoryloxy)-äthylamid,
-N-Acetyl-muramyl-L-α-aminobutyryl-D-isoglutaminyl-L-alanin-2-(1'-hexadecyl-rac-glycero-3'-hydroxyphosphoryloxy)-äthylamid,
-N-Acetyl-muramyl-L-α-aminobutyryl-D-isoglutaminyl-L-alanin-2-(1',3'-dipalmitoyl-glycero-2'-hydroxyphosphoryloxy)-äthylamid,
-N-Acetyl-muramyl-L-α-aminobutyryl-D-glutaminyl-L-alanin-2-(1',2'-dipalmitoyl-sn-glycero-3'-hydroxyphosphoryloxy)-äthylamid,
-N-Acetyl-muramyl-L-α-aminobutyryl-D-glutaminyl-L-alanin-2-(1'-palmitoyl-sn-glycero-3'-hydroxyphosphoryloxy)-äthylamid,
-N-Acetyl-muramyl-L-α-aminobutyryl-D-glutaminyl-L-alanin-2-(1'-O-palmitoyl-propandiol-3'-O-hydroxyphosphoryloxy)-äthylamid,
-N-Acetyl-muramyl-L-α-aminobutyryl-D-glutaminyl-L-alanin-2-(1',2'-dihexadecyl-rac-glycero-3'-hydroxyphosphoryloxy)-äthylamid,
-N-Acetyl-muramyl-L-α-aminobutyryl-D-glutaminyl-L-alanin-2-(1'-hexadecyl-rac-glycero-3'-hydroxyphosphoryloxy)-äthylamid,
-N-Acetyl-muramyl-L-α-aminobutyryl-D-glutaminyl-L-alanin-2-(1',3'-dipalmitoyl-glycero-2'-hydroxyphosphoryloxy)-äthylamid,
-N-Acetyl-desmethylmuramyl-L-alanyl-D-glutamyl-($C_\alpha$)-glycinamid-($C_\gamma$)-2-(1',2'-dipalmitoyl-sn-glycero-3'-hydroxyphosphoryloxy)-äthylamid,
-N-Acetyl-desmethylmuramyl-L-alanyl-D-glutymyl-($C_\alpha$)-glycinamid-($C_\gamma$)-L-alanin-2-(1',2'-dipalmitoyl-sn-glycero-3'-hydroxyphosphoryloxy)-äthylamid,
-N-Acetyl-desmethylmuramyl-L-alanyl-D-glutaminyl-L-alanin-2-(1',2'-dipalmitoyl-sn-glycero-3'-hydroxyphosphoryloxy)-äthylamid,
-N-Acetyl-desmethylmuramyl-L-alanyl-D-glutamin-2-(1',2'-dipalmitoyl-sn-glycero-3'-hydroxyphosphoryloxy)-äthylamid,
-N-Acetyl-desmethylmuramyl-L-seryl-D-glutamin-2-(1',2'-dipalmitoyl-sn-glycero-3'-hydroxyphosphoryloxy)-äthylamid,
-N-Acetyl-desmethylmuramyl-L-α-aminobutyryl-D-glutamin-2-(1',2'-dipalmitoyl-sn-glycero-3'-hydroxyphosphoryloxy)-äthylamid,
-N-Acetylmuramyl-L-α-aminobutyryl-D-glutamin-2-(1',2'-dipalmitoyl-sn-glycero-3'-hydroxyphosphoryloxy)-äthylamid,
-N-Acetyl-muramyl-L-valyl-D-isoglutamin-2-(1',2'-dipalmitoyl-sn-glycero-3'-hydroxyphosphoryloxy)-äthylamid,
-N-Acetyl-muramyl-L-valyl-D-isoglutaminyl-L-alanin-2-(1',2'-dipalmitoyl-sn-glycero-3'-hydroxyphosphoryloxy)-äthylamid,

-N-Propionyl-normuramyl-L-alanyl-D-isoglutamin-2-(1′,2′-dipalmitoyl-sn-glycero-3′-hydroxyphosphoryloxy)-äthylamid,
-N-Propionyl-normuramyl-L-alanyl-D-isoglutaminyl-L-alanin-2-(1′,2′-dipalmitoyl-sn-glycero-3′-hydroxyphosphoryloxy)-äthylamid,
-N-Acetyl-muramyl-L-prolyl-D-isoglutamin-2-(1′,2′-dipalmitoyl-sn-glycero-3′-hydroxyphosphoryloxy)-äthylamid,
-N-Acetyl-muramyl-L-prolyl-Di-isoglutaminyl-L-alanin-2-(1′,2′-dipalmitoyl-sn-glycero-3′-hydroxyphosphoryloxy)-äthylamid,
-N-Benzoyl-normuramyl-L-α-aminobutyryl-D-isoglutamin-2-(1′,2′-dipalmitoyl-sn-glycero-3′-hydroxyphosphoryloxy)-äthylamid,
-N-Benzoyl-normuramyl-L-α-aminobutyryl-D-isoglutaminyl-L-alanin-2-(1′,2-dipalmitoyl-sn-glycero-3′-hydroxyphosphoryloxy)-äthylamid,
-N-Acetyl-muramyl-L-threonyl-D-isoglutamin-2-(1′,2′-dipalmitoyl-sn-glycero-3′-hydroxyphosphoryloxy)-äthylamid,
-N-Acetyl-muramyl-L-threonyl-D-isoglutaminyl-L-alanin-2-(1′,2′-dipalmitoyl-sn-glycero-3′-hydroxyphosphoryloxy)-äthylamid,
-N-Acetyl-desmethylmuramyl-L-alanyl-D-isoglutamin-2-(1′-hexadecyl-sn-glycero-3′-hydroxyphosphoryloxy)-äthylamid,
-N-Acetyl-muramyl-L-α-aminobutyryl-D-isoglutaminyl-L-alanin-2-(1′,2′-dipalmitoyl-rac-glycero-3′-hydroxyphosphoryloxy)-äthylamid oder
-N-Benzoyl-muramyl-L-α-aminobutyryl-D-isoglutaminyl-L-alanin-2-(1′,2′-dipalmitoyl-sn-glycero-3′-hydroxyphosphoryloxy)-äthylamid.

**Patentansprüche für die Vertragsstaaten: BE, CH, DE, FR, GB, IT, LI, LU, NL, SE**

1. Muramylpeptide der Formel

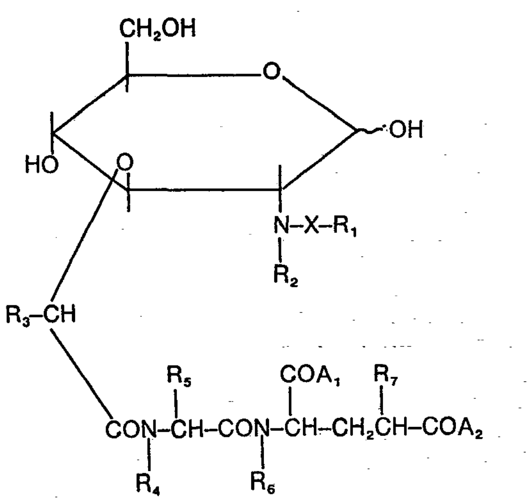

(D) (L) (D)

(I)

worin X Carbonyl oder Carbonyloxy, $R_1$ gegebenenfalls substituiertes Alkyl oder Aryl, $R_2$, $R_4$ und $R_6$ Wasserstoff oder Niederalkyl, $R_3$ Wasserstoff oder Niederalkyl, $R_5$ Wasserstoff, Niederalkyl, freies oder funktionell abgewandeltes Hydroxyniederalkyl, freies oder funktionell abgewandeltes Hydroxyniederalkyl, freies oder funktionell abgewandeltes Mercaptoniederalkyl, gegebenenfalls substituiertes Aminoniederalkyl, Cycloalkyl, Cycloalkyl-niederalkyl, gegebenenfalls substituiertes Aryl oder Aralkyl, stickstoffhaltiges Heterocyclyl oder Heterocyclylniederalkyl, oder $R_4$ und $R_5$ zusammen auch Alkylen mit 3–4 Kohlenstoffatomen, $R_7$ Wasserstoff oder gegebenenfalls verestertes oder amidiertes Carboxyl und einer der Reste $A_1$ und $A_2$ einen Rest der Formel

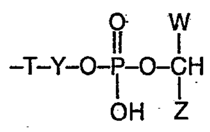

(II)

bedeuten, worin T für HN oder O steht, Y eine gegebenenfalls substituierte Alkylengruppe, die auch durch ein oder zwei Oxycarbonyl und/oder Iminocarbonyl unterbrochen sein kann, W Wasserstoff und Z eine 1,2-Dihydroxyäthyl- oder 2-Hydroxyäthylgruppe, in denen mindestens eine Hydroxygruppe mit einer gegebenenfalls ungesättigten aliphatischen Carbonsäure mit 12–90 C-Atomen verestert oder mit einem gegebenenfalls ungesättigten aliphatischen Alkohol mit 10–22 C-Atomen veräthert ist, oder W und Z je eine mit einer gegebenenfalls ungesättigten aliphatischen Carbonsäure mit 12–90 C-Atomen veresterte oder mit einem gegebenenfalls ungesättigten aliphatischen Alkohol mit 10–22 C-Atomen verätherte Hydroxymethylgruppe darstellen, und der andere der Reste $A_1$ und $A_2$ freies oder veräthertes Hydroxy, Amino, Niederalkylamino oder Carboxamidoniederalkyl-amino ist, und ihre Salze, wobei das Präfix «Nieder» Reste bis und mit 7 C-Atomen bezeichnet.

2. Muramylpeptide gemäss Patentanspruch 1, worin Y ein Niederalkylenrest mit 2–3 Kohlenstoffatomen ist, und ihre Salze.

3. Muramylpeptide gemäss Patentanspruch 1, worin Y einen Rest der Formel

$$-Y_1-COO-Y_2- \quad (IIIa)$$

$$-Y_1-OOC-Y_2- \quad (IIIb)$$

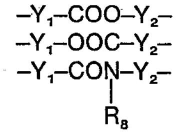

(IIIc)

bzw. 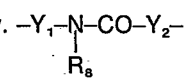 (IIId)

darstellt, worin einer der Reste $Y_1$ und $Y_2$ ein gegebenenfalls substituierter Niederalkylenrest und der andere ein gegebenenfalls substituierter Niederalkylenrest, der auch durch Oxycarbonyl oder $-N-R_8$-Iminocarbonyl unterbrochen sein kann, und $Y_1$ und $Y_2$ zusammen mehr als zwei Kohlenstoffatome aufweisen und $R_8$ Wasserstoff oder Niederalkyl ist, und ihre Salze.

4. Muramylpeptide der Formel I gemäss Patentanspruch 1, worin X Carbonyl, $R_1$ gegebenenfalls substituiertes Alkyl oder Aryl, $R_2$, $R_3$, $R_4$ und $R_6$ Wasserstoff oder Niederalkyl, $R_5$ Wasserstoff, gegebenenfalls durch Hydroxy, Niederalkoxy, Mercapto, Niederalkylmercapto oder Halogen substituiertes Niederalkyl, Cycloalkyl oder Cycloalkylniederalkyl, worin der Cycloalkylrest 4–6 Kohlenstoffatome enthält, gegebenenfalls substituiertes Phenyl oder Phenylniederalkyl, ein oder zwei Azaatome enthaltendes Heterocyclyl oder Heterocyclylniederalkyl oder $R_4$ und $R_5$ zusammen auch Alkylen mit 3–4 Kohlenstoffatomen, $R_7$ Wasserstoff und einer der Reste $A_1$ und $A_2$ einen Rest der Formel

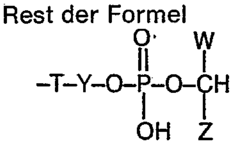 (II)

bedeuten, worin T für HN oder O steht, Y gegebenenfalls substituiertes Alkylen, das auch durch Carbonyloxy oder Carbonylimino unterbrochen sein kann, W Wasserstoff und Z eine 1,2-Dihydroxyäthyl- oder 2-Hydroxyäthylgruppe darstellen, in der mindestens eine der Hydroxygruppen in der im Anspruch 1 angegebenen Weise verestert oder veräthert ist, und der andere der Reste $A_1$ und $A_2$ Hydroxy, Niederalkoxy, Amino, Niederalkylamino oder Carboxamidoniederalkylamino ist, und ihre Salze.

5. Muramylpeptide der Formel I gemäss Patentanspruch 1, worin X Carbonyl, $R_1$ gegebenenfalls durch Hydroxy, Niederalkoxy oder Halogen substituiertes Niederalkyl oder gegebenenfalls durch Hydroxy, Niederalkoxy, Niederalkyl oder Halogen substituiertes Phenyl, $R_2$, $R_4$ und $R_6$ Wasserstoff, $R_3$ Wasserstoff oder Niederalkyl, $R_5$ Wasserstoff, gegebenenfalls durch Hydroxy, Niederalkoxy, Mercapto, Niederalkylmercapto oder Halogen substituiertes Niederalkyl mit 1–3 Kohlenstoffatomen, Cycloalkyl oder Cycloalkylniederalkyl, worin der Cycloalkylrest 4–6 Kohlenstoffatome und der Niederalkylrest 1–3 Kohlenstoffatome enthält, gegebenenfalls durch Hydroxy, Niederalkoxy oder Halogen substituiertes Phenyl oder Phenylniederalkyl mit 1–3 Kohlenstoffatomen im Niederalkylrest, ein oder zwei Azaatome enthaltendes Heterocyclyl oder Heterocyclylniederalkyl mit 5–6 Ringgliedern und 1–3 Kohlenstoffatomen im Niederalkylrest oder $R_4$ und $R_5$ zusammen auch Alkylen mit 3–4 Kohlenstoffatomen, $R_7$ Wasserstoff und einer der Reste $A_1$ und $A_2$ einen Rest der Formel

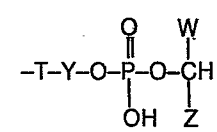 (II)

bedeuten, worin T für HN oder O steht, Y gegebenenfalls substituiertes Niederalkylen oder einen Rest der Formel

$$-Y_1-COO-Y_2- \quad (IIIa)$$

oder

$$-Y_1-CONH-Y_2- \quad (IVc)$$

bedeutet, worin $Y_1$ und $Y_2$ je gegebenenfalls substituiertes Niederalkylen sind, W Wasserstoff und Z eine 1,2-Dihydroxyäthyl- oder 2-Hydroxyäthylgruppe, in der mindestens eine der Hydroxygruppen mit einer gegebenenfalls ein- oder zweifach ungesättigten aliphatischen Carbonsäure mit 16–22 Kohlenstoffatomen oder mit einer natürlichen oder synthetischen Mycolsäure verestert oder mit einem gegebenenfalls ein- oder zweifach ungesättigten, aliphatischen Alkohol mit 12–18 Kohlenstoffatomen veräthert ist, darstellen, und der andere der Reste $A_1$ und $A_2$ Hydroxy, Niederalkoxy, Amino, Niederalkylamino oder Carboxamidoniederalkylamino ist, und ihre Salze.

6. Muramylpeptide der Formel I gemäss Patentanspruch 1, worin X Carbonyl, $R_1$ Niederalkyl mit 1–3 Kohlenstoffatomen oder Phenyl, $R_2$, $R_4$ und $R_6$ Wasserstoff, $R_3$ Wasserstoff oder Niederalkyl mit 1–3 Kohlenstoffatomen, $R_5$ Wasserstoff, gegebenenfalls durch Hydroxy, Methoxy, Mercapto, Methylmercapto oder Halogen substituiertes Niederalkyl mit 1–3 Kohlenstoffatomen, gegebenenfalls durch Hydroxy, Methoxy oder Halogen substituiertes Phenyl oder Phenylmethyl, ein oder zwei Azaatome enthaltendes Heterocyclyl oder Heterocyclylmethyl mit 5 Ringgliedern oder $R_4$ und $R_5$ zusammen auch Trimethylen, $R_7$ Wasserstoff und einer der Reste $A_1$ und $A_2$ einen Rest der Formel

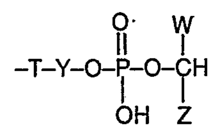 (II)

bedeuten, worin T für HN oder O steht, Y Niederalkylen mit 2–3 Kohlenstoffatomen oder einen Rest der im Anspruch 5 abgebildeten Formeln (IIIa) oder IVc) bedeutet, worin $Y_1$ und $Y_2$ unabhängig voneinander je Niederalkylen mit 1–3 Kohlenstoffatomen bedeuten, das unsubstituiert oder durch gegebenenfalls durch Hydroxy, Niederalkoxy, Mercapto oder Niederalkylmercapto substituiertes Niederalkyl mit 1–3 Kohlenstoffatomen oder durch gegebenenfalls durch Hydroxy, Methoxy oder Halogen substituiertes Phenyl oder Phenylniederalkyl oder durch ein oder zwei Azaatome enthaltendes Heterocyclyl oder Heterocyclylniederalkyl mit 5–6 Ringgliedern und 1–3 Kohlenstoffatomen im Niederalkylrest substituiert ist, W Wasserstoff und Z eine 1,2-Dihydroxyäthyl- oder 2-Hydroxyäthylgruppe sind, in der mindestens eine der Hydroxygruppen mit einer gegebenenfalls ein- oder zweifach ungesättigten aliphatischen Carbonsäure mit 16–20 Kohlenstoffatomen verestert oder mit einem gegebenenfalls ein- oder zweifach ungesättigten aliphatischen Alkohol mit 12–18 Kohlenstoffatomen veräthert ist, und der andere der Reste $A_1$ und $A_2$ Hydroxy, Niederalkoxy, Amino, Niederalkylamino oder Carboxamidoniederalkylamino ist, und ihre Salze.

7. Muramylpeptide der in Anspruch 1 dargestellten Formel I, worin X Carbonyl oder Carbonyloxy, $R_1$ Niederalkyl mit 1–3 Kohlenstoffatomen oder Phenyl, $R_2$, $R_4$ und $R_6$ Wasserstoff, $R^3$ Wasserstoff oder Niederalkyl mit 1–3 Kohlenstoffatomen,

$R^5$ Wasserstoff oder Niederalkyl, $R^7$ Wasserstoff, $A_1$ Amino, Niederalkylmaino, Hydroxy oder Niederalkoxy und $A_2$ einen Rest der in Anspruch 1 dargestellten Formel (II), worin T für NH oder O steht, Y Niederalkylen mit 2–3 Kohlenstoffatomen oder einen Rest der Formel

$$-CH_2-CO-NH-CH_2-CH_2-$$

bedeuten, W für Wasserstoff und Z für eine 1,2-Dihydroxyäthyl- oder 2-Hydroxyäthyl-Gruppe stehen, in der eine oder zwei Hydroxygruppen mit gleichen oder verschiedenen gegebenenfalls ein- oder zweifach ungesättigten Alkancarbonsäuren mit 16–22 Kohlenstoffatomen verestert oder mit einem gegebenenfalls ein- oder zweifach ungesättigten Alkanol mit 12–18 Kohlenstoffatomen veräthert sind oder worin zwei vicinale Hydroxygruppen unter Bildung eines Dioxolanringes formal mit einem Bisniederalkylketon ketalisiert oder mit einem unsubstituierten aliphatischen Aldehyd mit bis zu 20 C-Atomen acetalisiert sind, oder worin W und Z je eine Hydroxymethylgruppe darstellen, die mit einer gegebenenfalls ein- oder zweifach ungesättigten Alkancarbonsäure mit 16–22 Kohlenstoffatomen verestert oder mit einem gegebenenfalls ein- oder zweifach ungesättigten Alkanol mit 12–18 Kohlenstoffatomen veräthert ist, und ihre Salze, wobei das Präfix «Nieder» Reste bis und mit 7 C-Atomen bezeichnet.

8. Verbindungen gemäss Anspruch 7, worin die Bedeutungen für $A_1$ und $A_2$ vertauscht sind, und ihre Salze.

9. N-Acetylmuramyl-L-alanyl-D-iso-glutamin-2-(1′,2′-dipalmitoyl-sn-glycero-3′-hydroxyphosphoryloxy)-äthylamid und seine Salze nach Anspruch 1.

10. N-Acetyl-desmethylmuramyl-L-alanyl-D-isoglutamin-2-(1′,2′-dipalmitoyl-sn-glycero-3′-hydroxyphosphoryloxy)-äthyl-amid und seine Salze nach Anspruch 1.

11. N-Acetylmuramyl-L-alanyl-D-iso-glutaminyl-γ-oxy-essigsäure-2-(1′,2′-di-palmitoyl-sn-glycero-3′-hydroxyphosphoryl-oxy)-äthylamid und seine Salze nach Anspruch 1.

12. N-Acetylmuramyl-L-alanyl-D-iso-glutaminyl-L-alanin-2-(1′,2′-dipalmitoyl-sn-glycero-3′-hydroxyphosphoryloxy)-äthylamid und seine Salze nach Anspruch 1.

13. N-Acetyl-desmethylmuramyl-L-alanyl-D-isoglutamin-2-(1′,2′-dihexadecyl-sn-glycero-3′-hydroxyphosphoryloxy)-äthylamid und seine Salze nach Anspruch 1.

14. N-Acetyl-desmethylmuramyl-L-alanyl-D-isoglutamin-2-(1′-hexadecyl-sn-glycero-3′-hydroxyphosphoryloxy)-äthyl-amid und seine Salze nach Anspruch 1.

15. N-Acetyl-desmethylmuramyl-L-alanyl-D-isoglutamin-2-(3′-palmitoyl-rac-glycero-1′-hydroxyphosphoryloxy)-äthyl-amid und seine Salze nach Anspruch 1.

16. N-Acetyl-desmethylmuramyl-L-alanyl-D-isoglutamin-2-(1′-palmitoyl-2′-oleoyl-sn-glycero-3′-hydroxyphosphoryloxy)-äthylamid und seine Salze nach Anspruch 1.

17. N-Acetyl-desmethylmuramyl-L-alanyl-D-isoglutamin-2-(1′-O-palmitoyl-propandiol-3′-O-hydroxyphosphoryloxy)-äthylamid und seine Salze nach Anspruch 1.

18. N-Acetyl-muramyl-L-α-aminobutyryl-D-isoglutaminyl-L-alanin-2-(1′,2′-di-palmitoyl-sn-glycero-3′-hydroxyphosphoryloxy)-äthylamid und seine Salze nach Anspruch 1.

19. N-Acetyl-muramyl-L-α-aminobutyryl-D-isoglutaminyl-L-alanin-2-(1′,2′-dipalmitoyl-rac-glycero-3′-hydroxyphosphoryloxy)-äthylamid und seine Salze nach Anspruch 1.

20. N-Benzoyl-muramyl-L-α-aminobutyryl-D-isoglutaminyl-L-alanin-2-(1′,2′-dipalmitoyl-sn-glycero-3′-hydroxyphosphoryloxy)-äthylamid und seine Salze nach Anspruch 1.

21. N-Propionyl-desmethylmuramyl-L-alanyl-D-isoglutaminyl-L-alanin-1-(1′,2′-dipalmitoyl-sn-glycero-3′-hydroxyphosphoryloxy)-äthylamid und seine Salze nach Anspruch 1.

22. N-Acetyl-muramyl-L-valyl-D-iso-glutaminyl-L-alanin-2-(1′,2′-dipalmitoyl-sn-glycero-3′-hydroxyphosphoryloxy)-äthylamid und seine Salze nach Anspruch 1.

23. N-Benzoyl-desmethylmuramyl-L-α-aminobutyryl-D-isoglutaminyl-L-alanin-2-(1′,2′-dipalmitoyl-sn-glycero-3′-hydroxyphosphoryloxy)-äthylamid und seine Salze nach Anspruch 1.

24. N-Acetyl-desmethylmuramyl-L-alanyl-D-glutamyl-($C_\alpha$)-glycinamid-($C_\gamma$)-L-alanin-2-(1′,2′-dipalmitoy-sn-glycero-3′-hydroxyphosphoryloxy)-äthylamid und seine Salze nach Anspruch 1.

25. N-Acetyl-muramyl-L-alanyl-D-iso-glutaminyl-L-alanin-2-(3′-palmitoyl-rac-glycero-1′-hydroxyphosphoryloxy)-äthylamid und seine Salze nach Anspruch 1.

26. N-Acetyl-muramyl-L-alanyl-D-iso-glutaminyl-L-alanin-2-(1′,2′-hexadecyliden-sn-glycero-3′-hydroxyphosphoryloxy)-äthylamid und seine Salze.

27. N-Acetyl-desmethylmuramyl-L-alanyl-D-isoglutaminyl-L-alanin-2-(1′,2′-hexadecyliden-sn-glycero-3′-hydroxyphosphoryloxy)-äthylamid und seine Salze.

28. Die pharmazeutisch verwendbaren Salze der Verbindungen nach den Ansprüchen 1–27.

29. Die Natriumsalze der Verbindungen nach den Ansprüchen 1–27.

30. Pharmazeutische Präparate enthaltend eine der in den Patentansprüchen 1–29 genannten Verbindungen zusammen mit einem pharmazeutischen Trägermaterial.

31. Immunisierungsmittel enthaltend eine der in einem der Ansprüche 1–29 genannten Verbindungen zusammen mit einem geeigneten Trägermaterial.

32. Eine der in den Ansprüchen 1–29 genannten Verbindungen zur Verwendung als Arzneimittel.

33. Verwendung der in den Ansprüchen 1–29 genannten Verbindungen zur Herstellung eines Immuniserungsmittels bzw. Impfstoffes.

34. Verfahren zur Herstellung von Muramylpeptiden der Formel I gemäss einem der Patentan-

sprüche 1–29 und ihren Salzen, dadurch gekennzeichnet, dass man in an sich bekannter Weise

a) eine Verbindung der Formel

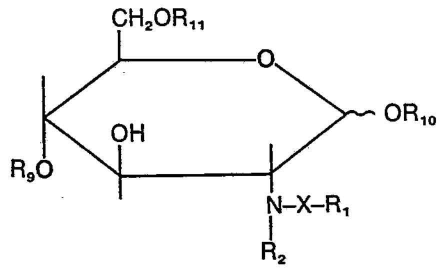

(V)

worin X, $R_1$ und $R_2$ die obengenannte Bedeutung haben, wobei gegebenenfalls darin vorhandene Hydroxygruppen mit einer leicht abspaltbaren Schutzgruppe geschützt sind, und $R_9$, $R_{10}$ und $R_{11}$ eine leicht abspaltbare Schutzgruppe bedeuten, oder eine Metallverbindung davon mit einer Verbindung der Formel

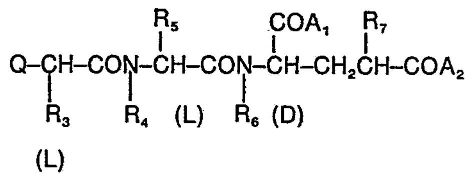

(VI)

oder (D,L)

umsetzt, worin Q eine reaktionsfähige veresterte Hydroxygruppe darstellt, $R_3$, $R_4$, $R_5$, $R_6$, $R_7$, $A_1$ und $A_2$ die oben angegebene Bedeutung haben und gegebenenfalls darin vorhandene Hydroxygruppen durch eine leicht abspaltbare Schutzgruppe geschützt sind, und vorhandene Schutzgruppen abspaltet, oder

b) eine Verbindung der Formel

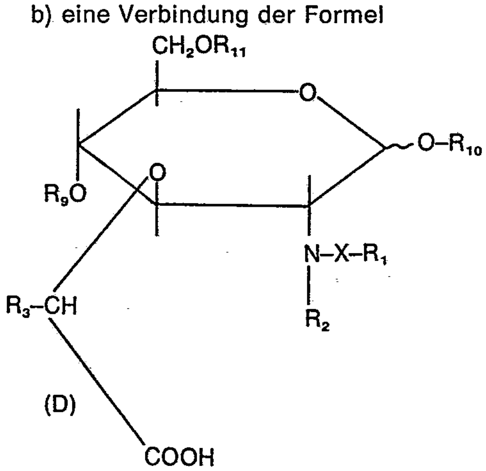

(VII)

worin X, $R_1$, $R_2$ und $R_3$ die obengenannte Bedeutung besitzen und $R_9$, $R_{10}$ und $R_{11}$ für Wasserstoff oder eine leicht abspaltbare Schutzgruppe stehen, oder ein Derivat davon, mit einer Verbindung der Formel

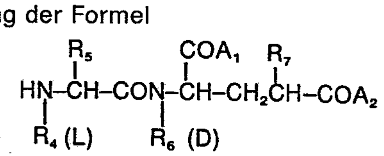

(VIII)

worin $R_4$, $R_5$, $R_6$, $R_7$, $A_1$ und $A_2$ die obengenannte Bedeutung besitzen, mit der Massgabe, dass in diesen Resten vorhandene Carboxy- und, wenn erwünscht, freie Hydroxygruppen durch leicht abspaltbare Schutzgruppen geschützt sind, oder einem Derivat davon kondensiert und vorhandene Schutzgruppen abspaltet, oder

c) eine Verbindung der Formel

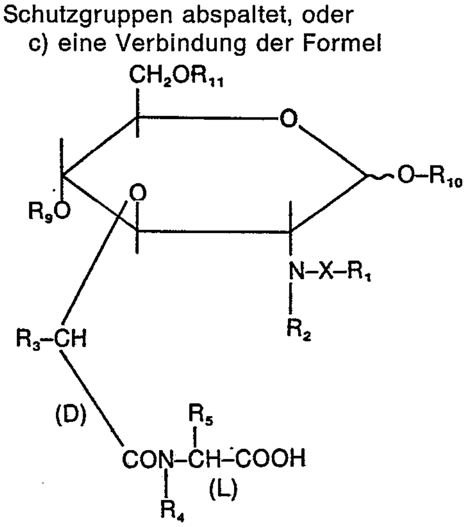

(IX)

worin X, $R_1$, $R_2$, $R_3$, $R_4$ und $R_5$ die obengenannte Bedeutung haben, mit der Massgabe, dass darin enthaltene freie Hydroxygruppen gegebenenfalls mit einer leicht abspaltbaren Schutzgruppe geschützt sind, und $R_9$, $R_{10}$ und $R_{11}$ Wasserstoff oder leicht abspaltbare Schutzgruppen darstellen, oder Derivate davon mit einer Verbindung der Formel

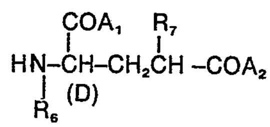

(X)

worin $R_6$, $R_7$, $A_1$ und $A_2$ die obgenannte Bedeutung haben, mit der Massgabe, dass in den Resten $R_7$, $A_1$ und $A_2$ vorhandene freie Carboxylgruppen durch leicht abspaltbare Schutzgruppen geschützt sind, kondensiert und vorhandene Schutzgruppen abspaltet, oder

d) eine Verbindung der Formel

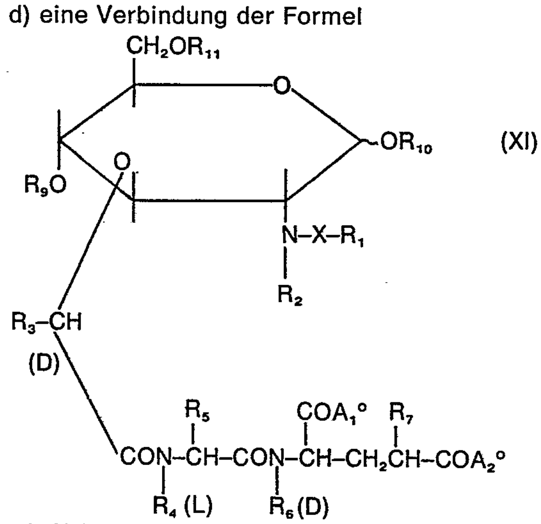

(XI)

worin X, $R_1$, $R_2$, $R_3$, $R_4$, $R_5$, $R_6$ und $R_7$ die obgenannte Bedeutung haben, $R_9$, $R_{10}$ und $R_{11}$ Wasserstoff oder eine leicht abspaltbare Schutzgruppe darstellen und einer der Reste $A_1^\circ$ und $A_2^\circ$ eine aktivierte Hydroxygruppe darstellt und der andere veräthertes Hydroxy, Amino, Niederalkylamino oder Carboxamidoniederalkylamino ist, mit einer Verbindung der Formel

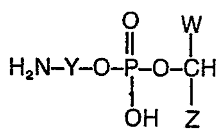

(XII)

worin Y, W und Z die obgenannte Bedeutung besitzen, kondensiert und vorhandene Schutzgruppen abspaltet, oder

e) eine Verbindung der Formel

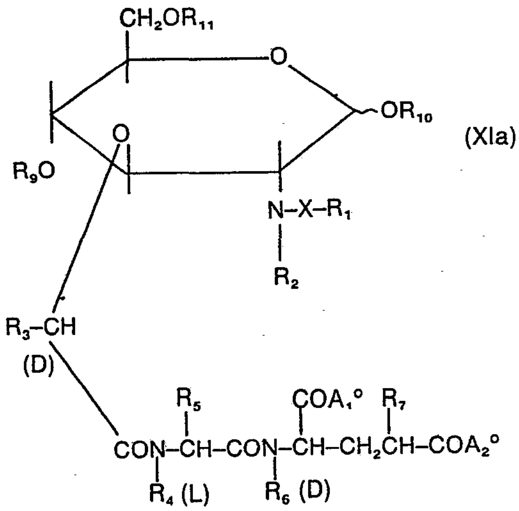

(XIa)

worin X, $R_1$, $R_2$, $R_3$, $R_4$, $R_5$, $R_6$ und $R_7$ die obengenannte Bedeutung haben, $R_9$, $R_{10}$ und $R_{11}$ Wasserstoff oder eine leicht abspaltbare Schutzgruppe darstellen und einer der Reste $A_1°$ und $A_2°$ eine Hydroxygruppe darstellt und der andere veräthertes Hydroxy, Amino, Niederalkylamino oder Carboxamidoniederalkylamino ist, mit einer Verbindung der Formel

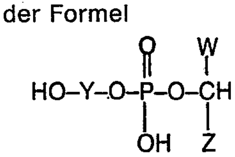

(XIIa)

worin Y, W und Z die obgenannte Bedeutung besitzen, wobei die Säure XIa oder der Alkohol XIIa in reaktionsfähiger Form vorliegt, verestert und gegebenenfalls vorhandene Schutzgruppen abspaltet, oder

f) in einer Verbindung der Formel

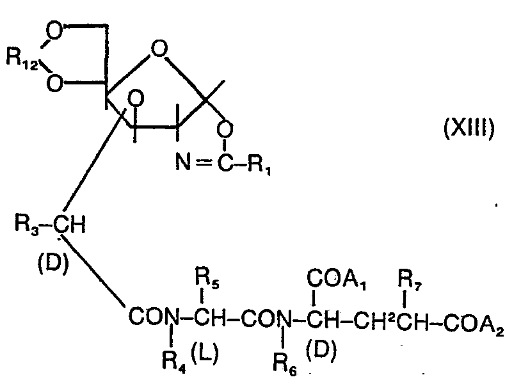

(XIII)

worin $R_1$, $R_3$, $R_4$, $R_5$, $R_6$, $R_7$, $A_1$ und $A_2$ die obengenannte Bedeutung haben und $R_{12}$ eine Alkyliden- oder Cycloalkilidengruppe ist, den Oxazolin- und den Dioxolanring sauer aufspaltet, und gegebenenfalls vorhandene Schutzgruppen abspaltet, oder

g) eine Verbindung der Formel

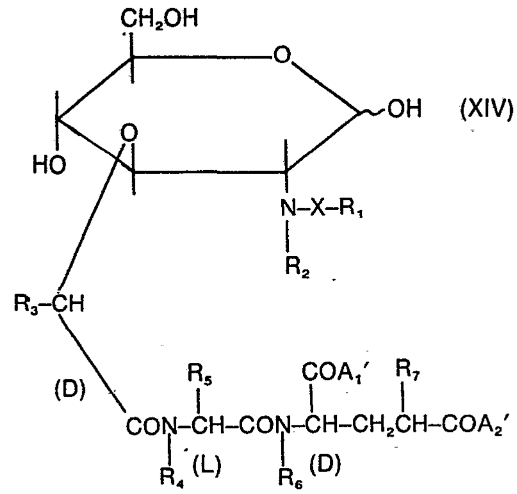

(XIV)

worin X, $R_1$, $R_2$, $R_3$, $R_4$, $R_5$, $R_6$ und $R_7$ die obengenannten Bedeutungen haben, wobei darin vorhandene Hydroxygruppen gegebenenfalls durch leicht abspaltbare Schutzgruppen geschützt sind, einer der Reste $A_1'$ und $A_2'$ einen Rest der Formel

$$-T-Y_1-M_1 \qquad (XV)$$

bedeutet, worin T die obengenannte Bedeutung hat, $Y_1$ gegebenenfalls substituiertes Alkylen, das durch eine Oxycarbonyl- oder Iminocarbonylgruppe unterbrochen sein kann, wobei darin vorhandene Hydroxygruppen gegebenenfalls durch leicht abspaltbare Schutzgruppen geschützt sind, und $M_1$ eine freie Aminogruppe, eine Carbonsäuregruppe oder ein aktiviertes Derivat dieser beiden Gruppen bedeuten, und der andere der Reste $A_1'$ und $A_2'$ veräthertes Hydroxy oder Amino, Niederalkylamino oder Carboxamidoniederalkylamino ist, mit einer Verbindung der Formel

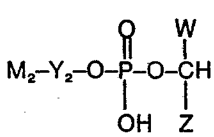

(XVI)

worin $M_2$ eine freie Aminogruppe oder ein aktiviertes Derivat davon bedeutet, wenn $M_1$ für eine Carbonsäuregruppe oder ein aktiviertes Derivat derselben steht, oder worin $M_2$ eine Carbonsäure oder ein aktiviertes Derivat derselben bedeutet, wenn $M_1$ für eine freie Aminogruppe oder ein aktiviertes Derivat davon steht, $Y_2$ gegebenenfalls substituiertes Alkylen, das durch eine Oxycarbonyl- oder Iminocarbonylgruppe unterbrochen sein kann, bedeutet, mit der Massgabe, dass die über eine Iminocarbonylgruppe miteinander verbundenen Reste $Y_1$ und $Y_2$ dem obengenannten Rest Y entsprechen, und W und Z die obengenannten Bedeutungen haben, wobei in den Resten $Y_2$, W und Z vorhandene Hydroxygruppen gegebenenfalls durch leicht abspaltbare Schutzgruppen geschützt sind, kondensiert und gegebenenfalls vorhandene Schutzgruppen abspaltet, oder

h) eine Verbindung der Formel

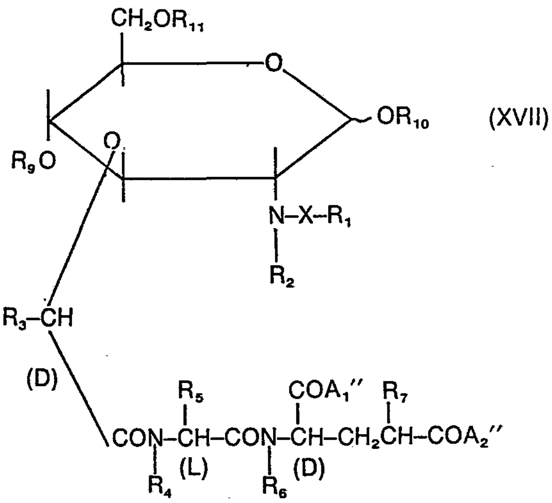

(XVII)

worin X, $R_1$, $R_2$, $R_3$, $R_4$, $R_5$, $R_6$ und $R_7$ die obengenannten Bedeutungen haben, $R_9$, $R_{10}$ und $R_{11}$ leicht abspaltbare Schutzgruppen darstellen, einer der Reste $A_1''$ und $A_2''$ einen Rest der Formel

$$-T-Y_1-M_3 \qquad (XVIII)$$

bedeutet, worin T die obengenannte Bedeutung hat, $Y_1$ gegebenenfalls substituiertes Alkylen, das durch eine Oxycarbonyl- oder Iminocarbonylgruppe unterbrochen sein kann, wobei darin vorhandene Hydroxygruppen gegebenenfalls durch leicht abspaltbare Schutzgruppen geschützt sind, und $M_3$ eine freie Hydroxy- oder Carboxylgruppe, die gegebenenfalls in reaktionsfähiger Form vorliegt, bedeuten, und der andere der Reste $A_1''$ und $A_2''$ veräthertes Hydroxy, Amino, Niederalkylamino oder Carboxamidoniederalkylamino ist, mit einer Verbindung der Formel

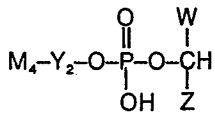

(XIX)

worin $M_4$ eine gegebenenfalls in reaktionsfähiger Form vorliegende Hydroxygruppe bedeutet, wenn $M_3$ eine gegebenenfalls in reaktionsfähiger Form vorliegende Carboxylgruppe bedeutet, oder worin $M_4$ eine gegebenenfalls in reaktionsfähiger Form vorliegende Carboxylgruppe bedeutet, wenn $M_3$ eine gegebenenfalls in reaktionsfähiger Form vorliegende Hydroxygruppe bedeutet, $Y_2$ gegebenenfalls substituiertes Alkylen, das durch eine Oxycarbonyl- oder Iminocarbonylgruppe unterbrochen sein kann, bedeutet, mit der Massgabe, dass die über eine Oxycarbonylgruppe miteinander verbundenen Reste $Y_1$ und $Y_2$ dem obengenannten Rest Y entsprechen, und W und Z die obengenannte Bedeutung haben, wobei in den Resten $Y_2$, W und Z vorhandene Hydroxygruppen gegebenenfalls durch leicht abspaltbare Schutzgruppen geschützt sind, verestert und gegebenenfalls vorhandene Schutzgruppen abspaltet, oder

i) eine Verbindung der Formel

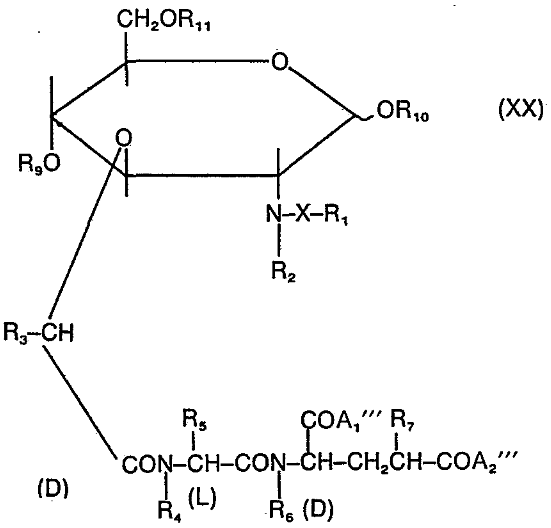

(XX)

worin X, $R_1$, $R_2$, $R_3$, $R_4$, $R_5$, $R_6$ und $R_7$ die obengenannten Bedeutungen haben, wobei gegebenenfalls darin vorhandene Hydroxygruppen mit leicht abspaltbaren Schutzgruppen geschützt sind, $R_9$, $R_{10}$ und $R_{11}$ leicht abspaltbare Schutzgruppen bedeuten und einer der Reste $A_1'''$ und $A_2''''$ für –T–Y–OH steht, worin Y und T die obgenannten Bedeutungen besitzen, wobei gegebenenfalls darin vorhandene Hydroxygruppen mit leicht abspaltbaren Schutzgruppen geschützt sind, und der andere der Reste $A_1'''$ und $A_2'''$ für veräthertes Hydroxy, Amino, Niederalkylamino oder Carboxamidoniederalkylamino steht, mit einer den Rest der Formel

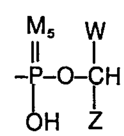

(XXI)

worin = $M_5$ ein Elektronenpaar oder Oxo bedeutet und W und Z die obengenannten Bedeutungen haben, wobei gegebenenfalls darin vorhandene Hydroxygruppen mit leicht abspaltbaren Schutzgruppen geschützt sind, abgebenden Verbindung umsetzt, falls = $M_5$ ein Elektronenpaar ist, mit einem schwachen Oxydationsmittel oxydiert, und vorhandene Schutzgruppen abspaltet, oder

k) eine Verbindung der Formel

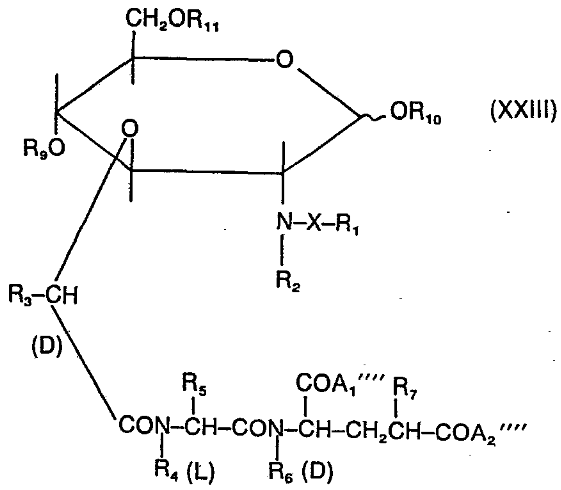

(XXIII)

worin X, $R_1$, $R_2$, $R_3$, $R_4$, $R_5$, $R_6$ und $R_7$ die obengenannten Bedeutungen besitzen, wobei gegebenenfalls darin vorhandene Hydroxygruppen mit leicht abspaltbaren Schutzgruppen geschützt sind, $R_9$, $R_{10}$ und $R_{11}$ leicht abspaltbare Schutzgruppen bedeuten und einer der Reste $A_1''''$ und $A_2''''$ für

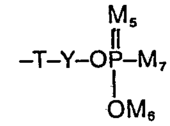

(XXIV)

worin T und Y die obengenannte Bedeutung besitzen, wobei gegebenenfalls darin vorhanden Hydroxygruppen mit leicht abspaltbaren Schutzgruppen geschützt sind, und worin $= M_5$ ein Elektronenpaar oder Oxo, $M_6$ eine leicht abspaltbare Schutzgruppe und $M_7$ freies oder in reaktionsfähiger Form vorliegendes Hydroxy bedeuten, und der andere der Reste $A_1''''$ und $A_2''''$ für freies oder veräthertes Hydroxy, Amino, Niederalkylamino oder Carboxamidoniederalkylamino steht, mit einer Verbindung der Formel

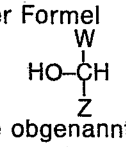

(XXV)

worin W und Z die obgenannte Bedeutung besitzen, wobei gegebenenfalls darin vorhandene Hydroxygruppen mit leicht abspaltbaren Schutzgruppen geschützt sind, umsetzt, falls $= M_5$ ein Elektronenpaar ist, mit einem schwachen Oxydationsmittel oxydiert, und vorhandene Schutzgruppen abspaltet, oder

l) eine Verbindung der Formel

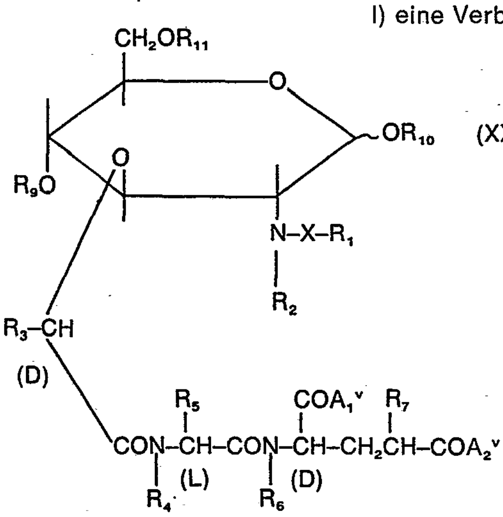

(XXVI)

worin X, $R_1$, $R_2$, $R_3$, $R_4$, $R_5$, $R_6$ und $R_7$ die obengenannten Bedeutungen haben, wobei gegebenenfalls darin vorhandene Hydroxygruppen mit leicht abspaltbaren Schutzgruppen geschützt sind, $R_9$, $R_{10}$ und $R_{11}$ leicht abspaltbare Schutzgruppen bedeuten und einer der Reste $A_1^{v}$ und $A_2^{v}$ für einen Rest der Formel XXVII

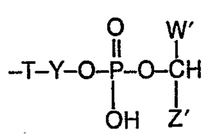

(XXVII)

steht, worin T und Y die obengenannten Bedeutungen haben, wobei in einem Rest der Formel XXVII vorhandene freie funktionelle Gruppen mit Ausnahme der an der Reaktion teilnehmenden Hydroxygruppe(n) gegebenenfalls durch leicht abspaltbare Schutzgruppen geschützt sind, und W′ und Z′ die obengenannte Bedeutung von W bzw. Z haben, jedoch mit der Massgabe, dass mindestens eine Hydroxygruppe in dem Rest 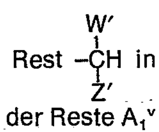 in freier Form vorliegt, und der andere der Reste $A_1^{v}$ und $A_2^{v}$ für freies oder veräthertes Hydroxy, Amino, Niederalkylamino oder Carboxamidoniederalkyl-amino steht, mit einer gegebenenfalls ungesättigten aliphatischen Carbonsäure mit 12–90 C-Atomen oder einem reaktionsfähigen Derivat davon verestert, oder mit einem gegebenenfalls ungesättigten aliphatischen Alkohol mit 10–22 C-Atomen oder einem reaktionsfähigen Derivat davon veräthert und die vorhandenen Schutzgruppen abspaltet, und, wenn erwünscht, eine erhaltene Verbindung der Formel I in ihre Salze überführt, wobei das vorstehend verwendete Präfix «Nieder-» Reste bis und mit 7 C-Atomen bezeichnet und der vorstehend verwendete Ausdruck «obengenannte Bedeutung» die Bedeutung des jeweiligen Restes in dem Stoffanspruch angibt, auf den man diesen Verfahrensanspruch bezieht.

35. Die nach dem Verfahren des Anspruchs 34 erhältlichen Verbindungen:

**Patentansprüche für den Vertragsstaat: AT**

1. Verfahren zur Herstellung von Muramylpeptiden der Formel

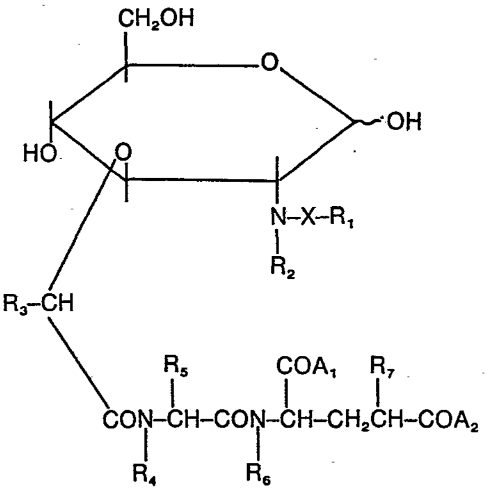

(I)

(D) (L) (D)

worin X Carbonyl oder Carbonyloxy, $R_1$ gegebenenfalls substituiertes Alkyl oder Aryl, $R_2$, $R_4$ und $R_6$ Wasserstoff oder Niederalkyl, $R_3$ Wasserstoff oder Niederalkyl, $R_5$ Wasserstoff, Niederalkyl, freies oder funktionell abgewandeltes Hydroxyniederalkyl, freies oder funktionell abgewandeltes Mercaptoniederalkyl, gegebenenfalls substituiertes Aminoniederalkyl, Cycloalkyl, Cycloalkylniederalkyl, gegebenenfalls substituiertes Aryl oder Aralkyl, stickstoffhaltiges Heterocyclyl oder Heterocyclylniederalkyl, oder $R_4$ und $R_5$ zusammen auch Alkylen mit 3–4 Kohlenstoffatomen, $R_7$ Wasserstoff oder gegebenenfalls verestertes oder amidiertes Carboxyl und einer der Reste $A_1$ und $A_2$ einen Rest der Formel

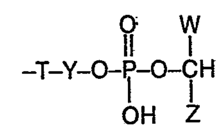

(II)

bedeuten, worin T für HN oder O steht, Y eine gegebenenfalls substituierte Alkylengruppe, die auch durch ein oder zwei Oxycarbonyl und/oder Iminocarbonyl unterbrochen sein kann, W Wasserstoff und Z eine 1,2-Dihydroxyäthyl- oder 2-Hydroxyäthylgruppe, in denen mindestens eine Hydroxygruppe mit einer gegebenenfalls ungesättigten aliphatischen Carbonsäure mit 12–90 C-Atomen verestert oder mit einem gegebenenfalls ungesättigten aliphatischen Alkohol mit 10–22 C-Atomen veräthert ist, oder W und Z je eine mit einer gegebenenfalls ungesättigten aliphatischen Carbonsäure mit 12–90 C-Atomen veresterte oder mit einem gegebenenfalls ungesättigten aliphatischen Alkohol mit 10–22 C-Atomen verätherte Hydroxymethylgruppe darstellen, und der andere der Reste $A_1$ und $A_2$ freies oder veräthertes Hydroxy, Amino, Niederalkylamino oder Carboxamidoniederalkyl-amino ist, oder ihren Salzen, dadurch gekennzeichnet, dass man in an sich bekannter Weise

a) eine Verbindung der Formel

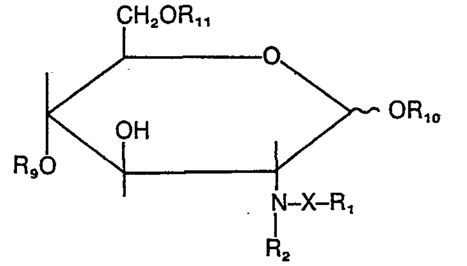

(V)

worin X, $R_1$ und $R_2$ die obengenannte Bedeutung haben, wobei gegebenenfalls darin vorhandene Hydroxygruppen mit einer leicht abspaltbaren Schutzgruppe geschützt sind, und $R_9$, $R_{10}$ und $R_{11}$ eine leicht abspaltbare Schutzgruppe bedeuten, oder eine Metallverbindung davon mit einer Verbindung der Formel

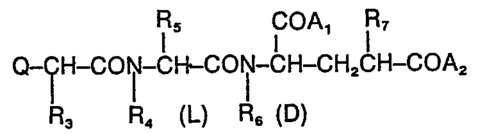

(VI)

(L)

oder (D,L)

umsetzt, worin Q eine reaktionsfähige veresterte Hydroxygruppe darstellt, $R_3$, $R_4$, $R_5$, $R_6$, $R_7$, $A_1$ und $A_2$ die oben angegebene Bedeutung haben und gegebenenfalls darin vorhandene Hydroxygruppen durch eine leicht abspaltbare Schutzgruppe geschützt sind, und vorhandene Schutzgruppen abspaltet, oder

b) eine Verbindung der Formel

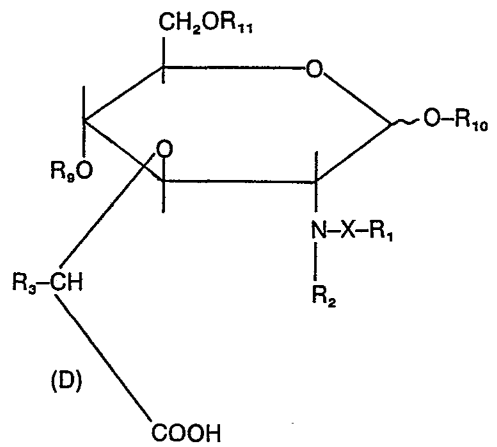

(VII)

worin X, $R_1$, $R_2$ und $R_3$ die obengenannte Bedeutung besitzen und $R_9$, $R_{10}$ und $R_{11}$ für Wasserstoff oder eine leicht abspaltbare Schutzgruppe stehen, oder ein Derivat davon, mit einer Verbindung der Formel

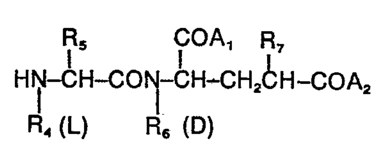

(VIII)

worin $R_4$, $R_5$, $R_6$, $R_7$, $A_1$ und $A_2$ die obengenannte Bedeutung besitzen, mit der Massgabe, dass in diesen Resten vorhandene Carboxy- und, wenn erwünscht, freie Hydroxygruppen durch leicht abspaltbare Schutzgruppen geschützt sind, oder einem Derivat davon kondensiert und vorhandene Schutzgruppen abspaltet, oder

c) eine Verbindung der Formel

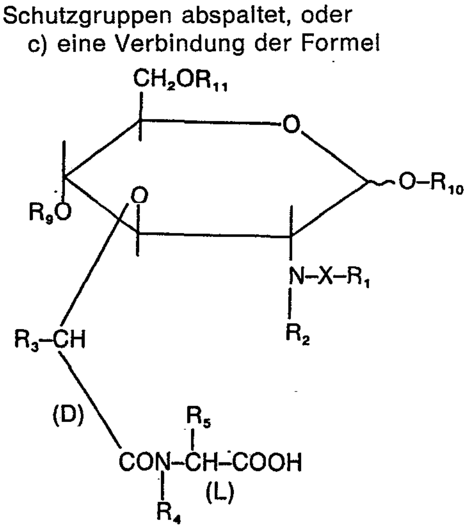

(IX)

worin X, $R_1$, $R_2$, $R_3$, $R_4$ und $R_5$ die obengenannte Bedeutung haben, mit der Massgabe, dass darin enthaltene freie Hydroxygruppen gegebenenfalls mit einer leicht abspaltbaren Schutzgruppe geschützt sind, und $R_9$, $R_{10}$ und $R_{11}$ Wasserstoff oder leicht abspaltbare Schutzgruppen darstellen, oder Derivate davon mit einer Verbindung der Formel

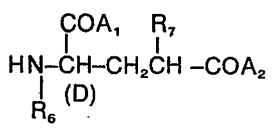

(X)

worin $R_6$, $R_7$, $A_1$ und $A_2$ die obgenannte Bedeutung haben, mit der Massgabe, dass in den Resten $R_7$, $A_1$ und $A_2$ vorhandene freie Carboxylgruppen durch leicht abspaltbare Schutzgruppen geschützt sind, kondensiert und vorhandene Schutzgruppen abspaltet, oder

d) eine Verbindung der Formel

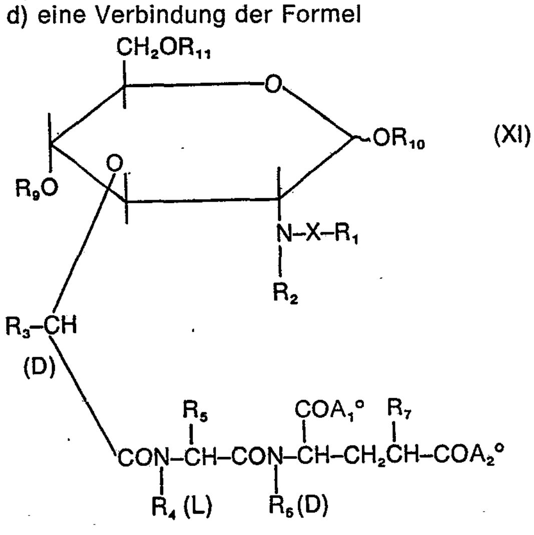

(XI)

worin X, $R_1$, $R_2$, $R_3$, $R_4$, $R_5$, $R_6$ und $R_7$ die obgenannte Bedeutung haben, $R_9$, $R_{10}$ und $R_{11}$ Wasserstoff oder eine leicht abspaltbare Schutzgruppe darstellen und einer der Reste $A_1°$ und $A_2°$ eine aktivierte Hydroxygruppe darstellt und der andere veräthertes Hydroxy, Amino, Niederalkylamino oder Carboxamidoniederalkylamino ist, mit einer Verbindung der Formel

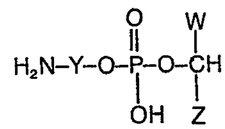

(XII)

worin Y, W und Z die obgenannte Bedeutung besitzen, kondensiert und vorhandene Schutzgruppen abspaltet, oder

e) eine Verbindung der Formel

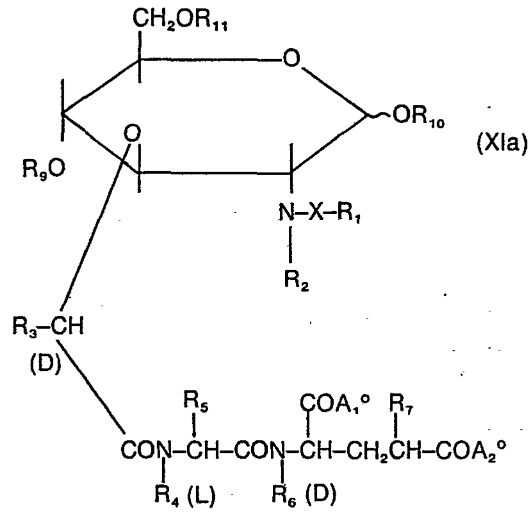

(XIa)

worin X, $R_1$, $R_2$, $R_3$, $R_4$, $R_5$, $R_6$ und $R_7$ die obengenannte Bedeutung haben, $R_9$, $R_{10}$ und $R_{11}$ Wasserstoff oder eine leicht abspaltbare Schutzgruppe darstellen und einer der Reste $A_1°$ und $A_2°$ eine Hydroxygruppe darstellt und der andere veräthertes Hydroxy, Amino, Niederalkylamino oder Carboxamidoniederalkylamino ist, mit einer Verbindung der Formel

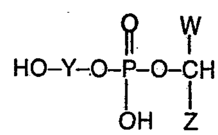

(XIIa)

worin Y, W und Z die obgenannte Bedeutung besitzen, wobei die Säure XIa oder der Alkohol XIIa in reaktionsfähiger Form vorliegt, verestert und gegebenenfalls vorhandene Schutzgruppen abspaltet, oder

f) in einer Verbindung der Formel

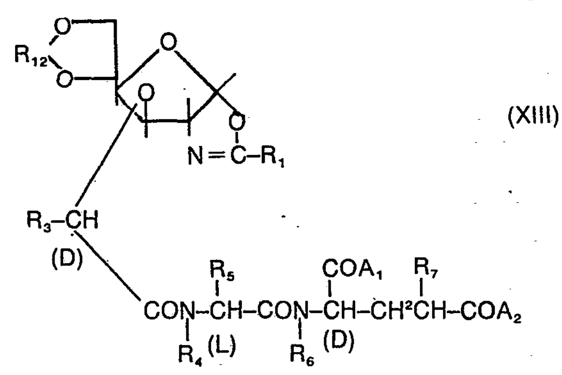

(XIII)

worin $R_1$, $R_3$, $R_4$, $R_5$, $R_6$, $R_7$, $A_1$ und $A_2$ die obengenannte Bedeutung haben und $R_{12}$ eine Alkyliden- oder Cycloalkilidengruppe ist, den Oxazolin- und den Dioxolanring sauer aufspaltet, und gegebenenfalls vorhandene Schutzgruppen abspaltet, oder

g) eine Verbindung der Formel

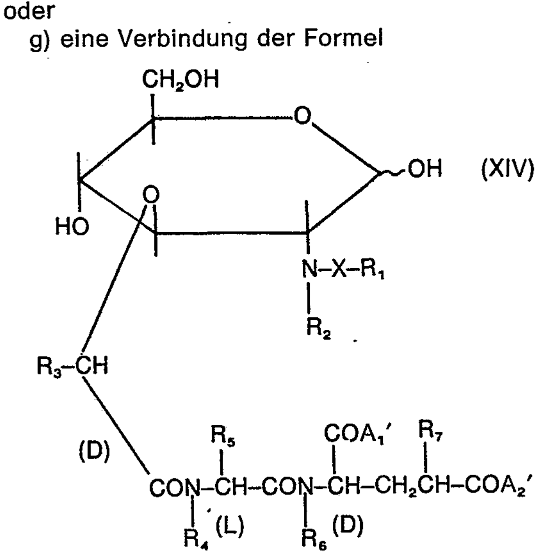

(XIV)

worin X, $R_1$, $R_2$, $R_3$, $R_4$, $R_5$, $R_6$ und $R_7$ die obengenannten Bedeutungen haben, wobei darin vorhandene Hydroxygruppen gegebenenfalls durch leicht abspaltbare Schutzgruppen geschützt sind, einer der Reste $A_1'$ und $A_2'$ einen Rest der Formel

$$-T-Y_1-M_1 \qquad (XV)$$

bedeutet, worin T die obengenannte Bedeutung hat, $Y_1$ gegebenenfalls substituiertes Alkylen, das durch eine Oxycarbonyl- oder Iminocarbonylgruppe unterbrochen sein kann, wobei darin vorhandene Hydroxygruppen gegebenenfalls durch leicht abspaltbare Schutzgruppen geschützt sind, und $M_1$ eine freie Aminogruppe, eine Carbonsäuregruppe oder ein aktiviertes Derivat dieser beiden Gruppen bedeuten, und der andere der Reste $A_1'$ und $A_2'$ veräthertes Hydroxy oderAmino, Niederalkylamino oder Carboxamidoniederalkylamino ist, mit einer Verbindung der Formel

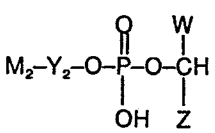

(XVI)

worin $M_2$ eine freie Aminogruppe oder ein aktiviertes Derivat davon bedeutet, wenn $M_1$ für eine Carbonsäuregruppe oder ein aktiviertes Derivat derselben steht, oder worin $M_2$ eine Carbonsäure oder ein aktiviertes Derivat derselben bedeutet, wenn $M_1$ für eine freie Aminogruppe oder ein aktiviertes Derivat davon steht, $Y_2$ gegebenenfalls substituiertes Alkylen, das durch eine Oxycarbonyl- oder Iminocarbonylgruppe unterbrochen sein kann, bedeutet, mit der Massgabe, dass die über eine Iminocarbonylgruppe miteinander verbundenen Reste $Y_1$ und $Y_2$ dem obengenannten Rest Y entsprechen, und W und Z die obengenannten Bedeutungen haben, wobei in den Resten $Y_2$, W und Z vorhandene Hydroxygruppen gegebenenfalls durch leicht abspaltbare Schutzgruppen geschützt sind, kondensiert und gegebenenfalls vorhandene Schutzgruppen abspaltet, oder

h) eine Verbindung der Formel

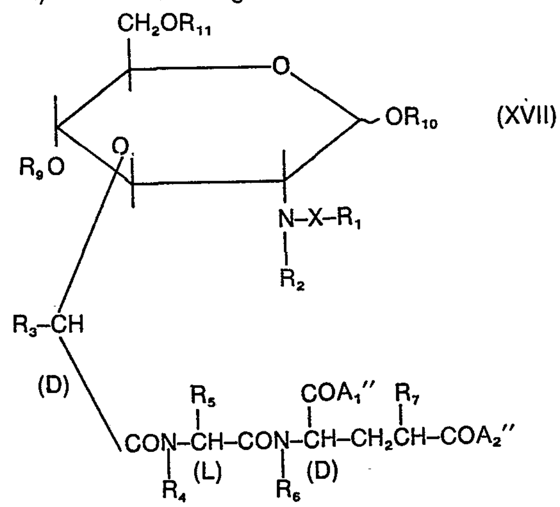

(XVII)

worin X, $R_1$, $R_2$, $R_3$, $R_4$, $R_5$, $R_6$ und $R_7$ die obengenannten Bedeutungen haben, $R_9$, $R_{10}$ und $R_{11}$ leicht abspaltbare Schutzgruppen darstellen, einer der Reste $A_1''$ und $A_2''$ einen Rest der Formel

$$-T-Y_1-M_3 \qquad (XVIII)$$

bedeutet, worin T die obengenannte Bedeutung hat, $Y_1$ gegebenenfalls substituiertes Alkylen, das durch eine Oxycarbonyl- oder Iminocarbonylgruppe unterbrochen sein kann, wobei darin vorhandene Hydroxygruppen gegebenenfalls durch leicht abspaltbare Schutzgruppen geschützt sind, und $M_3$ eine freie Hydroxy- oder Carboxylgruppe, die gegebenenfalls in reaktionsfähiger Form vorliegt, bedeuten, und der andere der Reste $A_1''$ und $A_2''$ veräthertes Hydroxy, Amino, Niederalkylamino oder Carboxamidoniederalkylamino ist, mit einer Verbindung der Formel

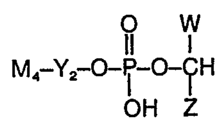

(XIX)

worin $M_4$ eine gegebenenfalls in reaktionsfähiger Form vorliegende Hydroxygruppe bedeutet, wenn $M_3$ eine gegebenenfalls in reaktionsfähiger Form vorliegende Carboxylgruppe bedeutet, oder worin $M_4$ eine gegebenenfalls in reaktionsfähiger Form vorliegende Carboxylgruppe bedeutet, wenn $M_3$ eine gegebenenfalls in reaktionsfähiger Form vorliegende Hydroxygruppe bedeutet, $Y_2$ gegebenenfalls substituiertes Alkylen, das durch eine Oxycarbonyl- oder Iminocarbonylgruppe unterbrochen sein kann, bedeutet, mit der Massgabe, dass die über eine Oxycarbonylgruppe miteinander verbundenen Reste $Y_1$ und $Y_2$ dem obengenannten Rest Y entsprechen, und W und Z die obengenannte Bedeutung haben, wobei in den Resten $Y_2$, W und Z vorhandene Hydroxygruppen gegebenenfalls durch leicht abspaltbare Schutzgruppen geschützt sind, verestert und gegebenenfalls vorhandene Schutzgruppen abspaltet, oder

i) eine Verbindung der Formel

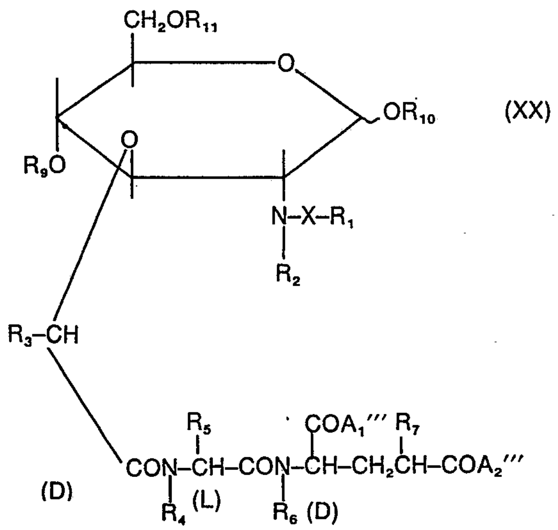

(XX)

worin X, $R_1$, $R_2$, $R_3$, $R_4$, $R_5$, $R_6$ und $R_7$ die obengenannten Bedeutungen haben, wobei gegebenenfalls darin vorhandene Hydroxygruppen mit leicht abspaltbaren Schutzgruppen geschützt sind, $R_9$, $R_{10}$ und $R_{11}$ leicht abspaltbare Schutzgruppen bedeuten und einer der Reste $A_1'''$ und $A_2'''$ für –T–Y–OH steht, worin Y und T die obgenannten Bedeutungen besitzen, wobei gegebenenfalls darin vorhandene Hydroxygruppen mit leicht abspaltbaren Schutzgruppen geschützt sind, und der andere der Reste $A_1'''$ und $A_2'''$ für veräthertes Hydroxy, Amino, Niederalkylamino oder Carboxamidoniederalkylamino steht, mit einer den Rest der Formel

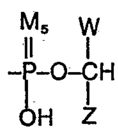

(XXI)

worin = $M_5$ ein Elektronenpaar oder Oxo bedeutet und W und Z die obengenannten Bedeutungen haben, wobei gegebenenfalls darin vorhandene Hydroxygruppen mit leicht abspaltbaren Schutzgruppen geschützt sind, abgebenden Verbindung umsetzt, falls = $M_5$ ein Elektronenpaar ist, mit einem schwachen Oxydationsmittel oxydiert, und vorhandene Schutzgruppen abspaltet, oder

k) eine Verbindung der Formel

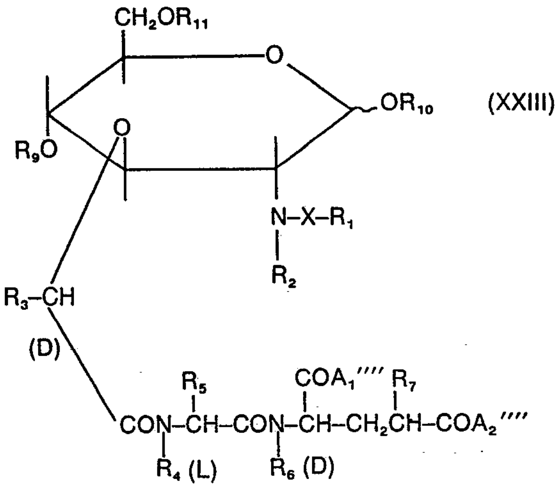

(XXIII)

worin X, $R_1$, $R_2$, $R_3$, $R_4$, $R_5$, $R_6$ und $R_7$ die obengenannten Bedeutungen besitzen, wobei gegebenenfalls darin vorhandene Hydroxygruppen mit leicht abspaltbaren Schutzgruppen geschützt sind, $R_9$, $R_{10}$ und $R_{11}$ leicht abspaltbare Schutzgruppen bedeuten und einer der Reste $A_1''''$ und $A_2''''$ für

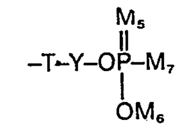

(XXIV)

worin T und Y die obengenannte Bedeutung besitzen, wobei gegebenenfalls darin vorhandene Hydroxygruppen mit leicht abspaltbaren Schutzgruppen geschützt sind, und worin = $M_5$ ein Elektronenpaar oder Oxo, $M_6$ eine leicht abspaltbare Schutzgruppe und $M_7$ freies oder in reaktionsfähiger Form vorliegendes Hydroxy bedeuten, und der andere der Reste $A_1''''$ und $A_2''''$ für freies oder veräthertes Hydroxy, Amino, Niederalkylamino oder Carboxamidoniederalkylamino steht, mit einer Verbindung der Formel

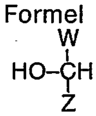

(XXV)

worin W und Z die obgenannte Bedeutung besitzen, wobei gegebenenfalls darin vorhandene Hydroxygruppen mit leicht abspaltbaren Schutzgruppen geschützt sind, umsetzt, falls = $M_5$ ein Elektronenpaar ist, mit einem schwachen Oxydationsmittel oxydiert, und vorhandene Schutzgruppen abspaltet, oder

l) eine Verbindung der Formel

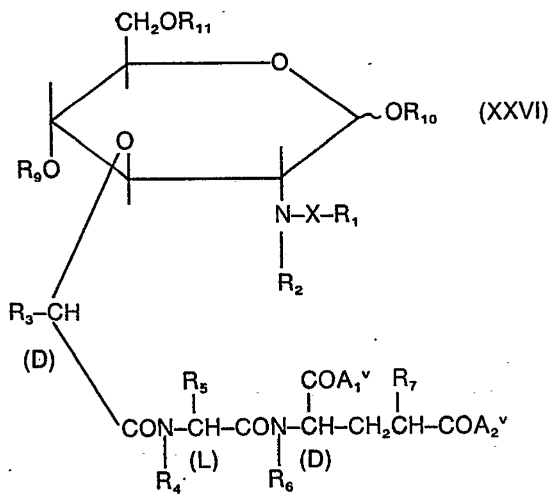

(XXVI)

worin X, $R_1$, $R_2$, $R_3$, $R_4$, $R_5$, $R_6$ und $R_7$ die obengenannten Bedeutungen haben, wobei gegebenenfalls darin vorhandene Hydroxygruppen mit leicht abspaltbaren Schutzgruppen geschützt sind, $R_9$, $R_{10}$ und $R_{11}$ leicht abspaltbare Schutzgruppen bedeuten und einer der Reste $A_1^v$ und $A_2^v$ für einen Rest der Formel XXVII

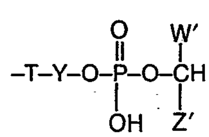

(XXVII)

steht, worin T und Y die obengenannten Bedeutungen haben, wobei in einem Rest der Formel XXVII vorhandene freie funktionelle Gruppen mit Ausnahme der an der Reaktion teilnehmenden Hydroxygruppe(n) gegebenenfalls durch leicht abspaltbare Schutzgruppen geschützt sind, und W′ und Z′ die obengenannte Bedeutung von W bzw. Z haben, jedoch mit der Massgabe, dass mindestens eine Hydroxygruppe in dem Rest 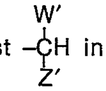 in freier Form vorliegt, und der andere der Reste $A_1^v$ und $A_2^v$ für freies oder veräthertes Hydroxy, Amino-, Niederalkylamino oder Carboxamidoniederalkyl-amino steht, mit einer gegebenenfalls ungesättigten aliphatischen Carbonsäure mit 12–90 C-Atomen oder einem reaktionsfähigen Derivat davon verestert, oder mit einem gegebenenfalls ungesättigten aliphatischen Alkohol mit 10–22 C-Atomen oder einem reaktionsfähigen Derivat davon veräthert und die vorhandenen Schutzgruppen abspaltet, und, wenn erwünscht, eine erhaltene Verbindung der Formel I in ihre Salze überführt, wobei das vorstehend verwendete Präfix «Nieder-» Reste bis und mit 7 C-Atomen bezeichnet.

2. Verfahren nach Anspruch 1, dadurch gekennzeichnet, dass man Muramylpeptide, worin Y ein Niederalkylenrest mit 2–3 Kohlenstoffatomen ist, oder ihre Salze herstellt.

3. Verfahren nach Anspruch 1, dadurch gekennzeichnet, dass man Muramylpeptide, worin Y einen Rest der Formel

$-Y_1-COO-Y_2-$ (IIIa)

$-Y_1-OOC-Y_2-$ (IIIb)

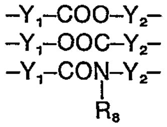 (IIIc)

bzw. 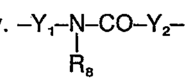 (IIId)

darstellt, worin einer der Reste $Y_1$ und $Y_2$ ein gegebenenfalls substituierter Niederalkylenrest und der andere ein gegebenenfalls substituierter Niederalkylenrest, der auch durch Oxycarbonyl oder $-N-R_8$-Iminocarbonyl unterbrochen sein kann, und $Y_1$ und $Y_2$ zusammen mehr als zwei Kohlenstoffatome aufweisen und $R_8$ Wasserstoff oder Niederalkyl ist, oder ihre Salze herstellt.

4. Verfahren nach Anspruch 1, dadurch gekennzeichnet, dass man Muramylpeptide der Formel I, worin X Carbonyl, $R_1$ gegebenenfalls substituiertes Alkyl oder Aryl, $R_2$, $R_3$, $R_4$ und $R_6$ Wasserstoff oder Niederalkyl, $R_5$ Wasserstoff, gegebenenfalls durch Hydroxy, Niederalkoxy, Mercapto oder Niederalkylmercapto oder Halogen substituiertes Niederalkyl, Cycloalkyl oder Cycloalkylniederalkyl, worin der Cycloalkylrest 4–6 Kohlenstoffatome enthält, gegebenenfalls substituiertes Phenyl oder Phenylniederalkyl, ein oder zwei Azaatome enthaltendes Heterocyclyl oder Heterocyclylniederalkyl oder $R_4$ und $R_5$ zusammen auch Alkylen mit 3–4 Kohlenstoffatomen, $R_7$ Wasserstoff und einer der Reste $A_1$ und $A_2$ einen Rest der Formel

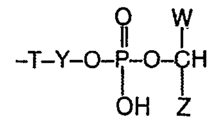 (II)

bedeuten, worin T für HN oder O steht, Y gegebenenfalls substituiertes Alkylen, das auch durch Carbonyloxy oder Carbonylimino unterbrochen sein kann, W Wasserstoff und Z eine 1,2-Dihydroxyäthyl- oder 2-Hydroxyäthylgruppe darstellen, in der mindestens eine der Hydroxygruppen in der im Anspruch 1 angegebenen Weise verestert oder veräthert ist, und der andere der Reste $A_1$ und $A_2$ Hydroxy, Niederalkoxy, Amino, Niederalkylamino oder Carboxamidoniederalkylamino ist, oder ihre Salze herstellt.

5. Verfahren nach Anspruch 1, dadurch gekennzeichnet, dass man Muramylpeptide der Formel I, worin X Carbonyl, $R_1$ gegebenenfalls durch Hydroxy, Niederalkoxy oder Halogen substituiertes Niederalkyl oder gegebenenfalls durch Hydroxy, Niederalkoxy, Niederalkyl oder Halogen substituiertes Phenyl, $R_2$, $R_4$ und $R_6$ Wasserstoff, $R_3$ Wasserstoff oder Niederalkyl, $R_5$ Wasserstoff, gegebenenfalls durch Hydroxy, Niederalkoxy, Mercapto, Niederalkylmercapto oder Halogen substituiertes Niederalkyl mit 1–3 Kohlenstoffatomen, Cycloalkyl oder Cycloalkylniederalkyl, worin der Cycloalkylrest 4–6 Kohlenstoffatome und der Niederalkylrest 1–3 Kohlenstoffatome enthält, gegebenenfalls durch Hydroxy, Niederalkoxy oder Halogen substituiertes Phenyl oder Phenylniederalkyl mit 1–3 Kohlenstoffatomen im Niederalkylrest, ein oder zwei Azaatome enthaltendes Heterocyclyl oder Heterocyclylniederalkyl mit 5–6 Ringgliedern und 1–3 Kohlenstoffatomen im Niederalkylrest oder $R_4$ und $R_5$ zusammen auch Alkylen mit 3–4 Kohlenstoffatomen, $R_7$ Wasserstoff und einer der Reste $A_1$ und $A_2$ einen Rest der Formel

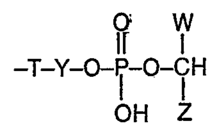 (II)

bedeuten, worin T für HN oder O steht, Y gegebenenfalls substituiertes Niederalkylen oder einen Rest der Formel

$-Y_1-COO-Y_2-$ (IIIa)

oder

$-Y_1-CONH-Y_2-$ (IVc)

bedeutet, worin $Y_1$ und $Y_2$ je gegebenenfalls substituiertes Niederalkylen sind, W Wasserstoff und Z eine 1,2-Dihydroxyäthyl- oder 2-Hydroxyäthylgruppe, in der mindestens eine der Hydroxygruppen mit einer gegebenenfalls ein- oder zweifach ungesättigten aliphatischen Carbonsäure mit 16–22 Kohlenstoffatomen oder mit einer natürlichen oder synthetischen Mycolsäure verestert oder mit einem gegebenenfalls ein- oder zweifach ungesättigten, aliphatischen Alkohol mit 12–18 Kohlenstoffatomen veräthert ist, darstellen, und der andere der Reste $A_1$ und $A_2$ Hydroxy, Nieder-

alkoxy, Amino, Niederalkylamino oder Carboxamidoniederalkylamino ist, oder ihre Salze herstellt.

6. Verfahren nach Anspruch 1, dadurch gekennzeichnet, dass man Muramylpeptide der Formel I, worin X Carbonyl, $R_1$ Niederalkyl mit 1–3 Kohlenstoffatomen oder Phenyl, $R_2$, $R_4$ und $R_6$ Wasserstoff, $R_3$ Wasserstoff oder Niederalkyl mit 1–3 Kohlenstoffatomen, $R_5$ Wasserstoff, gegebenenfalls durch Hydroxy, Methoxy, Mercapto, Methylmercapto oder Halogen substituiertes Niederalkyl mit 1–3 Kohlenstoffatomen, gegebenenfalls durch Hydroxy, Methoxy oder Halogen substituiertes Phenyl oder Phenylmethyl, ein oder zwei Azaatome enthaltendes Heterocyclyl oder Heterocyclylmethyl mit 5 Ringgliedern oder $R_4$ und $R_6$ zusammen auch Trimethylen, $R_7$ Wasserstoff und einer der Reste $A_1$ und $A_2$ einen Rest der Formel

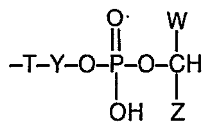

(II)

bedeuten, worin T für HN oder O steht, Y Niederalkylen mit 2–3 Kohlenstoffatomen oder einen Rest der im Anspruch 5 abgebildeten Formeln (IIIa) oder (IVc) bedeutet, worin $Y_1$ und $Y_2$ unabhängig voneinander je Niederalkylen mit 1–3 Kohlenstoffatomen bedeuten, das unsubstituiert oder durch gegebenenfalls durch Hydroxy, Niederalkoxy, Mercapto oder Niederalkylmercapto substituiertes Niederalkyl mit 1–3 Kohlenstoffatomen oder durch gegebenenfalls durch Hydroxy, Methoxy oder Halogen substituiertes Phenyl oder Phenylniederalkyl oder durch ein oder zwei Azaatome enthaltendes Heterocyclyl oder Heterocyclylniederalkyl mit 5–6 Ringgliedern und 1–3 Kohlenstoffatomen im Niederalkylrest substituiert ist, W Wasserstoff und Z eine 1,2-Dihydroxyäthyl- oder 2-Hydroxyäthylgruppe sind, in der mindestens eine der Hydroxygruppen mit einer gegebenenfalls ein- oder zweifach ungesättigten aliphatischen Carbonsäure mit 16–20 Kohlenstoffatomen verestert oder mit einem gegebenenfalls ein- oder zweifach ungesättigten aliphatischen Alkohol mit 12–18 Kohlenstoffatomen veräthert ist, und der andere der Reste $A_1$ und $A_2$ Hydroxy, Niederalkoxy, Amino, Niederalkylamino oder Carboxamidoniederalkylamino ist, oder ihre Salze herstellt.

7. Verfahren zur Herstellung von Muramylpeptiden der in Anspruch 1 dargestellten Formel I, worin

X Carbonyl oder Carbonyloxy. $R_1$ Niederalkyl mit 1–3 Kohlenstoffatomen oder Phenyl, $R_2$, $R_4$ und $R_6$ Wasserstoff, $R^3$ Wasserstoff oder Niederalkyl mit 1–3 Kohlenstoffatomen, $R^5$ Wasserstoff oder Niederalkyl, $R^7$ Wasserstoff, $A_1$ Amino, Niederalkylamino, Hydroxy oder Niederalkoxy und $A_2$ einen Rest der in Anspruch 1 dargestellten Formel (II), worin T für NH oder O steht, Y Niederalkylen mit 2–3 Kohlenstoffatomen oder einen Rest der Formel

$$-CH_2-CO-NH-CH_2-CH_2-$$

bedeuten, W für Wasserstoff und Z für eine 1,2-Dihydroxyäthyl- oder 2-Hydroxyäthyl-Gruppe stehen, in der eine oder zwei Hydroxygruppen mit gleichen oder verschiedenen gegebenenfalls ein- oder zweifach ungesättigten Alkancarbonsäuren mit 16–22 Kohlenstoffatomen verestert oder mit einem gegebenenfalls ein- oder zweifach ungesättigten Alkanol mit 12–18 Kohlenstoffatomen veräthert sind oder worin zwei vicinale Hydroxygruppen unter Bildung eines Dioxolanringes formal mit einem Bisniederalkylketon ketalisiert oder mit einem unsubstituierten aliphatischen Aldehyd mit bis zu 20 C-Atomen acetalisiert sind, oder worin W und Z je eine Hydroxymethylgruppe darstellen, die mit einer gegebenenfalls ein- oder zweifach ungesättigten Alkancarbonsäure mit 16–22 Kohlenstoffatomen verestert oder mit einem gegebenenfalls ein- oder zweifach ungesättigten Alkanol mit 12–18 Kohlenstoffatomen veräthert ist, oder von ihren Salzen analog einem der in Anspruch 1 genannten Verfahren.

8. Verfahren nach Anspruch 7, dadurch gekennzeichnet, dass man Verbindungen, worin die Bedeutungen für $A_1$ und $A_2$ vertauscht sind, oder ihre Salze herstellt.

9. Verfahren nach Anspruch 1, dadurch gekennzeichnet, dass man
N-Acetyl-muramyl-L-alanyl-D-isoglutamin-2-(1′,2′-dipalmitoyl-sn-glycero-3′-hydroxyphosphoryloxy)-äthylamid
oder ein Salz davon herstellt.

10. Verfahren nach Anspruch 1, dadurch gekennzeichnet, dass man
N-Acetyl-desmethylmuramyl-L-alanyl-D-isoglutamin-2-(1′,2′-dipalmitoyl-sn-glycero-3′-hydroxyphosphoryloxy)-äthylamid
oder ein Salz davon herstellt.

11. Verfahren nach Anspruch 1, dadurch gekennzeichnet, dass man
N-Acetyl-muramyl-L-alanyl-D-isoglutaminyl-γ-oxy-essigsäure-2-(1′,2′-dipalmitoyl-sn-glycero-3′-hydroxyphosphoryloxy)-äthylamid
oder ein Salz davon herstellt.

12. Verfahren nach Anspruch 1, dadurch gekennzeichnet, dass man
N-Acetyl-muramyl-L-alanyl-D-isoglutaminyl-L-alanin-2-(1′,2′-dipalmitoyl-sn-glycero-3′-hydroxyphosphoryloxy)-äthylamid
oder ein Salz davon herstellt.

13. Verfahren nach Anspruch 1, dadurch gekennzeichnet, dass man
N-Acetyl-desmethylmuramyl-L-alanyl-D-isoglutamin-2-(1′,2′-dihexadecyl-sn-glycero-3′-hydroxyphosphoryloxy)-äthylamid
oder ein Salz davon herstellt.

14. Verfahren nach Anspruch 1, dadurch gekennzeichnet, dass man
N-Acetyl-desmethylmuramyl-L-alanyl-D-isoglutamin-2-(1′-hexadecyl-sn-glycero-3′-hydroxyphosphoryloxy)-äthylamid
oder ein Salz davon herstellt.

15. Verfahren nach Anspruch 1, dadurch gekennzeichnet, dass man
N-Acetyl-desmethylmuramyl-L-alanyl-D-isoglutamin-2-(3'-palmitoyl-rac-glycero-1'-hydroxyphosphoryloxy)-äthylamid
oder ein Salz davon herstellt.

16. Verfahren nach Anspruch 1, dadurch gekennzeichnet, dass man
N-Acetyl-desmethylmuramyl-L-alanyl-D-isoglutamin-2-(1'-palmitoyl-2'-oleoyl-sn-glycero-3'-hydroxyphosphoryloxy)-äthylamid
oder ein Salz davon herstellt.

17. Verfahren nach Anspruch 1, dadurch gekennzeichnet, dass man
N-Acetyl-desmethylmuramyl-L-alanyl-D-isoglutamin-2-(1'-O-palmitoyl-propandiol-3'-O-hydroxyphosphoryloxy)-äthylamid
oder ein Salz davon herstellt.

18. Verfahren nach Anspruch 1, dadurch gekennzeichnet, dass man
N-Acetyl-muramyl-L-α-aminobutyryl-D-isoglutaminyl-L-alanin-2-(1',2'-dipalmitoyl-sn-glycero-3'-hydroxyphosphoryloxy)-äthylamid
oder ein Salz davon herstellt.

19. Verfahren nach Anspruch 1, dadurch gekennzeichnet, dass man
N-Acetyl-muramyl-L-α-aminobutyryl-D-isoglutaminyl-L-alanin-2-(1',2'-dipalmitoyl-rac-glycero-3'-hydroxyphosphoryloxy)-äthylamid
oder ein Salz davon herstellt.

20. Verfahren nach Anspruch 1, dadurch gekennzeichnet, dass man
N-Benzoyl-muramyl-L-α-aminobutyryl-D-isoglutaminyl-L-alanin-2-(1',2'-dipalmitoyl-sn-glycero-3'-hydroxyphosphoryloxy)-äthylamid
oder ein Salz davon herstellt.

21. Verfahren nach Anspruch 1, dadurch gekennzeichnet, dass man
N-Propionyl-desmethylmuramyl-L-alanyl-D-isoglutaminyl-L-alanin-2-(1',2'-dipalmitoyl-sn-glycero-3'-hydroxyphosphoryloxy)-äthylamid
oder ein Salz davon herstellt.

22. Verfahren nach Anspruch 1, dadurch gekennzeichnet, dass man
N-Acetyl-muramyl-L-valyl-D-isoglutaminyl-L-alanin-2-(1',2'-dipalmitoyl-sn-glycero-3'-hydroxyphosphoryloxy)-äthylamid
oder ein Salz davon herstellt.

23. Verfahren nach Anspruch 1, dadurch gekennzeichnet, dass man
N-Benzoyl-desmethylmuramyl-L-α-aminobutyryl-D-isoglutaminyl-L-alanin-2-(1',2'-dipalmitoyl-sn-glycero-3'-hydroxyphosphoryloxy)-äthylamid
oder ein Salz davon herstellt.

24. Verfahren nach Anspruch 1, dadurch gekennzeichnet, dass man
N-Acetyl-desmethylmuramyl-L-alanyl-D-glutamyl-($C_\alpha$)-glycinamid-($C_\gamma$)-L-alanin-2-(1',2'-dipalmitoyl-sn-glycero-3'-hydroxyphosphoryloxy)-äthylamid
oder ein Salz davon herstellt.

25. Verfahren nach Anspruch 1, dadurch gekennzeichnet, dass man
N-Acetyl-muramyl-L-alanyl-D-isoglutaminyl-L-alanin-2-(3'-palmitoyl-rac-glycero-1'-hydroxyphosphoryloxy)-äthylamid
oder ein Salz davon herstellt.

26. Verfahren nach Anspruch 1, dadurch gekennzeichnet, dass man
N-Acetyl-muramyl-L-alanyl-D-isoglutaminyl-L-alanin-2-(1',2'-hexadecyliden-sn-glycero-3'-hydroxyphosphoryloxy)-äthylamid
oder ein Salz davon herstellt.

27. Verfahren nach Anspruch 1, dadurch gekennzeichnet, dass man
N-Acetyl-desmethylmuramyl-L-alanyl-D-isoglutaminyl-L-alanin-2-(1',2'-hexadecyliden-sn-glycero-3'-hydroxyphosphoryloxy)-äthylamid
oder ein Salz davon herstellt.

28. Verfahren nach Anspruch 1, dadurch gekennzeichnet, dass man ein pharmazeutisch verwendbares Salz einer Verbindung nach einem der Ansprüche 1–27 herstellt.

29. Verfahren nach Anspruch 28, dadurch gekennzeichnet, dass man ein Natriumsalz herstellt.

30. Verfahren nach einem der Ansprüche 1–29, dadurch gekennzeichnet, dass man in einer Verbindung der Formel I, in der die Hydroxylgruppen in 1-, 4- und/oder 6-Stellung des Zuckerteils durch Schutzgruppen geschützt sind, diese Schutzgruppen abspaltet.

31. Verfahren nach einem der Ansprüche 1–29, dadurch gekennzeichnet, dass man in einer Verbindung der Formel I, in der eine oder mehrere Carboxylgruppen durch Schutzgruppen geschützt sind, diese Schutzgruppen abspaltet.

32. Verfahren nach einem der Ansprüche 1–29, dadurch gekennzeichnet, dass man zur Abspaltung von Schutzgruppen mit einer Säure behandelt.

33. Verfahren nach einem der Ansprüche 1–29, dadurch gekennzeichnet, dass man als Verbindungen mit einer oder mehreren reaktionsfähigen veresterten Hydroxygruppen Verbindungen verwendet, worin die Hydroxygruppe(n) mit einer starken anorganischen Säure oder einer Sulfonsäure verestert ist (sind).

34. Verfahren nach einem der Ansprüche 1–29, dadurch gekennzeichnet, dass man als Verbindungen mit einer oder mehreren reaktionsfähigen veresterten Hydroxygruppen Verbindungen verwendet, worin die Hydroxygruppe(n) mit einer Halogenwasserstoffsäure verestert ist (sind).

35. Verfahren nach einem der Ansprüche 1–29, dadurch gekennzeichnet, dass man als Verbindungen mit einer oder mehreren Hydroxyschutzgruppen Verbindungen verwendet, worin die Hydroxygruppe(n) mit Acyl-, Aroyl-, oder von Kohlensäurederivate sich ableitenden Resten oder mit in α-Stellung verzweigten Alkylresten oder mit α-Mono-, -Di- oder -Triarylniederalkylresten oder mit acetalbildenden Resten geschützt ist (sind).

36. Verfahren nach einem der Ansprüche 1–29, dadurch gekennzeichnet, dass man als Verbindungen mit einer oder mehreren Hydroxyschutzgruppen Verbindungen verwendet, worin die Hydroxygruppe(n) mit Niederalkanoyl-, Benzoyl-, Benzyloxycarbonyl-, Niederalkoxycarbonyl-, tert.Butyl- oder mit gegebenenfalls substituierten Benzyl-, Triphenylmethyl- oder Tetrahydropyranylresten geschützt ist (sind).

37. Verfahren nach einem der Ansprüche 1–29, dadurch gekennzeichnet, dass man als Verbindungen mit einer oder mehreren Carboxyschutzgruppen Verbindungen verwendet, worin die Carboxygruppe(n) mit tert.Butyl-, Benzyl- oder mit gegebenenfalls durch Halogen oder Niederalkoxy substituierten Triphenylmethyl- oder Benzhydrylresten geschützt ist (sind).

38. Verfahren nach einem der Ansprüche 1–29, dadurch gekennzeichnet, dass man als Verbindungen mit einer oder mehreren aktivierten Carbonsäuregruppen Verbindungen verwendet, worin die Carbonsäuregruppe(n) als Anhydrid, Azid, aktiviertes Amid oder als aktivierter Ester vorliegt (vorliegen).

39. Verfahren nach Anspruch 38, dadurch gekennzeichnet, dass man als Säureanhydride solche mit Kohlensäureniederalkylester, als Säureamide Imidazolide oder Isooxazolide und als aktivierte Ester Cyan- oder Carboxymethylester, Acetylaminoethylthioester, p-Nitro- oder 2,4,5-Trichlor-phenylester, N-Hydroxy-succinimid, N-Hydroxy-phthalimid- oder N-Hydroxy-piperidinester, 8-Hydroxy-chinolinester, Methoxyäthoxythioester oder durch Umsetzung mit Carbodiimid unter Zusatz von N-Hydroxysuccinimid oder einem 1-Hydroxybenzotriazol oder 3-Hydroxy-4-oxo-3,4-dihydro-benzo-[d]-1,2,3-triazin erhaltene Ester verwendet.

40. Mischverfahren zur Herstellung eines pharmazeutischen Präparates, dadurch gekennzeichnet, dass man eine Verbindung, die gemäss einem der Ansprüche 1 bis 39 erhalten wurde, mit einem pharmazeutischen Trägermaterial mischt.

**CLAIMS for the Contracting States: BE, CH, DE, FR, GB, IT, LI, LU, NL, SE**

1. Muramyl peptides of the formula

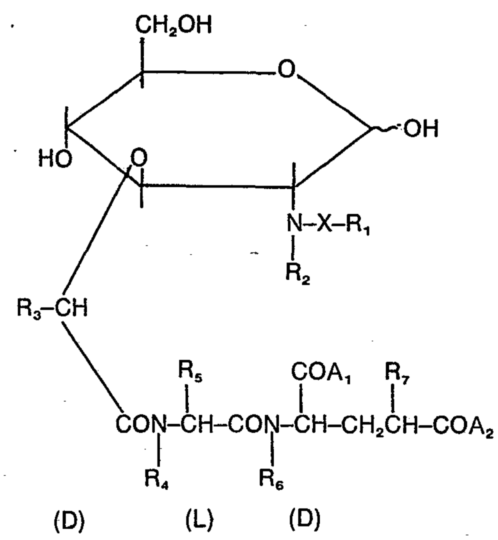

(I)

in which X represents carbonyl or carbonyloxy, $R_1$ represents optionally substituted alkyl or aryl, $R_2$, $R_4$ and $R_6$ represent hydrogen or lower alkyl, $R_3$ represents hydrogen or lower alkyl, $R_5$ represents hydrogen, lower alkyl, free or functionally modified hydroxy-lower alkyl, free or functionally modified mercapto-lower alkyl, optionally substituted amino-lower alkyl, cycloalkyl, cycloalkyl-lower alkyl, optionally substituted aryl or aralkyl, nitrogen-containing heterocyclyl or heterocyclyl-lower alkyl, or $R_4$ and $R_5$ together also represent alkylene having 3 or 4 carbon atoms, $R_7$ represents hydrogen or optionally esterified or amidated carboxyl, and one of the radicals $A_1$ and $A_2$ represents a radical of the formula

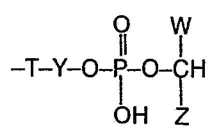

(II)

in which T represents HN or O, Y represents an optionally substituted alkylene group, which may also be interrupted by one or two oxycarbonyl and/or iminocarbonyl groups, W represents hydrogen, and Z represents a 1,2-dihydroxyethyl or 2-hydroxyethyl group in which at least one of the hydroxy groups is esterified by an optionally unsaturated aliphatic carboxylic acid having 12–90 C atoms, or is etherified by an optionally unsaturated aliphatic alcohol having 10–22 C atoms, or W and Z each represent a hydroxymethyl group

esterified by an optionally unsaturated aliphatic carboxylic acid having 12–90 C atoms or etherified by an optionally unsaturated aliphatic alcohol having 10–22 C atoms, and the other of the radicals $A_1$ and $A_2$ is free or etherified hydroxy, amino, lower alkylamino or carboxamido-lower alkylamino, and salts thereof, the prefix «lower» denoting radicals having up to and including 7 C atoms.

2. Muramyl peptides according to claim 1, in which Y is a lower alkylene radical which has 2 or 3 carbon atoms, and salts thereof.

3. Muramyl peptides according to claim 1, in which Y is a radical of the formula

$-Y_1-COO-Y_2-$ (IIIa)

$-Y_1-OOC-Y_2-$ (IIIb)

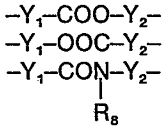 (IIIc)

or 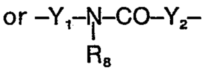 (IIId)

in which one of the radicals $Y_1$ and $Y_2$ represents an optionally substituted lower alkylene radical and the other represents an optionally substituted lower alkylene radical which may also be interrupted by oxycarbonyl or $-N-R_8$-iminocarbonyl, and $Y_1$ and $Y_2$ together have more than two carbon atoms, and $R_8$ represents hydrogen or lower alkyl, and salts thereof.

4. Muramyl peptides of the formula I according to claim 1, in which X represents carbonyl, $R_1$ represents optionally substituted alkyl or aryl, $R_2$, $R_3$, $R_4$ and $R_6$ represent hydrogen or lower alkyl, $R_5$ represents hydrogen, lower alkyl optionally substituted by hydroxy, lower alkoxy, mercapto, lower alkylmercapto or halogen; cycloalkyl or cycloalkyl-lower alkyl, wherein the cycloalkyl radical contains 4–6 carbon atoms; optionally substituted phenyl or phenyl-lower alkyl, heterocyclyl or heterocyclyl-lower alkyl containing one or two aza atoms, or $R_4$ and $R_5$ together also represent alkylene having 3 or 4 carbon atoms, $R_7$ represents hydrogen and one of the radicals $A_1$ and $A_2$ represents a radical of the formula

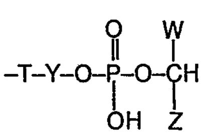 (II)

in which T represents HN or O, Y represents optionally substituted alkylene which may also be interrupted by carbonyloxy or carbonylimino, W represents hydrogen and Z represents a 1,2-dihydroxyethyl or 2-hydroxyethyl group, in which at least on of the hydroxy groups is esterified or etherified in the manner described in claim 1, and the other of the radicals $A_1$ and $A_2$ represents hydroxy, lower alkoxy, amino, lower alkylamino or carboxamido-lower alkylamino, and salts thereof.

5. Muramyl peptides of the formula I according to claim 1, in which X represents carbonyl, $R_1$ represents lower alkyl optionally substituted by hydroxy, lower alkoxy or halogen, or phenyl optionally substituted by hydroxy, lower alkoxy, lower alkyl or halogen, $R_2$, $R_4$ and $R_6$ represent hydrogen, $R_3$ represents hydrogen or lower alkyl, $R_5$ represents hydrogen; lower alkyl having 1–3 carbon atoms and optionally substituted by hydroxy, lower alkoxy, mercapto, lower alkylmercapto or halogen; cycloalkyl or cycloalkyl-lower alkyl in which the cycloalkyl radical contains 4–6 carbon atoms and the lower alkyl radical contains 1–3 carbon atoms; phenyl or phenyl-lower alkyl having 1–3 carbon atoms in the lower alkyl radical both optionally substituted by hydroxy, lower alkoxy or halogen; or heterocyclyl or heterocyclyl-lower alkyl having 1–3 carbon atoms in the lower alkyl radical and each containing 5 or 6 ring members and one or two aza atoms, or $R_4$ and $R_5$ together represent also alkylene having 3 or 4 carbon atoms, $R_7$ represents hydrogen, and one of the radicals $A_1$ and $A_2$ represents a radical of the formula

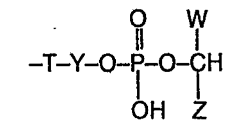 (II)

in which T represents HN or O, Y represents optionally substituted lower alkylene or a radical of the formula

or $-Y_1-COO-Y_2-$ (IIIa)

$-Y_1-CONH-Y_2-$ (IVc)

in which $Y_1$ and $Y_2$ each represent optionally substituted lower alkylene, W represents hydrogen, and Z represents a 1,2-dihydroxyethyl or 2-hydroxyethyl group in which at least one of the hydroxy groups is esterified by an optionally singly or doubly unsaturated aliphatic carboxylic acid having 16–22 carbon atoms or by a natural or synthetic mycolic acid, or is etherified by an optionally singly or doubly unsaturated aliphatic alcohol having 12–18 carbon atoms, and the other of the radicals $A_1$ and $A_2$ represents hydroxy, lower alkoxy, amino, lower alkylamino or carboxamido-lower alkylamino, and salts thereof.

6. Muramyl peptides of the formula I according to claim 1, in which X represents carbonyl, $R_1$ represents lower alkyl having 1–3 carbon atoms or phenyl, $R_2$, $R_4$ and $R_6$ represents hydrogen, $R_3$ represents hydrogen or lower alkyl having 1–3 carbon atoms, $R_5$ represents hydrogen; lower alkyl having 1–3 carbon atoms and optionally substituted by hydroxy, methoxy, mercapto, methylmercapto or halogen; phenyl or phenylmethyl optionally substituted by hydroxy, methoxy or halogen; heterocyclyl or heterocyclylmethyl each containing 5 ring members and one or two aza atoms, or $R_4$ and $R_5$ together represent also trimethylene, $R_7$ represents hydrogen, and one of the radicals $A_1$ and $A_2$ represents a radical of the formula

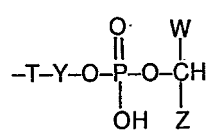 (II)

in which T represents HN or O, Y represents lower alkylene having 2 or 3 carbon atoms or a radical of the formula (IIIa) or (IVc) given in claim 5, in which $Y_1$ and $Y_2$ independently of one another each represent lower alkylene having 1–3 carbon atoms, which is unsubstituted or substituted by optionally hydroxy-, lower-alkoxy-, mercapto- or lower-alkylmercapto-substituted lower alkyl having 1–3 carbon atoms, or by optionally hydroxy-, methoxy- or halogen-substituted phenyl or phenyl-lower-alkyl, or by heterocyclyl or heterocyclyl-lower-alkyl having 1–3 carbon atoms in the lower alkyl radical and each containing 5 or 6 ring members and one or two aza atoms, W represents hydrogen, and Z represents a 1,2-dihydroxyethyl or 2-hydroxyethyl group in which at least one of the hydroxy groups is esterified with an optionally singly or doubly unsaturated aliphatic carboxylic acid having 16 to 20 carbon atoms or is etherified with an optionally singly or doubly unsaturated aliphatic alcohol having 12–18 carbon atoms, and the other of the radicals $A_1$ and $A_2$ represents hydroxy, lower alkoxy, amino, lower alkylamino or carboxamido-lower alkylamino, and salts thereof.

7. Muramyl peptides of the formula I according to claim 1, in which X represents carbonyl or carbonyloxy, $R_1$ represents lower alkyl having 1–3 carbon atoms or phenyl, $R_2$, $R_4$ and $R_6$ represent hydrogen, $R^3$ represents hydrogen or lower alkyl having 1–3 carbon atoms, $R^5$ represents hydrogen or lower alkyl, $R^7$ represents hydrogen, $A_1$ represents amino, lower alkylamino, hydroxy or lower alkoxy, and $A_2$ represents a radical of the formula (II) given in claim 1, in which T represents NH or O, Y represents lower alkylene having 2 or 3 carbon atoms or a radical of the formula

$$-CH_2-CO-NH-CH_2-CH_2-,$$

W represents hydrogen, and Z represents a 1,2-dihydroxyethyl or 2-hydroxyethyl group, in which one or two hydroxy groups are esterified by identical or different, optionally singly or doubly unsaturated alkanecarboxylic acids having 16–22 carbon atoms, or etherified by an optionally singly or doubly unsaturated alkanol having 12–18 carbon atoms, or wherein two adjacent hydroxy groups are ketalised, by formation of a dioxolane ring, with a bis-lower-alkyl ketone, or acetalised with an unsubstituted aliphatic aldehyde having up to 20 C atoms, or wherein W und Z each represent a hydroxymethyl group, which is esterified with an optionally singly or doubly unsaturated alkanecarboxylic acid having 16–22 carbon atoms, or etherified with an optionally singly or doubly unsaturated alkanol having 12–18 carbon atoms, and salts thereof, the prefix "lower" denoting radicals having up to and including 7 C atoms.

8. Compounds according to claim 7, wherein the meanings for $A_1$ and $A_2$ are exchanged, and salts thereof.

9. N-Acetylmuramyl-L-alanyl-D-iso-glutamine-2-(1′,2′-dipalmitoyl-sn-glycero-3′-hydroxyphosphoryloxy)-ethylamide and salts thereof, according to claim 1.

10. N-Acetyl-desmethylmuramyl-L-alanyl-D-isoglutamine-2-(1′,2′-dipalmitoyl-sn-glycero-3′-hydroxyphosphoryloxy)-ethylamide and salts thereof, according to claim 1.

11. N-Acetylmuramyl-L-alanyl-D-isoglutaminyl-γ-oxyacetic acid-2-(1′,2′-dipalmitoyl-sn-glycero-3′-hydroxyphosphoryloxy)-ethylamide and salts thereof, according to claim 1.

12. N-Acetylmuramyl-L-alanyl-D-isoglutaminyl-L-alanin-2-(1′,2′-dipalmitoyl-sn-glycero-3′-hydroxyphosphoryloxy)-ethylamide and salts thereof, according to claim 1.

13. N-Acetyl-desmethylmuramyl-L-alanyl-D-isoglutamine-2-(1′,2′-dihexadecyl-sn-glycero-3′-hydroxyphosphoryloxy)-ethylamide and salts thereof, according to claim 1.

14. N-Acetyl-desmethylmuramyl-L-alanyl-D-isoglutamine-2-(1′-hexadecyl-sn-glycero-3′-hydroxyphosphoryloxy)-ethylamide and salts thereof, according to claim 1.

15. N-Acetyl-desmethylmuramyl-L-alanyl-D-isoglutamine-2-(3′-palmitoyl-rac-glycero-1′-hydroxyphosphoryloxy)-ethylamide and salts thereof, according to claim 1.

16. N-Acetyl-desmethylmuramyl-L-alanyl-D-isoglutamine-2-(1′-palmitoyl-2′-oleoyl-sn-glycero-3′-hydroxyphosphoryloxy)-ethylamide and salts thereof, according to claim 1.

17. N-Acetyl-desmethylmuramyl-L-alanyl-D-isoglutamine-2-(1′-O-palmitoyl-propanediol-3′-O-hydroxyphosphoryloxy)-ethylamide and salts thereof, according to claim 1.

18. N-Acetyl-muramyl-L-α-aminobutyryl-D-isoglutaminyl-L-alanine-2-(1′,2′-dipalmitoyl-sn-glycero-3′-hydroxyphosphoryloxy)-ethylamide and salts thereof, according to claim 1.

19. N-Acetyl-muramyl-L-α-aminobutyryl-D-isoglutaminyl-L-alanine-2-(1′,2′-dipalmitoyl-rac-glycero-3′-hydroxyphosphoryloxy)-ethylamide and salts thereof, according to claim 1.

20. N-Benzoyl-muramyl-L-α-aminobutyryl-D-isoglutaminyl-L-alanin-2-(1′,-2′-dipalmitoyl-sn-glycero-3′-hydroxyphosphoryloxy)-ethylamide and salts thereof, according to claim 1.

21. N-Propionyl-desmethylmuramyl-L-alanyl-D-isoglutaminyl-L-alanine-2-(1′,2′-dipalmitoyl-sn-glycero-3′-hydroxyphosphoryloxy)-ethylamide and salts thereof, according to claim 1.

22. N-Acetyl-muramyl-L-valyl-D-isoglutaminyl-L-alanine-2-(1′,2′-dipalmitoyl-sn-glycero-3′-hydroxyphosphoryloxy)-ethylamide and salts thereof, according to claim 1.

23. N-Benzoyl-desmethylmuramyl-L-α-aminobutyryl-D-isoglutaminyl-L-alanine-2-(1′,2′-dipalmitoyl-sn-glycero-3′-hydroxyphosphoryloxy)-thylamide and salts thereof, according to claim 1.

24. N-Acetyl-desmethylmuramyl-L-alanyl-D-glutamyl-($C_\alpha$)-glycinamide-($C_\gamma$)-L-alanine-2-(1′,2′-dipalmitoyl-sn-glycero-3′-hydroxyphosphoryloxy-ethylamide and salts thereof, according to claim 1.

25. N-Acetyl-muramyl-L-alanyl-D-isoglutaminyl-L-alanine-2-(3′-palmitoyl-rac-glycero-1′-hy-

droxyphosphoryloxy)-ethylamide and salts thereof, according to claim 1.

26. N-Acetyl-muramyl-L-alanyl-D-isoglutaminyl-L-alanine-2-(1′,2′-hexadecylidene-sn-glycero-3′-hydroxyphosphoryloxy)-ethylamide and salts thereof.

27. N-Acetyl-desmethylmuramyl-L-alanyl-D-isoglutaminyl-L-alanine-2-(1′,2′-hexadecylidene-sn-glycero-3′-hydroxyphosphoryloxy)-ethylamide and salts thereof.

28. The pharmaceutically applicable salts of the compounds according to claims 1–27.

29. The sodium salts of the compounds according to claims 1–27.

30. Pharmaceutical preparations containing one of the compounds stated in claims 1–29, together with a pharmaceutical carrier substance.

31. Immunisation agent containing one of the compounds stated in one of the claims 1–29, together with a suitable carrier substance.

32. One of the compounds stated in claims 1–29 for use as a medicament.

33. Use of the compounds stated in claims 1–29 for producing an immunisation agent or vaccine.

34. Process for the manufacture of muramyl peptides of the formula I according to one of the claims 1–29 and salts thereof, characterised in that, in a manner known per se,

a) a compound of the formula

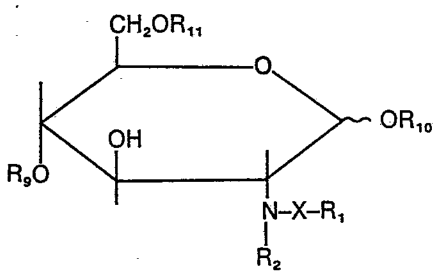

(V)

in which X, $R_1$ and $R_2$ have the meanings given above and hydroxy groups optionally present therein are protected by a protecting group that can readily be split off, and $R_9$, $R_{10}$ and $R_{11}$ represent a protecting group that can readily be split off, or a metal compound thereof, is reacted with a compound of the formula

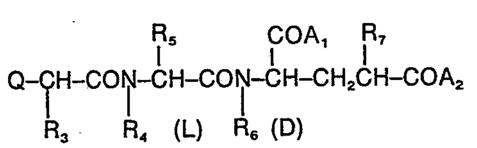

(VI)

(L)

or (D,L)

in which Q represents a reactive esterified hydroxy group, $R_3$, $R_4$, $R_5$, $R_6$, $R_7$, $A_1$ and $A_2$ have the meanings given above and hydroxy groups optionally present therein are protected by a protecting group that can readily be split off, and protecting groups present are split off, or

b) a compound of the formula

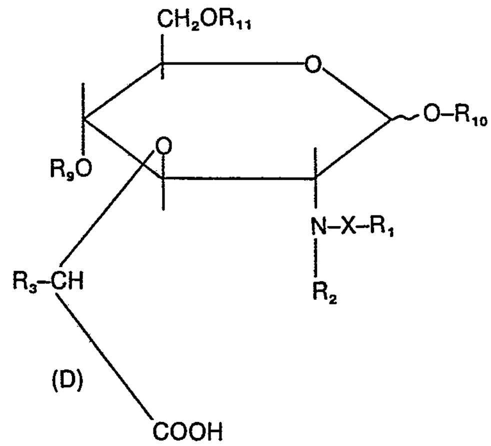

(VII)

in which X, $R_1$, $R_2$ and $R_3$ have the meanings given above, and $R_9$, $R_{10}$ and $R_{11}$ represent hydrogen or a protecting group that can readily be split off, or a derivative thereof, is condensed with a compound of the formula

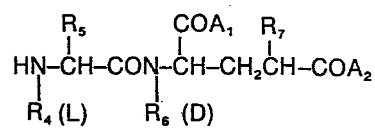

(VIII)

in which $R_4$, $R_5$, $R_6$, $R_7$, $A_1$ and $A_2$ have the meanings given above, provided that carboxy and, if desired, free hydroxy groups present in these radicals are protected by protecting groups that can readily be split off, or with a derivative thereof, and protecting groups present are split off, or

c) a compound of the formula

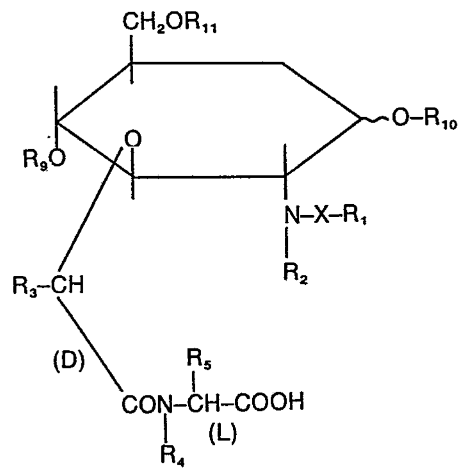

(IX)

in which X, $R_1$, $R_2$, $R_3$, $R_4$ and $R_5$ have the meanings given above, provided that free hydroxy groups contained therein are optionally protected by a protecting group that can readily be split off, and $R_9$, $R_{10}$ and $R_{11}$ represent hydrogen or protecting groups that can readily be split off, or a derivative thereof, is condensed with a compound of the formula

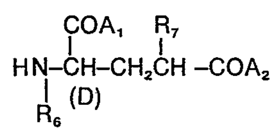

(X)

in which $R_6$, $R_7$, $A_1$ and $A_2$ have the meanings given above, provided that free carboxyl groups present in the radicals $R_7$, $A_1$ and $A_2$ are protected by protecting groups that can readily be split off, and protecting groups present are split off, or

d) a compound of the formula

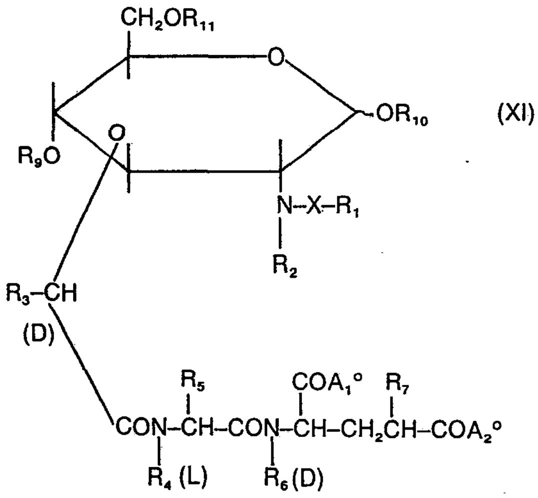

(XI)

in which X, $R_1$, $R_2$, $R_3$, $R_4$, $R_5$, $R_6$ and $R_7$ have the meanings given above, $R_9$, $R_{10}$ and $R_{11}$ represent hydrogen or a protecting group that can readily be split off, and one of the radicals $A_1°$ and $A_2°$ represent an activated hydroxy group, whilst the other represents etherified hydroxy, amino, lower alkylamino or carboxamido-lower alkylamino, is condensed with a compound of the formula

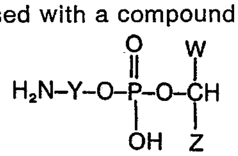

(XII)

in which Y, W and Z have the meanings given above, and protecting groups present are split off, or

e) a compound of the formula

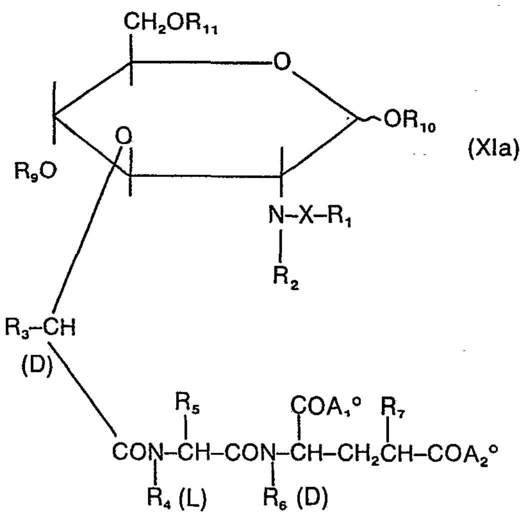

(XIa)

in which X, $R_1$, $R_2$, $R_3$, $R_4$, $R_5$, $R_6$ and $R_7$ have the meanings given above, $R_9$, $R_{10}$ and $R_{11}$ represent hydrogen or a protecting group that can readily be split off, and one of the radicals $A_1°$ and $A_2°$ represents a hydroxy group whilst the other represents etherified hydroxy, amino, lower alkylamino or carboxamido-lower alkylamino, is esterified with a compound of the formula

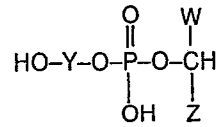

(XIIa)

in which Y, W and Z have the meanings given above, the acid XIa or the alcohol XIIa being present in reactive form, and protecting groups optionally present are split off, or

f) in a compound of the formula

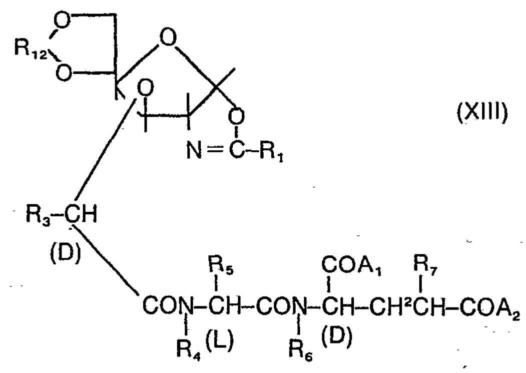

(XIII)

in which $R_1$, $R_3$, $R_4$, $R_5$, $R_6$, $R_7$, $A_1$ and $A_2$ have the meanings given above, and $R_{12}$ represents an alkylidene or cycloalkylidene group, the oxazoline and the dioxalane ring is split by acid means, and protecting groups optionally present are split off, or

g) a compound of the formula

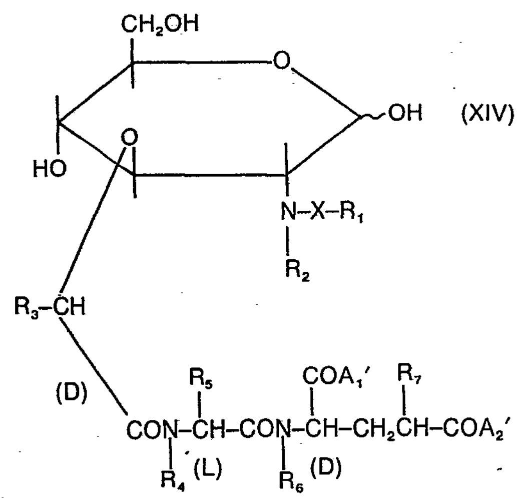

(XIV)

in which X, $R_1$, $R_2$, $R_3$, $R_4$, $R_5$, $R_6$ and $R_7$ have the meanings given above, and hydroxy groups present therein are optionally protected by protecting groups that can readily be split off, one of the radicals $A_1'$ and $A_2'$ represents a radical of the formula

$$-T-Y_1-M_1 \qquad (XV)$$

wherein T has the meaning given above, $Y_1$ represents optionally substituted alkylene which can be interrupted by an oxycarbonyl or iminocarbonyl group, and hydroxy groups present therein are optionally protected by protecting groups that can readily be split off, and $M_1$ represents a free amino group, a carboxylic acid group or an activated derivative of these two groups, whilst the other of the radicals $A_1'$ and $A_2'$ is etherified hydroxy or amino, lower alkylamino or carboxamido-lower alkylamino, is condensed with a compound of the formula

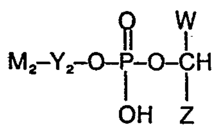

(XVI)

in which $M_2$ is a free amino group or an activated derivative thereof when $M_1$ represents a carboxylic acid group or an activated derivative thereof, or wherein $M_2$ is a carboxylic acid or an activated derivative thereof when $M_1$ represents a free amino group or an activated derivative thereof, $Y_2$ is optionally substituted alkylene which can be interrupted by an oxycarbonyl or iminocarbonyl group, with the proviso that the radicals $Y_1$ and $Y_2$ attached to one another by way of an iminocarbonyl group correspond to the aforementioned radical Y, and W and Z have the meanings given above, and in the radicals $Y_2$, W and Z, hydroxy groups present are optionally protected by protecting groups that are readily split off, and protecting groups optionally present are split off, or

h) a compound of the formula

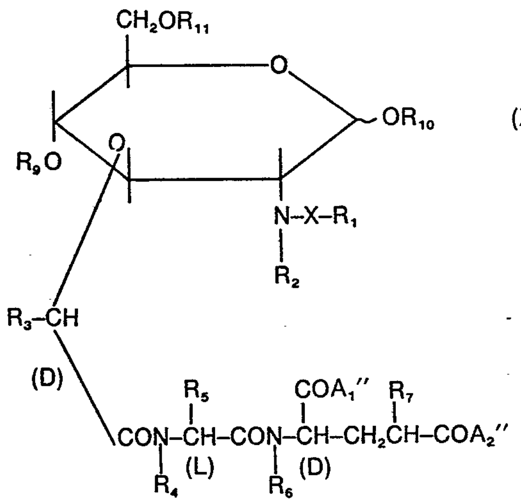

(XVII)

in which X, $R_1$, $R_2$, $R_3$, $R_4$, $R_5$, $R_6$ and $R_7$ have the meanings given above, $R_9$, $R_{10}$ and $R_{11}$ represent protecting groups that are readily split off, one of the radicals $A_1''$ and $A_2''$ is a radical of the formula

$$-T-Y_1-M_3 \qquad (XVIII)$$

in which T has the meaning given above, $Y_1$ represents optionally substituted alkylene which can be interrupted by an oxycarbonyl or iminocarbonyl group, and hydroxy groups present therein are optionally protected by protecting groups that can readily be split off, and $M_3$ represents a free hydroxy or carboxyl group which is optionally present in a reactive form, and the other of the radicals $A_1''$ and $A_2''$ is etherified hydroxy, amino, lower alkylamino or carboxamido-lower alkylamino, is esterified with a compound of the formula

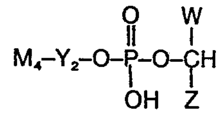

(XIX)

in which $M_4$ is a hydroxy group optionally present in a reactive form when $M_3$ is a carboxyl group optionally present in a reactive form, or in which $M_4$ is a carboxyl group optionally present in a reactive form when $M_3$ is a hydroxy group optionally present in an activated form, $Y_2$ is optionally substituted alkylene which can be interrupted by an oxycarbonyl or iminocarbonyl group, with the proviso that the radicals $Y_1$ and $Y_2$ attached to one another by way of an oxycarbonyl group correspond to the aforementioned radical Y, and W and Z have the meanings given above, and in the radicals $Y_2$, W and Z, hydroxy groups present are optionally protected by protecting groups that are readily split off, and protecting groups optionally present are split off, or

i) a compound of the formula

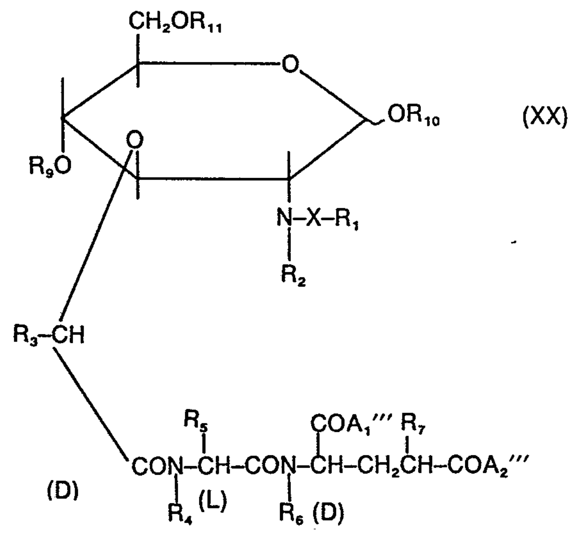

(XX)

in which X, $R_1$, $R_2$, $R_3$, $R_4$, $R_5$, $R_6$ and $R_7$ have the meanings given above, and hydroxy groups optionally present therein are protected by protecting groups that are readily split off, $R_9$, $R_{10}$ and $R_{11}$ represent protecting groups that can readily be split off, and one of the radicals $A_1'''$ and $A_2'''$ represents –T–Y–OH in which Y and T have the meanings given above, and hydroxy groups optionally present therein are protected by protecting groups that can readily be split off, and the other of the radicals $A_1'''$ and $A_2'''$ represents etherified hydroxy, amino, lower alkylamino or carboxamido-lower alkylamino, is reacted with a compound yielding the radical of the formula

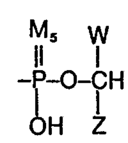

(XXI)

in which $=M_5$ represents an electron pair or oxo, and W and Z have the meanings given above, and hydroxy groups optionally present therein are protected by protecting groups that are readily split off, if $M_5$ is an electron pair is oxidised with a weak oxidising agent, and protecting groups present are split off, or

k) a compound of the formula

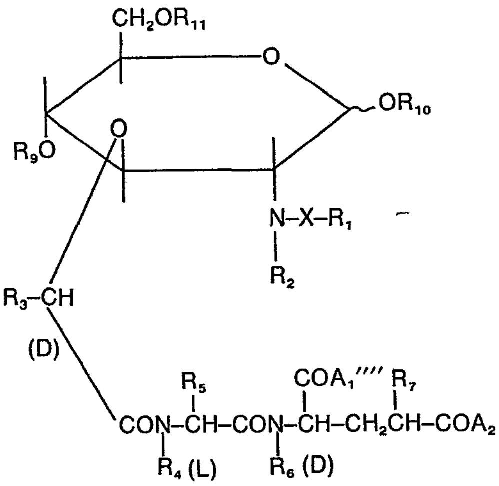

(XXIII)

in which X, $R_1$, $R_2$, $R_3$, $R_4$, $R_5$, $R_6$ and $R_7$ have the meanings given above, and hydroxy groups optionally present therein are protected by protecting groups that can readily be split off, $R_9$, $R_{10}$ and $R_{11}$ represent protecting groups that can readily be split off, and one of the radicals $A_1''''$ and $A_2''''$ represents

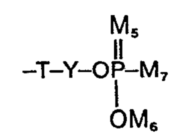

(XXIV)

in which T and Y have the meanings given above, and hydroxy groups optionally present therein are protected with protecting groups that are readily split off, and in which $=M_5$ is an electron pair or oxo, $M_6$ is a protection group that can readily be split off, and $M_7$ represents free hydroxy or hydroxy present in the reactive form, and the other of the radicals $A_1''''$ and $A_2''''$ represents free or etherified hydroxy, amino, lower alkylamino or carboxamido-lower alkylamino, is reacted with a compound of the formula

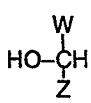

(XXV)

in which W and Z have the meanings given above, and hydroxy groups optionally present therein are protected by protecting groups that can readily be split off, if $=M_5$ represents an electron pair is oxidised with a weak oxidising agent, and protecting groups present are split off, or

l) a compound of the formula

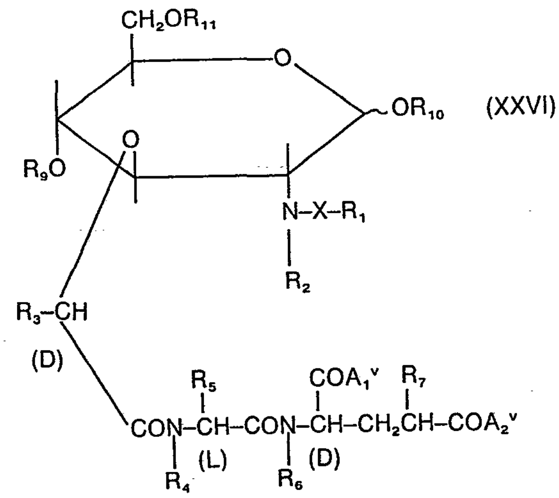

(XXVI)

in which X, $R_1$, $R_2$, $R_3$, $R_4$, $R_5$, $R_6$ and $R_7$ have the meanings given above, and hydroxy groups optionally present therein are protected by protecting groups that can readily be split off, $R_9$, $R_{10}$ and $R_{11}$ represent protecting groups that can readily be split off, and one of the radicals $A_1^v$ and $A_2^v$ represents a radical of the formula XXVII

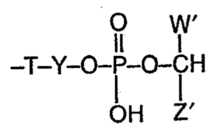

(XXVII)

in which T and Y have the meanings given above, and in a radical of the formula XXVII free functional groups, with the exception of the hydroxy group(s) participating in the reaction, are optionally protected by protecting groups which can readily be split off, and W' and Z' have the aforementioned meaning of W and Z, with the proviso however that at least one hydroxy group in the radical 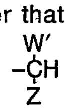 is present in the free form, and the other of the radicals $A_1^v$ and $A_2^v$ represents free or etherified hydroxy, amino, lower alkylamino or carboxamido-lower alkylamino, is esterified with an optionally unsaturated aliphatic carboxylic acid having 12–90 C atoms or with a reactive derivative thereof, or is etherified with an optionally unsaturated aliphatic alcohol having 10–22 C atoms or with a reactive derivative thereof, and the protecting groups present are split off, and, if desired, a resulting compound of the formula I is converted into salts thereof, the prefix "lower" employed in the foregoing denoting radicals having up to and including 7 C atoms, and the expression "above-mentioned meaning" used in the foregoing signifying the meaning of the particular radical in the product claim to which this process claim relates.

35. The compounds obtainable by the process according to claim 34.

**CLAIMS for the Contracting State AT**

1. Process for the manufacture of muramyl peptides of the formula

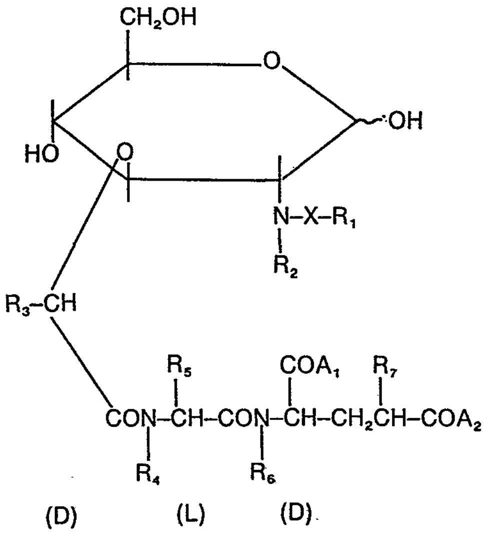

(I)

in which X represents carbonyl or carbonyloxy, $R_1$ represents optionally substituted alkyl or aryl, $R_2$, $R_4$ and $R_6$ represent hydrogen or lower alkyl, $R_3$ represents hydrogen or lower alkyl, $R_5$ represents hydrogen, lower alkyl, free or functionally modified hydroxy-lower alkyl, free or functionally modified mercapto-lower alkyl, optionally substituted amino-lower alkyl, cycloalkyl, cycloalkyl-lower alkyl, optionally substituted aryl or aralkyl, nitrogen-containing heterocyclyl or heterocyclyl-lower alkyl, or $R_4$ and $R_5$ together also represent alkylene having 3 or 4 carbon atoms, $R_7$ represents hydrogen or optionally esterified or amidated carbonyl, and one of the radicals $A_1$ and $A_2$ represents a radical of the formula

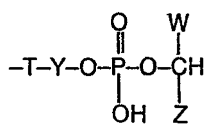

(II)

in which T represents HN or O, Y represents an optionally substituted alkylene group, which may also be interrupted by one or two oxycarbonyl and/or iminocarbonyl groups, W represents hydrogen, and Z represents a 1,2-dihydroxyethyl or 2-hydroxyethyl group in which at least one of the hydroxy groups is esterified by an optionally unsaturated aliphatic carboxylic acid having 12–90 C atoms, or is etherified by an optionally unsaturated aliphatic alcohol having 10–22 C atoms, or W and Z each represent a hydroxymethyl group esterified by an optionally unsaturated aliphatic carboxylic acid having 12–90 C atoms or etherified by an optionally unsaturated aliphatic alcohol having 10–22 C atoms, and the other of the radicals $A_1$ and $A_2$ is free or etherified hydroxy, amino, lower alkylamino or carboxamido-lower alkylamino, or salts thereof, characterised in that in a manner known per se,

a) a compound of the formula

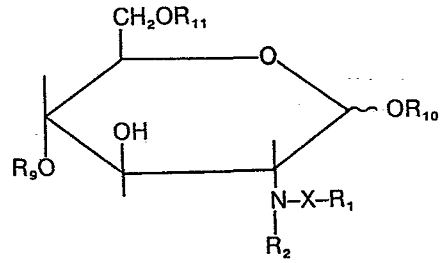

(V)

in which X, $R_1$ and $R_2$ have the meanings given above and hydroxy groups optionally present therein are protected by a protecting group that can readily be split off, and $R_9$, $R_{10}$ and $R_{11}$ represent a protecting group that can readily be split off, or a metal compound thereof, is reacted with a compound of the formula

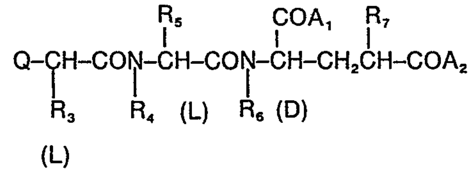

(VI)

or (D,L)

in which Q represents a reactive esterified hydroxy group, $R_3$, $R_4$, $R_5$, $R_6$, $R_7$, $A_1$ and $A_2$ have the meanings given above and hydroxy groups optionally present therein are protected by a protecting group that can readily be split off, and protecting groups present are split off, or

b) a compound of the formula

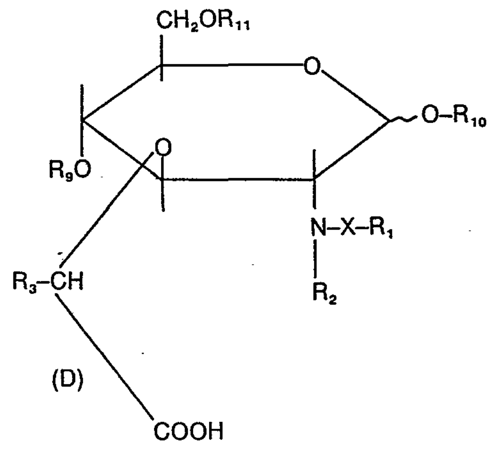

(VII)

in which X, $R_1$, $R_2$ and $R_3$ have the meanings given above, and $R_9$, $R_{10}$ and $R_{11}$ represent hydrogen or a protecting group that can readily be split off, or a derivative thereof, is condensed with a compound of the formula

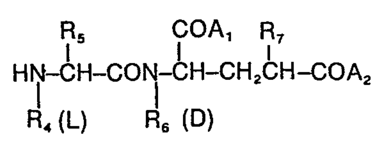

(VIII)

in which $R_4$, $R_5$, $R_6$, $R_7$, $A_1$ and $A_2$ have the meanings given above, provided that carboxy and, if desired, free hydroxy groups present in these radicals are protected by protecting groups that can readily be split off, or with a derivative thereof, and protecting groups present are split off, or

c) a compound of the formula

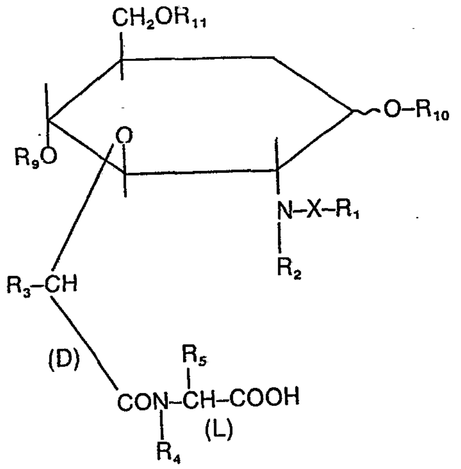

(IX)

in which X, $R_1$, $R_2$, $R_3$, $R_4$ and $R_5$ have the meanings given above, provided that free hydroxy groups contained therein are optionally protected by a protecting group that can readily be split off, and $R_9$, $R_{10}$ and $R_{11}$ represent hydrogen or protecting groups that can readily be split off, or a derivative thereof, is condensed with a compound of the formula

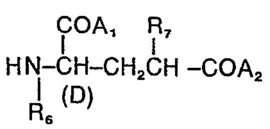

(X)

in which $R_6$, $R_7$, $A_1$ and $A_2$ have the meanings given above, provided that free carboxyl groups present in the radicals $R_7$, $A_1$ and $A_2$ are protected by protecting groups that can readily be split off, and protecting groups present are split off, or

d) a compound of the formula

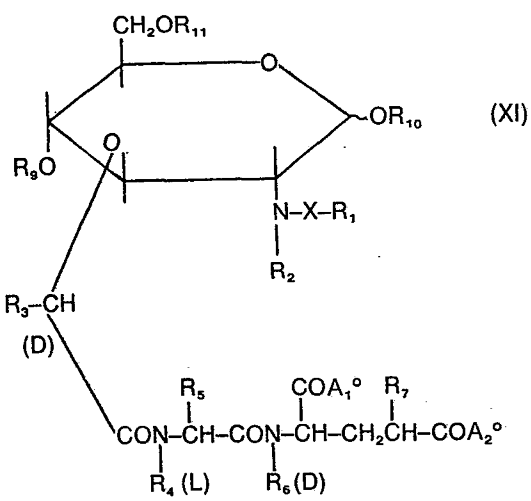

(XI)

in which X, $R_1$, $R_2$, $R_3$, $R_4$, $R_5$, $R_6$ and $R_7$ have the meanings given above, $R_9$ $R_{10}$ and $R_{11}$ represent hydrogen or a protecting group that can readily be split off, and one of the radicals $A_1°$ and $A_2°$ represent an activated hydroxy group, whilst the other represents etherified hydroxy, amino, lower alkylamino or carboxamido-lower alkylamino, is condensed with a compound of the formula

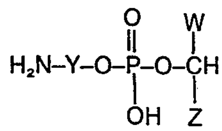

(XII)

in which Y, W and Z have the meanings given above, and protecting groups present are split off, or

e) a compound of the formula

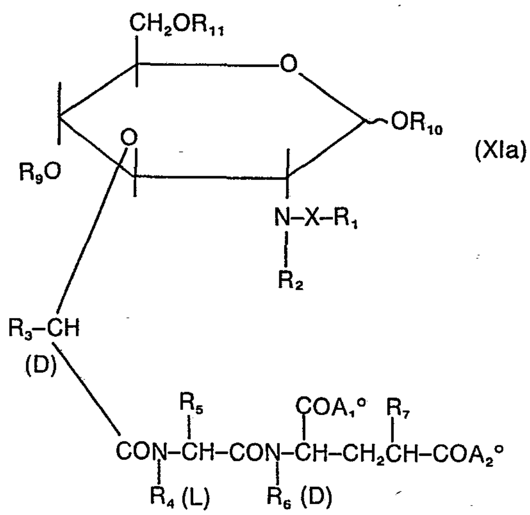

(XIa)

in which X, $R_1$, $R_2$, $R_3$, $R_4$, $R_5$, $R_6$ and $R_7$ have the meanings given above, $R_9$, $R_{10}$ and $R_{11}$ represent hydrogen or a protecting group that can readily be split off, and one of the radicals $A_1°$ and $A_2°$ represents a hydroxy group whilst the other represents etherified hydroxy, amino, lower alkylamino or carboxamido-lower alkylamino, is esterified with a compound of the formula

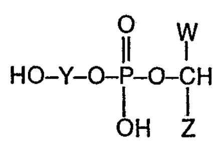

(XIIa)

in which Y, W and Z have the meanings given above, the acid XIa or the alcohol XIIa being present in reactive form, and protecting groups optionally present are split off, or

f) in a compound of the formula

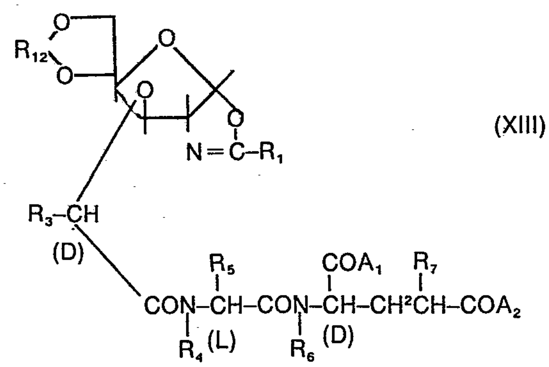

(XIII)

in which $R_1$, $R_3$, $R_4$, $R_5$, $R_6$, $R_7$, $A_1$ and $A_2$ have the meanings given above, and $R_{12}$ represents an alkylidene or cycloalkylidene group, the oxazoline and the dioxalane ring is split by acid means, and protecting groups optionally present are split off, or

g) a compound of the formula

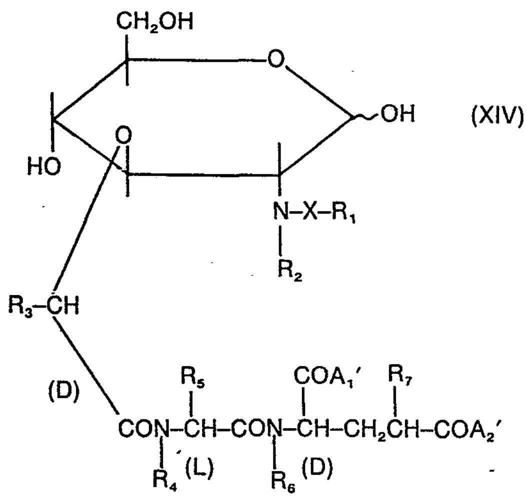

(XIV)

in which X, $R_1$, $R_2$, $R_3$, $R_4$, $R_5$, $R_6$ and $R_7$ have the meanings given above, and hydroxy groups present therein are optionally protected by protecting groups that can readily be split off, one of the radicals $A_1'$ and $A_2'$ represents a radical of the formula

$$-T-Y_1-M_1 \qquad (XV)$$

wherein T has the meaning given above, $Y_1$ represents optionally substituted alkylene which can be interrupted by an oxycarbonyl or iminocarbonyl group, and hydroxy groups present therein are optionally protected by protecting groups that can readily be split off, and $M_1$ represents a free amino group, a carboxylic acid group or an activated derivative of these two groups, whilst the other of the radicals $A_1'$ and $A_2'$ is etherified hydroxy or amino, lower alkylamino or carboxamido-lower alkylamino, is condensed with a compound of the formula

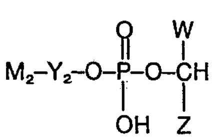

(XVI)

in which $M_2$ is a free amino group or an activated derivative thereof when $M_1$ represents a carboxylic acid group or an activated derivative thereof, or wherein $M_2$ is a carboxylic acid or an activated derivative thereof when $M_1$ represents a free amino group or an activated derivative thereof, $Y_2$ is optionally substituted alkylene which can be interrupted by an oxycarbonyl or iminocarbonyl group, with the proviso that the radicals $Y_1$ and $Y_2$ attached to one another by way of an iminocarbonyl group correspond to the aforementioned radical Y, and W and Z have the meanings given above, and in the radicals $Y_2$, W and Z, hydroxy groups present are optionally protected by protecting groups that are readily split off, and protecting groups optionally present are split off, or

h) a compound of the formula

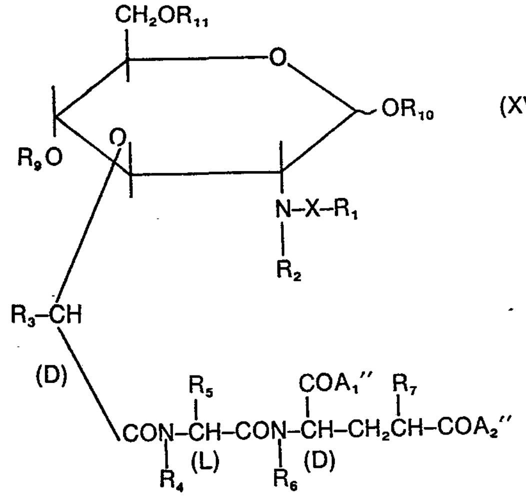

(XVII)

in which X, $R_1$, $R_2$, $R_3$, $R_4$, $R_5$, $R_6$ and $R_7$ have the meanings given above, $R_9$, $R_{10}$ and $R_{11}$ represent protecting groups that are readily split off, one of the radicals $A_1''$ and $A_2''$ is a radical of the formula

$$-T-Y_1-M_3 \qquad (XVIII)$$

in which T has the meaning given above, $Y_1$ represents optionally substituted alkylene which can be interrupted by an oxycarbonyl or iminocarbonyl group, and hydroxy groups present therein are optionally protected by protecting groups that can readily be split off, and $M_3$ represents a free hydroxy or carboxyl group which is optionally present in a reactive form, and the other of the radicals $A_1''$ and $A_2''$ is etherified hydroxy, amino, lower alkylamino or carboxamido-lower alkylamino, is esterified with a compound of the formula

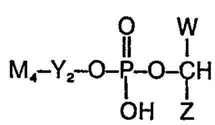

(XIX)

in which $M_4$ is a hydroxy group optionally present in a reactive form when $M_3$ is a carboxyl group optionally present in a reactive form, or in which $M_4$ is a carboxyl group optionally present in a reactive form when $M_3$ is a hydroxy group optionally present in an activated form, $Y_2$ is optionally substituted alkylene which can be interrupted by an oxycarbonyl or iminocarbonyl group, with the proviso that the radicals $Y_1$ and $Y_2$ attached to one another by way of an oxycarbonyl group correspond to the aforementioned radical Y, and W and Z have the meanings given above, and in the radicals $Y_2$, W and Z, hydroxy groups present are optionally protected by protecting groups that are readily split off, and protecting groups optionally present are split off, or

i) a compound of the formula

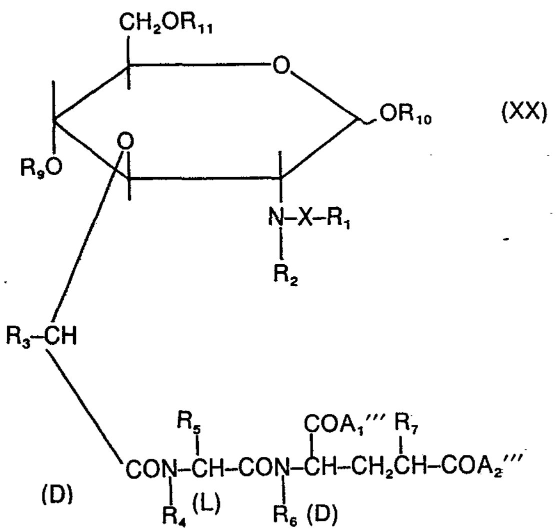

(XX)

in which X, $R_1$, $R_2$, $R_3$, $R_4$, $R_5$, $R_6$ and $R_7$ have the meanings given above, and hydroxy groups optionally present therein are protected by protecting groups that are readily split off, $R_9$, $R_{10}$ and $R_{11}$ represent protecting groups that can readily be split off, and one of the radicals $A_1'''$ and $A_2'''$ represents –T–Y–OH in which Y and T have the meanings given above, and hydroxy groups optionally present therein are protected by protecting groups that can readily be split off, and the other of the radicals $A_1'''$ and $A_2'''$ represents etherified hydroxy, amino, lower alkylamino or carboxamido-lower alkylamino, is reacted with a compound yielding the radical of the formula

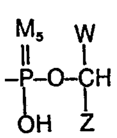

(XXI)

in which $=M_5$ represents an electron pair or oxo, and W and Z have the meanings given above, and hydroxy groups optionally present therein are protected by protecting groups that are readily split off, if $=M_5$ is an electron pair is oxidised with a weak oxidising agent, and protecting groups present are split off, or

k) a compound of the formula

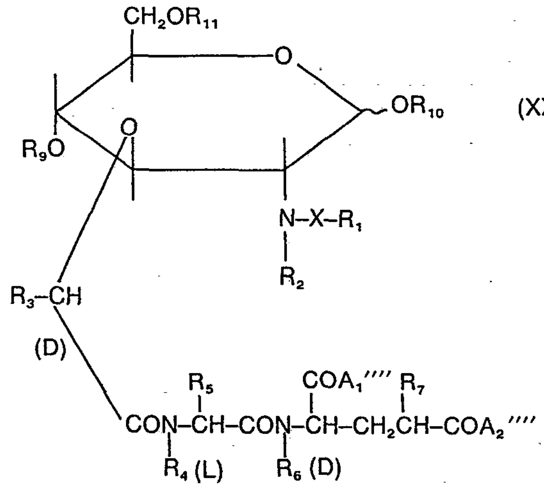

(XXIII)

in which X, $R_1$, $R_2$, $R_3$, $R_4$, $R_5$, $R_6$ and $R_7$ have the meanings given above, and hydroxy groups optionally present therein are protected by protecting groups that can readily be split off, $R_9$, $R_{10}$ and $R_{11}$ represent protecting groups that can readily be split off, and one of the radicals $A_1''''$ and $A_2''''$ represents

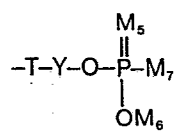

(XXIV)

in which T and Y have the meanings given above, and hydroxy groups optionally present therein are protected with protecting groups that are readily split off, and in which $=M_5$ is an electron pair or oxo, $M_6$ is a protecting group that can readily be split off, and $M_7$ represents free hydroxy or hydroxy present in the reactive form, and the other of the radicals $A_1''''$ and $A_2''''$ represents free or etherified hydroxy, amino, lower alkylamino or carboxamido-lower alkylamino, is reacted with a compound of the formula

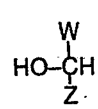

(XXV)

in which W and Z have the meanings given above, and hydroxy groups optionally present therein are protected by protecting groups that can readily be split off, if $=M_5$ represents an electron pair is oxidised with a weak oxidising agent, and protecting groups present are split off, or

l) a compound of the formula

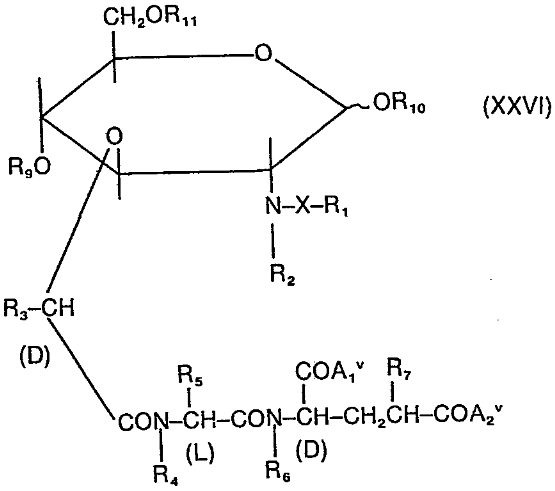 (XXVI)

in which X, $R_1$, $R_2$, $R_3$, $R_4$, $R_5$, $R_6$ and $R_7$ have the meanings given above, and hydroxy groups optionally present therein are protected by protecting groups that can readily be split off, $R_9$, $R_{10}$ and $R_{11}$ represent protecting groups that can readily be split off, and one of the radicals $A_1^v$ and $A_2^v$ represents a radical of the formula XXVII

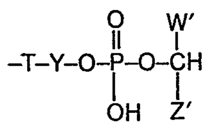 (XXVII)

in which T and Y have the meanings given above, and in a radical of the formula XXVII free functional groups, with the exception of the hydroxy group(s) participating in the reaction, are optionally protected by protecting groups which can readily be split off, and W′ and Z′ have the aforementioned meaning of W and Z, with the proviso however that at least one hydroxy group in the radical 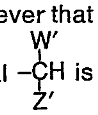 is present in the free form, and the other of the radicals $A_1^v$ and $A_2^v$ represents free or etherified hydroxy, amino, lower alkylamino or carboxamido-lower alkylamino, is esterified with an optionally unsaturated aliphatic carboxylic acid having 12–90 C atoms or with a reactive derivative thereof, or is etherified with an optionally unsaturated aliphatic alcohol having 10–22 C atoms or with a reactive derivative thereof, and the protecting groups present are split off, and, if desired, a resulting compound of the formula I is converted into salts thereof, the prefix "lower" employed in the foregoing denoting radicals having up to and including 7 C atoms.

2. Process according to claim 1, characterised in that muramyl peptides are produced in which Y is a lower alkylene radical which has 2 or 3 carbon atoms, or salts thereof.

3. Process according to claim 1, characterised in that muramyl peptides are produced in which Y is a radical of the formula

$-Y_1-COO-Y_2-$ (IIIa)

$-Y_1-OOC-Y_2-$ (IIIb)

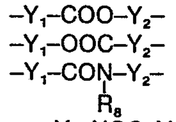 (IIIc)

or 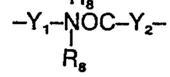 (IIId)

in which one of the radicals $Y_1$ and $Y_2$ represents an optionally substituted lower alkylene radical and the other represents an optionally substituted lower alkylene radical which may also be interrupted by oxycarbonyl or $-N-R_8$-iminocarbonyl, and $Y_1$ and $Y_2$ together have more than two carbon atoms, and $R_8$ represents hydrogen or lower alkyl, or salts thereof.

4. Process according to claim 1, characterised in that muramyl peptides of the formula I are produced in which X represents carbonyl, $R_1$ represents optionally substituted Alkyl or aryl, $R_2$, $R_3$, $R_4$ and $R_6$ represent hydrogen or lower alkyl, $R_5$ represents hydrogen, lower alkyl optionally substituted by hydroxy, lower alkoxy, mercapto, lower alkylmercapto or halogen; cycloalkyl or cycloalkyl-lower alkyl, wherein the cycloalkyl radical contains 4–6 carbon atoms; optionally substituted phenyl or phenyl-lower alkyl, heterocyclyl or heterocylyl-lower alkyl containing one or two aza atoms, or $R_4$ and $R_5$ together also represent alkylene having 3 or 4 carbon atoms, $R_7$ represents hydrogen and one of the radicals $A_1$ and $A_2$ represents a radical of the formula

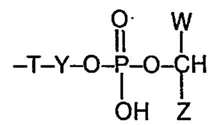 (II)

in which T represents HN or O, Y represents optionally substituted alkylene which may also be interrupted by carbonyloxy or carbonylimino, W represents hydrogen and Z represents a 1,2-dihydroxyethyl or 2-hydroxyethyl group, in which at least one of the hydroxy groups is esterified or etherified in the manner described in claim 1, and the other of the radicals $A_1$ and $A_2$ represents hydroxy, lower alkoxy, amino, lower alkylamino or carboxamido-lower alkylamino, or salts thereof.

5. Process according to claim 1, characterised in that muramyl peptides of the formula I are produced in which X represents carbonyl, $R_1$ represents lower alkyl optionally substituted by hydroxy, lower alkoxy or halogen, or phenyl optionally substituted by hydroxy, lower alkoxy, lower alkyl or halogen, $R_2$, $R_4$ and $R_6$ represent hydrogen, $R_3$ represents hydrogen or lower alkyl, $R_5$ represents hydrogen; lower alkyl having 1–3 carbon atoms and optionally substituted by hydroxy, lower alkoxy, mercapto, lower alkylmercapto or halogen; cycloalkyl or cycloalkyl-lower alkyl in which the cycloalkyl radical contains 4–6 carbon atoms and the lower alkyl radical contains 1–3 carbon atoms; phenyl or phenyl-lower alkyl having 1–3 carbon atoms in the lower alkyl radical both optionally substituted by hydroxy, lower alkoxy or halogen; or heterocyclyl or heterocyclyl-lower alkyl having 1–3 carbon atoms in the lower alkyl radical and each containing 5 or 6 ring members and one or two aza atoms, or $R_4$ and $R_5$ together represent also alkylene having 3 or 4 carbon atoms, $R_7$ represents hydrogen, and one of the radicals $A_1$ and $A_2$ represents a radical of the formula

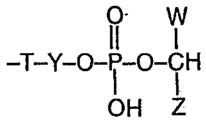

(II)

in which T represents HN or O, Y represents optionally substituted lower alkylene or a radical of the formula

$$-Y_1-COO-Y_2- \quad \text{(IIIa)}$$
$$\text{or} \quad -Y_1-CONH-Y_2- \quad \text{(IVc)}$$

in which $Y_1$ and $Y_2$ each represent optionally substituted lower alkylene, W represents hydrogen, and Z represents a 1,2-dihydroxyethyl or 2-hydroxyethyl group in which at least one of the hydroxy groups is esterified by an optionally singly or doubly unsaturated aliphatic carboxylic acid having 16–22 carbon atoms or by a natural or synthetic mycolic acid, or is etherified by an optionally singly or doubly unsaturated aliphatic alcohol having 12–18 carbon atoms, and the other of the radicals $A_1$ and $A_2$ represents hydroxy, lower alkoxy, amino, lower alkylamino or carboxamido-lower alkylamino, or salts thereof.

6. Process according to claim 1, characterised in that muramyl peptides of the formula I are produced in which X represents carbonyl, $R_1$ represents lower alkyl having 1–3 carbon atoms or phenyl, $R_2$, $R_4$ and $R_6$ represent hydrogen, $R_3$ represents hydrogen or lower alkyl having 1–3 carbon atoms, $R_5$ represents hydrogen; lower alkyl having 1–3 carbon atoms and optionally substituted by hydroxy, methoxy, mercapto, methylmercapto or halogen; phenyl or phenylmethyl optionally substituted by hydroxy, methoxy or halogen; heterocyclyl or heterocyclylmethyl each containing 5 ring members and one or two aza atoms, or $R_4$ and $R_5$ together represent also trimethylene, $R_7$ represents hydrogen, and one of the radicals $A_1$ and $A_2$ represents a radical of the formula

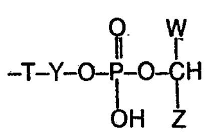

(II)

in which T represents HN or O, Y represents lower alkylene having 2 or 3 carbon atoms or a radical of the formula (IIIa) or (IVc) given in claim 5, in which $Y_1$ and $Y_2$ independently of one another each represent lower alkylene having 1–3 carbon atoms, which is unsubstituted or substituted by optionally hydroxy-, lower-alkoxy-, mercapto- or lower-alkylmercapto-substituted lower alkyl having 1–3 carbon atoms, or by optionally hydroxy-, methoxy- or halogen-substituted phenyl or phenyl-lower-alkyl, or by heterocyclyl or heterocyclyl-lower-alkyl having 1–3 carbon atoms in the lower alkyl radical and each containing 5 or 6 ring members and one or two aza atoms, W represents hydrogen, and Z represents a 1,2-dihydroxyethyl or 2-hydroxyethyl group in which at least one of the hydroxy groups is esterified with an optionally singly or doubly unsaturated aliphatic carboxylic acid having 16 to 20 carbon atoms or is etherified with an optionally singly or doubly unsaturated aliphatic alcohol having 12–18 carbon atoms, and the other of the radicals $A_1$ and $A_2$ represents hydroxy, lower alkoxy, amino, lower alkylamino or carboxamido-lower alkylamino, or salts thereof.

7. Process for producing muramyl peptides of the formula I given in claim 1, in which X represents carbonyl or carbonyloxy, $R_1$ represents lower alkyl having 1–3 carbon atoms or phenyl, $R_2$, $R_4$ and $R_6$ represent hydrogen, $R^3$ represents hydrogen or lower alkyl having 1–3 carbon atoms, $R^5$ represents hydrogen or lower alkyl, $R^7$ represents hydrogen, $A_1$ represents amino, lower alkylamino, hydroxy or lower alkoxy, and $A_2$ represents a radical of the formula (II) given in claim 1, in which T represents NH or O, Y represents lower alkylene having 2 or 3 carbon atoms or a radical of the formula

$$-CH_2-CO-NH-CH_2-CH_2-,$$

W represents hydrogen, and Z represents a 1,2-dihydroxyethyl or 2-hydroxyethyl group, in which one or two hydroxy groups are esterified by identical or different, optionally singly or doubly unsaturated alkanecarboxylic acids having 16–22 carbon atoms, or etherified by an optionally singly or doubly unsaturated alkanol having 12–18 carbon atoms, or wherein two adjacent hydroxy groups are ketalised, by formation of a dioxolane ring, with a bis-lower-alkyl ketone, or acetalised with an unsubstituted aliphatic aldehyde having up to 20 C atoms, or wherein W and Z each represent a hydroxymethyl group, which is esterified with an optionally singly or doubly unsaturated alkanecarboxylic acid having 16–22 carbon atoms, or etherified with an optionally singly or doubly unsaturated alkanol having 12–18 carbon atoms, or salts thereof, the process being analogous to one of the processes mentioned in claim 1.

8. Process according to claim 7, characterised in that compounds are produced, or salts thereof, wherein the meanings for $A_1$ and $A_2$ are exchanged.

9. Process according to claim 1, characterised in that
N-acetylmuramyl-L-alanyl-D-isoglutamine-2-(1′,2′-dipalmitoyl-sn-glycero-3′-hydroxyphosphoryloxy)-ethylamide
or a salt thereof is produced.

10. Process according to claim 1, characterised in that N-acetyl-desmethylmuramyl-L-alanyl-D-isoglutamine-2-(1′,2′-dipalmitoyl-sn-glycero-3′-hydroxyphosphoryloxy)-ethylamide or a salt thereof is produced.

11. Process according to claim 1, characterised in that N-acetylmuramyl-L-alanyl-D-isoglutaminyl-γ-oxyacetic acid-2-(1′,2′-dipalmitoyl-sn-glycero-3′-hydroxyphosphoryloxy)-ethylamide or a salt thereof is produced.

12. Process according to claim 1, characterised in that N-acetylmuramyl-L-alanyl-D-isoglutaminyl-L-alanine-2-(1′,2′-dipalmitoyl-sn-glycero-3′-hydroxyphosphoryloxy)-ethylamide or a salt thereof is produced.

13. Process according to claim 1, characterised in that N-acetyl-desmethylmuramyl-L-alanyl-D-isoglutamine-2-(1′,2′-dihexadecyl-sn-glycero-3′-hydroxyphosphoryloxy)-ethylamide or a salt thereof is produced.

14. Process according to claim 1, characterised in that N-acetyl-desmethylmuramyl-L-alanyl-D-isoglutamine-2-(1′-hexadecyl-sn-glycero-3′-hydroxyphosphoryloxy)-ethylamide or a salt thereof is produced.

15. Process according to claim 1, characterised in that N-acetyl-desmethylmuramyl-L-alanyl-D-isoglutamine-2-(3′-palmitoyl-rac-glycero-1′-hydroxyphosphoryloxy)-ethylamide or a salt thereof is produced.

16. Process according to claim 1, characterised in that N-acetyl-desmethylmuramyl-L-alanyl-D-isoglutamine-2-(1′-palmitoyl-2′-oleoyl-sn-glycero-3′-hydroxyphosphoryloxy)-ethylamide or a salt thereof is produced.

17. Process according to claim 1, characterised in that N-acetyl-desmethylmuramyl-L-alanyl-D-isoglutamine-2-(1′-O-palmitoyl-propanediol-3′-O-hydroxyphosphoryloxy)-ethylamide or a salt thereof is produced.

18. Process according to claim 1, characterised in that N-acetyl-muramyl-L-α-aminobutyryl-D-isoglutaminyl-L-alanine-2-(1′,2′-dipalmitoyl-sn-glycero-3′-hydroxyphosphoryloxy)-ethylamide or a salt thereof is produced.

19. Process according to claim 1, characterised in that N-acetyl-muramyl-L-α-aminobutyryl-D-isoglutaminyl-L-alanine-2-(1′,2′-dipalmitoyl-rac-glycero-3′-hydroxyphosphoryloxy)-ethylamide or a salt thereof is produced.

20. Process according to claim 1, characterised in that N-benzoyl-muramyl-L-α-aminobutyryl-D-isoglutaminyl-L-alanine-2-(1′,2′-dipalmitoyl-sn-glycero-3′-hydroxyphosphoryloxy)-ethylamide or a salt thereof is produced.

21. Process according to claim 1, characterised in that N-propionyl-desmethylmuramyl-L-alanyl-D-isoglutaminyl-L-alanine-2-(1′,2′-dipalmitoyl-sn-glycero-3′-hydroxyphosphoryloxy)-ethylamide or a salt thereof is produced.

22. Process according to claim 1, characterised in that N-acetyl-muramyl-L-valyl-D-isoglutaminyl-L-alanine-2-(1′,2′-dipalmitoyl-sn-glycero-3′-hydroxyphosphoryloxy)-ethylamide or a salt thereof is produced.

23. Process according to claim 1, characterised in that N-benzoyl-desmethylmuramyl-L-α-aminobutyryl-D-isoglutaminyl-L-alanine-2-(1′,2′-dipalmitoyl-sn-glycero-3′-hydroxyphosphoryloxy)-ethylamide or a salt thereof is produced.

24. Process according to claim 1, characterised in that N-acetyl-desmethylmuramyl-L-alanyl-D-glutamyl-($C_\alpha$)-glycinamide-($C_\gamma$)-L-alanine-2-(1′,2′-dipalmitoyl-sn-glycero-3′-hydroxyphosphoryloxy)-ethylamide or a salt thereof is produced.

25. Process according to claim 1, characterised in that N-acetyl-muramyl-L-alanyl-D-isoglutaminyl-L-alanine-2-(3′-palmitoyl-rac-glycero-1′-hydroxyphosphoryloxy)-ethylamide or a salt thereof is produced.

26. Process according to claim 1, characterised in that N-acetyl-muramyl-L-alanyl-D-isoglutaminyl-L-alanine-2-(1′,2′-hexadecylidene-sn-glycero-3′-hydroxyphosphoryloxy)-ethylamide or a salt thereof is produced.

27. Process according to claim 1, characterised in that N-acetyl-desmethylmuramyl-L-alanyl-D-isoglutaminyl-L-alanine-2-(1′,2′-hexadecylidene-sn-glycero-3′-hydroxyphosphoryloxy)-ethylamide or a salt thereof is produced.

28. Process according to claim 1, characterised in that a pharmaceutically applicable salt of a compound according to one of claims 1–27 is produced.

29. Process according to claim 28, characterised in that a sodium salt is produced.

30. Process according to one of the claims 1 to 29, characterised in that in a compound of the formula I in which the hydroxyl groups are protected in the 1-, 4- and/or 6-position of the sugar moiety by protecting groups, these protecting groups are split off.

31. Process according to one of the claims 1–29, characterised in that in a compound of the formula I in which one or more of the carboxyl groups are protected by protecting groups, these protecting groups are split off.

32. Process according to one of the claims 1–29, characterised in that the protecting groups are split off by treating with an acid.

33. Process according to one of the claims 1–29, characterised in that there are used as compounds having one or more reactive esterified hydroxy groups compounds in which the hydroxy group(s) is(are) esterified with a strong inorganic acid or a sulphonic acid.

34. Process according to one of the claims 1–29, characterised in that there are used as compounds having one or more reactive esterified hydroxy groups compounds in which the hydroxy group(s) is(are) esterified with a hydrohalic acid.

35. Process according to one of the claims 1–29, characterised in that there are used as compounds having one or more hydroxy-protecting groups compounds in which the hydroxy group(s) is(are) protected by acyl radicals, aroyl radicals, radicals derived from carbonic acid derivatives, alkyl radicals branched in the α-position, α-mono-, α-di- or α-tri-aryl-lower alkyl radicals, or by acetal-forming radicals.

36. Process according to one of the claims 1–29, characterised in that there are used as compounds having one or more hydroxy-protecting groups, compounds in which the hydroxy group(s) is(are) protected by lower alkanoyl, benzoyl, benzyloxycarbonyl, lower alkoxycarbonyl or tert.-butyl radicals, or by optionally substituted benzyl, triphenylmethyl or tetrahydropyranyl radicals.

37. Process according to one of the claims 1–29, characterised in that there are used as compounds having one or more carboxy-protecting groups compounds in which the carboxy group(s) is(are) protected by tert.-butyl or benzyl radicals or benzhydryl or triphenylmethyl radicals optionally substituted by halogen or lower alkoxy.

38. Process according to one of the claims 1–29, characterised in that there are used as compounds having one or more activated carboxylic acid groups compounds in which the carboxylic acid group(s) is(are) present in the form of an anhydride, azide, activated amide or activated ester.

39. Process according to claim 38, characterised in that there are used as acid anhydrides those with carbonic acid lower alkyl esters, as acid amides imidazolides or isoxazolides and as activated esters, the cyanomethyl or carboxymethyl ester, acetylaminoethylthio ester, p-nitro- or 2,4,5-trichlorophenyl ester, N-hydroxy-succinimide ester, N-hydroxy-phthalimide ester or N-hydroxy-piperidine ester, 8-hydroxy-quinoline ester, or methoxyethoxythio ester, or esters obtained by reacting with carbodiimide with the addition of N-hydroxysuccinimide or a 1-hydroxybenztriazole or 3-hydroxy-4-oxo-3,4-dihydrobenzo[d]-1,2,3-triazine.

40. Mixing process for producing a pharmaceutical preparation, characterised in that a compound, which has been obtained according to any one of claims 1 to 39, is mixed together with a pharmaceutical carrier substance.

**REVENDICATIONS pour les Etats contractants: BE, CH, DE, FR, GB, IT, LI, LU, NL, SE**

1. Muramyl-peptides de formule

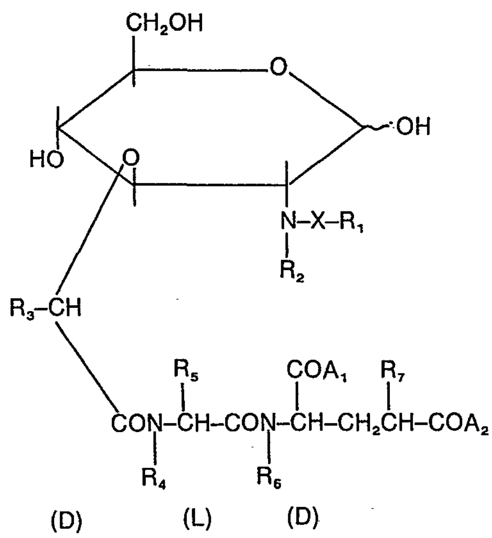

(I)

où X représente un carbonyle ou un carbonyloxy, $R_1$ représente un alcoyle ou un aryle éventuellement substitué, $R_2$, $R_4$ et $R_6$ représentent un hydrogène ou un alcoyle inférieur, $R_3$ représente un hydrogène ou un alcoyle inférieur, $R_5$ représente un hydrogène, un alcoyle inférieur, un hydroxyalcoyle inférieur libre ou fonctionnellement modifié, un mercapto-alcoyle inférieur libre ou fonctionnellement modifié, un aminoalcoyle inférieur éventuellement substitué, un cycloalcoyle, un cycloalcoylalcoyle inférieur, un aryle ou aralcoyle éventuellement substitué, un hétérocyclyle ou hétérocyclyl-alcoyle inférieur contenant de l'azote, ou $R_4$ et $R_5$ représentent également ensemble un alcoylène en $C_3$ à $C_4$, $R_7$ représente un hydrogène ou un carboxyle éventuellement estérifié ou amidé et l'un des radicaux $A_1$ et $A_2$ représente un radical de formule

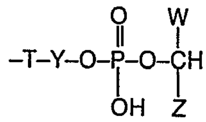

(II)

où T représente NH ou O, Y représente un groupe alcoylène éventuellement substitué, qui peut également être interrompu par un ou deux radicaux oxycarbonyle et/ou iminocarbonyle, W représente un hydrogène et Z un groupe 1,2-dihydroxyéthyle ou 2-hydroxyéthyle, où au moins l'un des groupes hydroxy est estérifié avec un acide carboxylique aliphatique éventuellement insaturé en $C_{12}$ à $C_{90}$ ou est éthérifié avec un alcool aliphatique éventuellement insaturé en $C_{10}$ à $C_{22}$, ou W et Z représentent chacun un groupe hydroxyméthyle estérifié avec un acide carboxylique aliphatique éventuellement insaturé en $C_{12}$ à $C_{90}$ ou éthérifié avec un alcool aliphatique éventuellement insaturé en $C_{10}$ à $C_{22}$, et l'autre des radicaux $A_1$ et $A_2$ est un hydroxy, un amino, un alcoyle inférieur-amino ou un carboxamido-alcoyle inférieur-amino libre ou éthérifié, et leurs sels, où le préfixe «inférieur» désigne des radicaux ayant jusqu'à 7 atomes de carbone compris.

2. Muramyl-peptides selon la revendication 1, où Y est un radical alcoylène inférieur en $C_2$ à $C_3$, et leurs sels.

3. Muramyl-peptides selon la revendication 1, où Y représente un radical de formule

$-Y_1-COO-Y_2-$ (IIIa)

$-Y_1-OOC-Y_2-$ (IIIb)

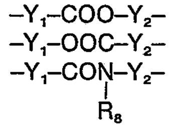

(IIIc)

ou 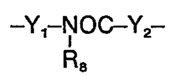 (IIId)

où l'un des radicaux $Y_1$ et $Y_2$ représente un radical alcoylène inférieur éventuellement substitué et l'autre un radical alcoylène inférieur éventuellement substitué, qui peut également être interrompu par un oxycarbonyle ou un $N-R_8$-iminocarbonyle, et $Y_1$ et $Y_2$ présentent ensemble plus de deux atomes de carbone, et $R_8$ est un hydrogène ou un alcoyle inférieur, et leurs sels.

4. Muramyl-peptides de formule I selon la revendication 1, où X représente un carbonyle, $R_1$ représente un alcoyle ou aryle éventuellement substitué, $R_2$, $R_3$, $R_4$ et $R_6$ représentent un hydrogène ou un alcoyle inférieur, $R_5$ représente un hydrogène, un alcoyle inférieur, un cycloalcoyle ou un cycloalcoyle-alcoyle inférieur éventuellement substitué par un hydroxy, un alcoxy inférieur, un mercapto ou un alcoyle inférieur-mercapto, ou un halogène, où le radical cycloalcoyle contient de 4 à 6 atomes de carbone, un phényle ou phényl-alcoyle inférieur éventuellement substitué, un hétérocyclyle ou hétérocyclyl-alcoyle inférieur contenant un ou deux atomes d'aza, ou bien où $R_4$ et $R_5$ représentent également ensemble un alcoylène en $C_3$ à $C_4$, $R_7$ représente un hydrogène et l'un des radicaux $A_1$ et $A_2$ représente un radical de formule

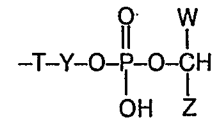

(II)

où T représente NH ou O, Y représente un alcoylène éventuellement substitué, qui peut également être interrompu par un carbonyloxy ou un carbonylimino, W représente un hydrogène et Z représente un groupe 1,2-dihydroxyéthyle ou 2-hydroxyéthyle, où au moins l'un des groupes hydroxy est estérifié ou éthérifié de la manière indiquée dans la revendication 1, et l'autre des radicaux $A_1$ et $A_2$ est un hydroxy, un alcoxy inférieur, un amino, un alcoyle inférieur-amino ou un carboxamido-alcoyle inférieur-amino, et leurs sels.

5. Muramyl-peptides de formule I selon la revendication 1, où X représente un carbonyle, $R_1$ représente un alcoyle inférieur éventuellement substitué par un hydroxy, un alcoxy inférieur ou un halogène, ou un phényle éventuellement substitué par un hydroxy, un alcoxy inférieur, un alcoyle inférieur ou un halogène, $R_2$, $R_4$ et $R_6$ représentent un hydrogène, $R_3$ représente un hydrogène ou un alcoyle inférieur, $R_5$ représente un hydrogène, un alcoyle inférieur en $C_1$ à $C_3$ éventuellement substitué par un hydroxy, un alcoxy inférieur, un mercapto, un alcoyle inférieur-mercapto ou un halogène, un cycloalcoyle ou un cycloalcoyl-alcoyle inférieur, où le radical cycloalcoyle contient de 4 à 6 atomes de carbone et le radical alcoyle inférieur contient de 1 à 3 atomes de carbone, un phényle ou un phényl-alcoyle inférieur avec de 1 à 3 atomes de carbone dans le radical alcoyle inférieur éventuellement substitué par un hydroxy, un alcoxy inférieur ou un halogène, un hétérocyclyle ou hétérocyclyl-alcoyle inférieur avec 5 à 6 chaînons et de 1 à 3 atomes de carbone dans le radical alcoyle inférieur contenant 1 ou 2 atomes d'aza ou bien où $R_4$ et $R_5$ forment également ensemble un alcoylène en $C_3$ à $C_4$, $R_7$ représente un hydrogène, et l'un des radicaux $A_1$ et $A_2$ représente un radical de formule

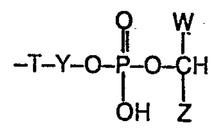 , (II)

où T représente HN ou O, Y représente un alcoyle inférieur éventuellement substitué ou un radical de formule

$-Y_1-COO-Y_2-$ (IIIa)

ou $-Y_1-CONH-Y_2-$ (IVc)

où $Y_1$ et $Y_2$ représentent chacun un alcoylène inférieur éventuellement substitué, W représente un hydrogène et Z représente un groupe 1,2-dihydroxyéthyle ou 2-hydroxyéthyle, où au moins l'un des groupes hydroxy est estérifié avec un acide carboxylique aliphatique éventuellement

mono- ou bi-insaturé en $C_{16}$ à $C_{22}$ ou avec un acide mycolique naturel ou synthétique, ou éthérifié avec un alcool aliphatique éventuellement mono- ou bi-insaturé en $C_{12}$ à $C_{18}$, et l'autre des radicaux $A_1$ et $A_2$ est un hydroxy, un alcoxy inférieur, un amino, un alcoyle inférieur-amino ou un carboxamido-alcoyle inférieur-amino, et leurs sels.

6. Muramyl-peptides de formule I selon la revendication I, où X représente un carbonyle, $R_1$ représente un alcoyle inférieur en $C_1$ à $C_3$ ou un phényle, $R_2$, $R_4$ et $R_6$ représentent un hydrogène, $R_3$ représente un hydrogène ou un alcoyle inférieur en $C_1$ à $C_3$, $R_5$ représente un hydrogène, un alcoyle inférieur en $C_1$ à $C_3$ éventuellement substitué par un hydroxy, un méthoxy, un mercapto, un méthylmercapto ou un halogène, un phényle ou phénylméthyle éventuellement substitué par un hydroxy, un méthoxy ou un halogène, un hétérocyclyle ou hétérocyclylméthyle contenant 1 ou 2 atomes d'aza avec 5 chaînons, ou $R_4$ et $R_5$ représentent également ensemble un triméthylène, $R_7$ représente un hydrogène et l'un des radicaux $A_1$ et $A_2$ représente un radical de formule

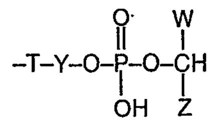

(II)

où T représente HN ou O, Y représente un alcoylène inférieur en $C_2$ à $C_3$ ou un radical de formules (IIIa) ou (IVc), représentées dans la revendication 5, où $Y_1$ et $Y_2$ représentent chacun indépendamment l'un de l'autre un alcoylène inférieur en $C_1$ à $C_3$, qui est non substitué ou substitué par un alcoyle inférieur en $C_1$ à $C_3$ éventuellement substitué par un hydroxy, un alcoxy inférieur, un mercapto ou un alcoyle inférieur-mercapto ou par un phényle ou phényl-alcoyle inférieur éventuellement substitué par un hydroxy, un méthoxy ou un halogène ou par un hétérocyclyle ou hétérocyclyl-alcoyle inférieur ayant de 5 à 6 chaînons et de 1 à 3 atomes de carbone dans le radical alcoyle inférieur, et contenant 1 ou 2 atomes d'aza, W représente un hydrogène et Z est un groupe 1,2-dihydroxyéthyle ou 2-hydroxyéthyle, où au moins l'un des groupes hydroxy est estérifié avec un acide carboxylique aliphatique en $C_{16}$ à $C_{20}$ éventuellement mono- ou bi-insaturé ou éthérifié avec un alcool aliphatique en $C_{12}$ à $C_{18}$ éventuellement mono- ou bi-insaturé, et l'autre des radicaux $A_1$ et $A_2$ est un hydroxy, un alcoxy inférieur, un amino, un alcoyle inférieur-amino ou un carboxamido-alcoyle inférieur-amino, et leurs sels.

7. Muramyl-peptides de formule I représentée dans la revendication 1, ou X représente un carbonyle ou un carbonyloxy, $R_1$ représente un alcoyle inférieur en $C_1$ à $C_3$ ou un phényle, $R_2$, $R_4$ et $R_6$ représentent un hydrogène, $R_3$ représente un hydrogène ou un alcoyle inférieur en $C_1$ à $C_3$, $R_5$ représente un hydrogène ou un alcoyle inférieur, $R_7$ représente un hydrogène, $A_1$ représente un amino, un alcoyle inférieur-amino, un hydroxy ou un alcoxy inférieur et $A_2$ représente un radical de la formule (II) représentée dans la revendication 1, où T représente NH ou O, Y représente un alcoylène inférieur en $C_2$ à $C_3$ ou un radical de formule

$$-CH_2-CO-NH-CH_2-CH_2-$$

W représente un hydrogène et Z représente un groupe 1,2-dihydroxyéthyle ou 2-hydroxyéthyle, où 1 ou 2 groupes hydroxy sont estérifiés avec des acides alcane-carboxyliques en $C_{16}$ à $C_{22}$ semblables ou différents éventuellement mono- ou bi-insaturés ou éthérifiés avec un alcanol en $C_{12}$ à $C_{18}$ éventuellement mono- ou bi-insaturé, ou bien où deux groupes hydroxy voisins sont cétalisés formellement avec une bis-alcoyle inférieur-cétone avec formation d'un noyau dioxolane ou acétalisés avec un aldéhyde aliphatique non substitué ayant jusqu'à 20 atomes de carbone, ou bien où W et Z représentent chacun un groupe hydroxyméthyle, qui est estérifié avec un acide alcanecarboxylique en $C_{16}$ à $C_{22}$ éventuellement mono- ou bi-insaturé ou éthérifié avec un alcool insaturé en $C_{12}$ à $C_{18}$ éventuellement mono- ou bi-insaturé, et leurs sels, où le préfixe «inférieur» désigne des radicaux ayant jusqu'à 7 atomes de carbone compris.

8. Composés selon la revendication 7 où les significations de $A_1$ et $A_2$ sont échangées, et leurs sels.

9. N-acétylmuramyl-L-alanyl-D-isoglutamine-2-(1',2'-dipalmitoyl-sn-glycéro-3'-hydroxyphosphoryloxy)-éthylamide et ses sels selon la revendication 1.

10. N-acétyl-desméthylmuramyl-L-alanyl-D-isoglutamine-2-(1',2'-dipalmitoyl-sn-glycéro-3'-hydroxyphosphoryloxy)-éthylamide et ses sels selon la revendication 1.

11. 2-(1',2'-dipalmitoyl-sn-glycéro-3'-hydroxyphosphoryloxy)-éthylamide de l'acide N-acétylmuramyl-L-alanyl-D-isoglutaminyl-γ-oxy-acétique et ses sels selon la revendication 1.

12. N-Acétylmuramyl-L-alanyl-D-isoglutaminyl-L-alanine-2-(1',2'-dipalmitoyl-sn-glycéro-3'-hydroxyphosphoryloxy)-éthylamide et ses sels selon la revendication 1.

13. N-Acétyl-desméthylmuramyl-L-alanyl-D-isoglutamin-2-(1',2'-dihexadécyl-sn-glycéro-3'-hydroxyphosphoryloxy)-éthylamide et ses sels selon la revendication 1.

14. N-Acétyl-desméthylmuramyl-L-alanyl-D-isoglutamin-2-(1'-hexadécyl-sn-glycéro-3'-hydroxyphosphoryloxy)-éthylamide et ses sels selon la revendication 1.

15. N-Acétyl-desméthylmuramyl-L-alanyl-D-isoglutamine-2-(3'-palmitoyl-rac-glycéro-1'-hydroxyphosphoryloxy)-éthylamide et ses sels selon la revendication 1.

16. N-Acétyl-desméthylmuramyl-L-alanyl-D-isoglutamine-2-(1'-palmitoyl-2'-oléoyl-sn-glycéro-3'-hydroxyphosphoryloxy)-éthylamide et ses sels selon la revendication 1.

17. N-acétyl-desméthylmuramyl-L-alanyl-D-isoglutamine-2-(1'-O-palmitoyl-propandiol-3′-O-hydroxyphosphoryloxy)-éthylamide et ses sels selon la revendication 1.

18. N-Acétyl-muramyl-L-α-aminobutyryl-D-isoglutaminyl-L-alanine-2-(1′,2′-dipalmitoyl-sn-glycéro-3′-hydroxyphosphoryloxy)-ethylamide et ses sels selon la revendication 1.

19. N-Acétyl-muramyl-L-α-aminobutyryl-D-isoglutaminyl-L-alanine-2-(1′,2′-dipalmitoyl-rac-glycéro-3′-hydroxyphosphoryloxy)-éthylamide et ses sels selon la revendication 1.

20. N-Benzoyl-muramyl-L-α-aminobutyryl-D-isoglutaminyl-L-alanine-2-(1′,2′-dipalmitoyl-sn-glycéro-3′-hydroxyphosphoryloxy)-éthylamide et ses sels selon la revendication 1.

21. N-Propionyl-desméthylmuramyl-L-alanyl-D-isoglutaminyl-L-alanine-2-(1′,2′-dipalmitoyl-sn-glycéro-3′-hydroxyphosphoryloxy)-éthylamide et ses sels selon la revendication 1.

22. N-Acétyl-muramyl-L-valyl-D-isoglutaminyl-L-alanine-2-(1′,2′-dipalmitoyl-sn-glycéro-3′-hydroxyphosphoryloxy)-éthylamide et ses sels selon la revendication 1.

23. N-Benzoyl-desméthylmuramyl-L-α-aminobutyryl-D-isoglutaminyl-L-alanine-2-(1′2′-dipalmitoyl-sn-glycéro-3′-hydroxyphosphoryloxy)-éthylamide et ses sels selon la revendication 1.

24. N-Acétyl-desméthylmuramyl-L-alanyl-D-glutamyl-($C_α$)-glycinamide-($C_γ$)-L-alanine-2-(1′,2′-dipalmitoyl-sn-glycéro-3′-hydroxyphosphoryloxy)-éthylamide et ses sels selon la revendication 1.

25. N-Acétyl-muramyl-L-alanyl-D-isoglutaminyl-L-alanine-2-(3′-palmitoyl-rac-glycéro-1′-hydroxyphosphoryloxy)-éthylamide et ses sels selon la revendication 1.

26. N-Acétyl-muramyl-L-alanyl-D-isoglutaminyl-L-alanine-2-(1′,2′-hexadécylidène-sn-glycéro-3′-hydroxyphosphoryloxy)-éthylamide et ses sels selon la revendication 1.

27. N-Acétyl-desméthylmuramyl-L-alanyl-D-isoglutaminyl-L-alanine-2-(1′,2′-hexadécylidène-sn-glycéro-3′-hydroxyphosphoryloxy)-éthylamide et ses sels selon la revendication 1.

28. Les sels pharmaceutiquement acceptables des composés selon les revendications 1–27.

29. Les sels de sodium des composés selon les revendications 1–27.

30. Préparations pharmaceutiques contenant un des composés mentionnés dans les revendications 1–29 avec un support pharmaceutique.

31. Agent immunisant contenant l'un des composés mentionnés dans l'une des revendications 1–29 avec un support approprié.

32. Un des composés mentionnés dans les revendications 1–29 aux fins d'application comme médicament.

33. Application des composés mentionnés dans les revendications 1–29 à la préparation d'un agent immunisant ou d'un vaccin.

34. Procédé de préparation de muramylpeptides de formule I selon l'une des revendications 1–29 et de leurs sels, caractérisé en ce que de façon classique

a) on fait réagir un composé de formule

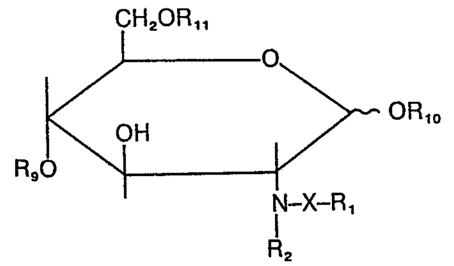

(V)

où X, $R_1$ et $R_2$ ont la signification donnée ci-dessus, où les groupes hydroxy qui y sont éventuellement présents sont protégés avec un groupe protecteur facilement séparable, et $R_9$, $R_{10}$ et $R_{11}$ représentent un groupe protecteur facilement séparable, ou un de ses composés métalliques, avec un composé de formule

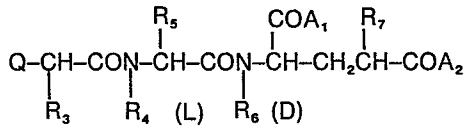

(VI)

(L)

ou (D,L)

où Q représente un groupe hydroxy estérifié réactif, $R_3$, $R_4$, $R_5$, $R_6$, $R_7$, $A_1$ et $A_2$ ont la signification donnée ci-dessus et les groupes hydroxy qui y sont éventuellement présents sont protégés par un groupe protecteur facilement séparable, et on sépare les groupes protecteurs présents, ou

b) on condense un composé de formule

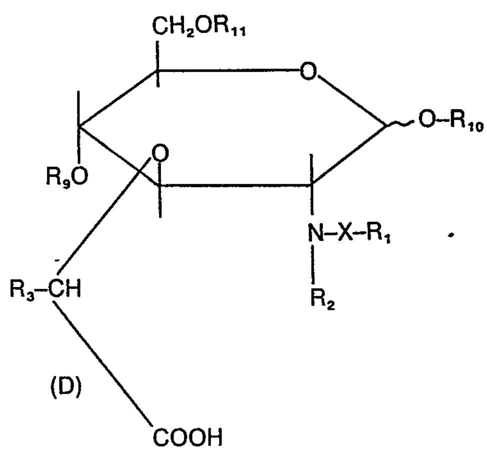

(VII)

où X, $R_1$, $R_2$ et $R_3$ ont la signification mentionnée ci-dessus et $R_9$, $R_{10}$ et $R_{11}$ représentent un hydro-

gène ou un groupe protecteur facilement séparable, ou un de ses dérivés, avec un composé de formule

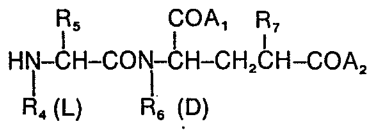

(VIII)

où $R_4$, $R_5$, $R_6$, $R_7$, $A_1$ et $A_2$ ont la signification donnée ci-dessus, avec la précision que les groupes carboxy et, si on le désire, les groupes hydroxy libres présents dans ces radicaux sont protégés par les groupes protecteurs facilement séparables, ou un de ses dérivés, et on sépare les groupes protecteurs présents, ou

c) on condense un composé de formule

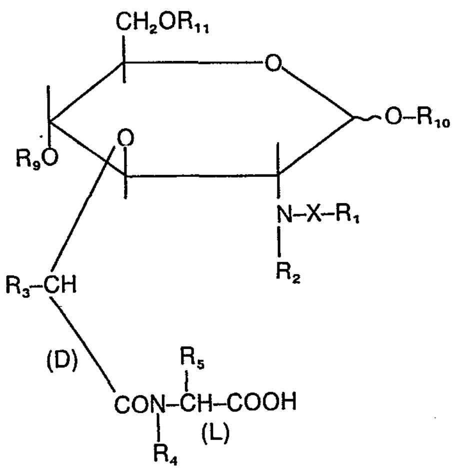

(IX)

où X, $R_1$, $R_2$, $R_3$, $R_4$ et $R_5$ ont la signification donnée ci-dessus, avec la précision que les groupes hydroxy libres qui y sont contenus sont éventuellement protégés avec un groupe protecteur facilement séparable, et $R_9$, $R_{10}$ et $R_{11}$ représentent un hydrogène ou des groupes protecteurs facilement séparables, ou ses dérivés, avec un composé de formule

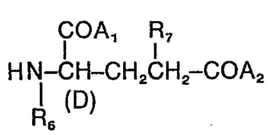

(X)

où $R_6$, $R_7$, $A_1$ et $A_2$ ont la signification donnée ci-dessus, avec la précision que les groupes carboxyle libres présents sont protégés par des groupes protecteurs facilement séparables et on sépare les groupes protecteurs présents, ou

d) on condense un composé de formule

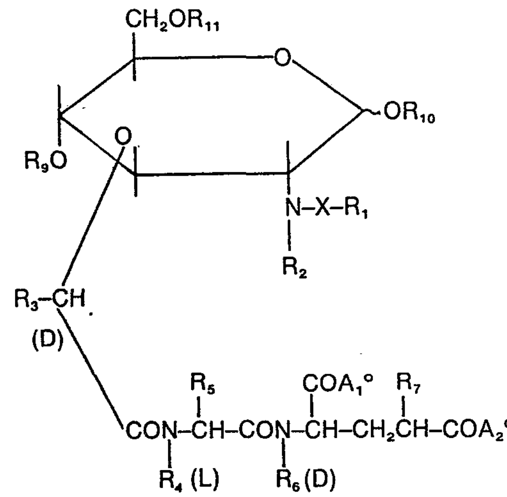

(XI)

où X, $R_1$, $R_2$, $R_3$, $R_4$, $R_5$, $R_6$ et $R_7$ ont la signification donnée ci-dessus, $R_9$, $R_{10}$ et $R_{11}$ représentent un hydrogène ou un groupe protecteur facilement séparable et l'un des radicaux $A_1°$ et $A_2°$ représente un groupe hydroxy activé et l'autre est un hydroxy éthérifié, un amino, un alcoyle inférieur-amino ou un carboxamido-alcoyle inférieur-amino, avec un composé de formule

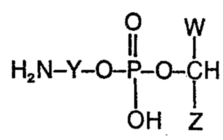

(XII)

où Y, W et Z ont la signification donnée ci-dessus, et on sépare les groupes protecteurs présents, ou

e) on estérifie un composé de formule

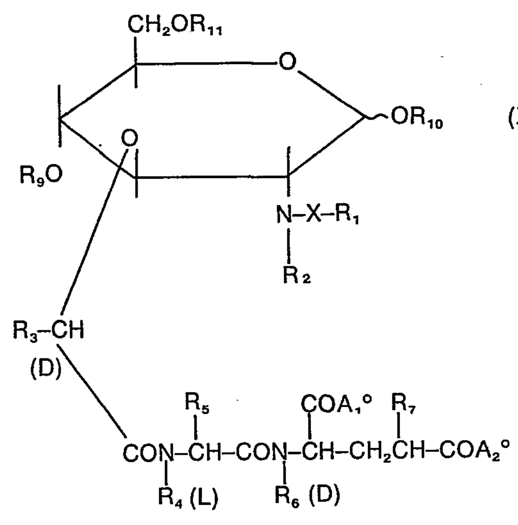

(XIa)

où X, $R_1$, $R_2$, $R_3$, $R_4$, $R_5$, $R_6$ et $R_7$ ont la signification donnée ci-dessus, $R_9$, $R_{10}$ et $R_{11}$ représentent un hydrogène ou un groupe protecteur facilement séparable et l'un des radicaux $A_1°$ et $A_2°$ représente un groupe hydroxy et l'autre est un hydroxy éthérifié, un amino, un alcoyle inférieur-amino ou un carboxamido-alcoyle inférieur-amino, avec un composé de formule

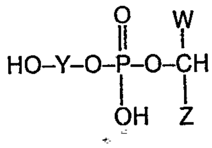

(XIIa)

où Y, W et Z ont la signification donnée ci-dessus, où l'acide XIa ou l'alcool XIIa se présente sous forme réactive, et l'on sépare les groupes protecteurs éventuellement présents, ou

f) dans un composé de formule

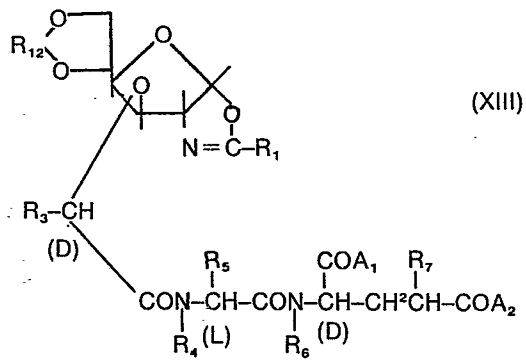

(XIII)

où $R_1$, $R_3$, $R_4$, $R_5$, $R_6$, $R_7$, $A_1$ et $A_2$ ont la signification donnée ci-dessus et $R_{12}$ est un groupe alcoylidène ou cycloalcoylidène, on effectue un clivage acide du noyau oxazoline et du noyau dioxolane, et on sépare les groupes protecteurs éventuellement présents, ou

g) on condense un composé de formule

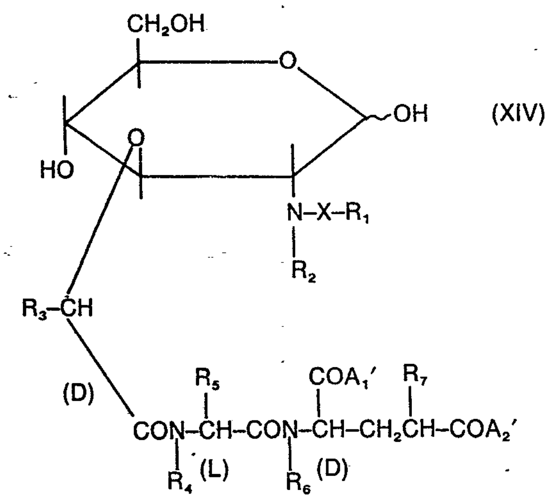

(XIV)

où X, $R_1$, $R_2$, $R_3$, $R_4$, $R_5$, $R_6$ et $R_7$ ont les significations données ci-dessus, où les groupes hydroxy qui y sont présents sont également protégés par des groupes protecteurs facilement séparables, l'un des radicaux $A_1'$ et $A_2'$ représente un radical de formule

$$-T-Y_1-M_1 \qquad (XV)$$

où T a la signification donnée ci-dessus, $Y_1$ représente un alcoylène éventuellement substitué, qui peut être interrompu par un groupe oxycarbonyle ou iminocarbonyle, où les groupes hydroxy qui y sont présents sont éventuellement protégés par des groupes protecteurs facilement séparables, et $M_1$ représente un groupe amino libre, un groupe acide carboxylique ou un dérivé activé de ces deux groupes, et l'autre des radicaux $A_1'$ et $A_2'$ représente un hydroxy éthérifié ou un amino, un alcoyle inférieur-amino ou un carboxamidoalcoyle inférieur-amino, avec un composé de formule

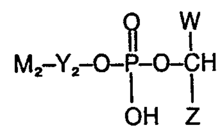

(XVI)

où $M_2$ représente un groupe amino libre ou un de ses dérivés activés si $M_1$ représente un groupe acide carboxylique ou un de ses dérivés activés, ou bien où $M_2$ représente un acide carboxylique ou un de ses dérivés activés si $M_1$ représente un groupe amino libre ou un de ses dérivés activés, $Y_2$ représente un alcoylène éventuellement substitué, qui peut être interrompu par un groupe oxycarbonyle ou iminocarbonyle, avec la précision que les radicaux $Y_1$ et $Y_2$ liés l'un à l'autre par l'intermédiaire d'un groupe iminocarbonyle correspondent au radical Y mentionné ci-dessus, et W et Z ont les significations données ci-dessus, où dans les radicaux $Y_2$, W et Z les groupes hydroxy présents sont éventuellement protégés par des groupes protecteurs facilement séparables, et on sépare les groupes protecteurs éventuellement présents, ou

h) on estérifie un composé de formule

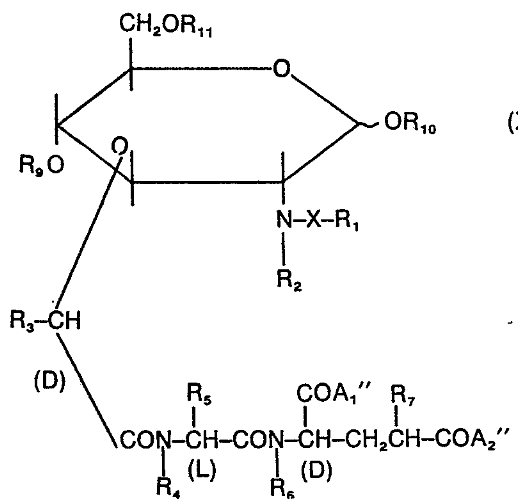

(XVII)

où X, $R_1$, $R_2$, $R_3$, $R_4$, $R_5$, $R_6$ et $R_7$ ont les significations mentionnées ci-dessus, $R_9$, $R_{10}$ et $R_{11}$ représentent des groupes protecteurs facilement séparables, l'un des radicaux $A_1''$ et $A_2''$ représente un radical de formule

$$-T-Y_1-M_3 \qquad (XVIII)$$

où T a la signification donnée ci-dessus, $Y_1$ représente un alcoylène éventuellement substitué qui

peut être interrompu par un groupe oxycarbonyle ou iminocarbonyle, où les groupes hydroxy qui y sont présents sont éventuellement protégés par des groupes protecteurs facilement séparables, et $M_3$ représente un groupe hydroxy ou carboxyle libre qui se présente éventuellement sous forme réactive, et l'autre des radicaux $A_1''$ et $A_2''$ est un hydroxy éthérifié, un amino, un alcoyle inférieur-amino ou un carboxamido-alcoyle inférieur-amino, avec un composé de formule

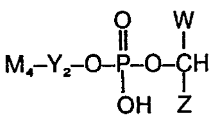

(XIX)

où $M_4$ représente un groupe hydroxy éventuellement présent sous forme réactive si $M_3$ représente un groupe carboxyle éventuellement présent sous forme réactive, ou bien où $M_4$ représente un groupe carboxyle éventuellement présent sous forme réactive si $M_3$ représente un groupe hydroxy éventuellement présent sous forme réactive, $Y_2$ représente un alcoylène éventuellement substitué qui peut être interrompu par un groupe oxycarbonyle ou iminocarbonyle, avec la précision que les radicaux $Y_1$ et $Y_2$ liés l'un à l'autre par l'intermédiaire d'un groupe oxycarbonyle correspondent au radical Y mentionné ci-dessus, et W et Z ont la signification donnée ci-dessus, où dans les radicaux $Y_2$, W et Z sont des groupes hydroxy éventuellement protégés par des groupes protecteurs facilement séparables, et on sépare les groupes protecteurs éventuellement présents, ou

i) on fait réagir un composé de formule

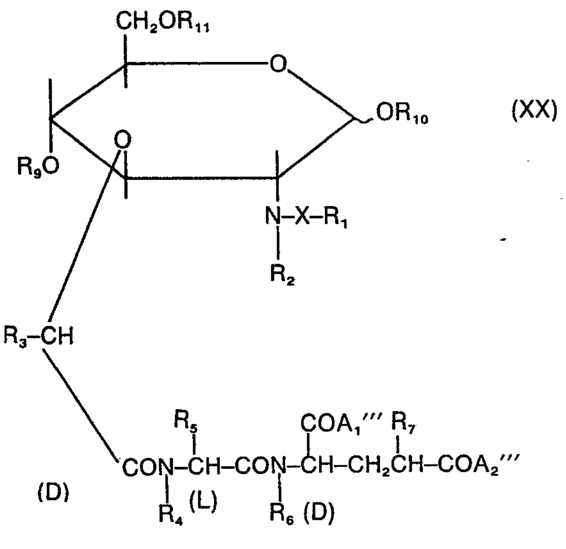

(XX)

où X, $R_1$, $R_2$, $R_3$, $R_4$, $R_5$, $R_6$ et $R_7$ ont les significations données ci-dessus, où les groupes hydroxy qui y sont éventuellement présents sont protégés avec des groupes protecteurs facilement séparables, $R_9$, $R_{10}$ et $R_{11}$ représentent des groupes protecteurs facilement séparables et l'un des radicaux $A_1'''$ et $A_2'''$ représente –T–Y–OH, où Y et T ont les significations mentionnées ci-dessus, où les groupes hydroxy qui y sont éventuellement présents sont protégés avec des groupes protecteurs facilement séparables, et l'autre des radicaux $A_1'''$ et $A_2'''$ représente un hydroxy éthérifié, un amino, un alcoyle inférieur-amino, un carboxamido-alcoyle inférieur-amino, avec un composé donnant le radical de formule

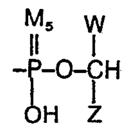

(XXI)

où $=M_5$ représente une paire d'électrons ou un oxo et W et Z ont les significations données ci-dessus, où les groupes hydroxy qui y sont éventuellement présents sont protégés avec des groupes protecteurs facilement séparables si $=M_5$ est une paire d'électrons, on oxyde avec un oxydant faible, et on sépare les groupes protecteurs, ou

k) on fait réagir un composé de formule

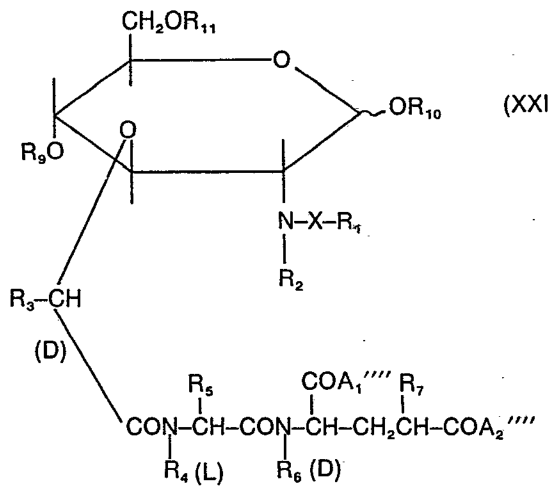

(XXIII)

où X, $R_1$, $R_2$, $R_3$, $R_4$, $R_5$, $R_6$ et $R_7$ ont les significations données ci-dessus, où les groupes hydroxy qui y sont éventuellement présents sont protégés avec des groupes protecteurs facilement séparables et l'un des radicaux $A_1''''$ et $A_2''''$ représente

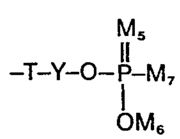

(XXIV)

où T et Y ont la signification donnée ci-dessus, où les groupes hydroxy qui y sont éventuellement présents sont protégés avec des groupes protecteurs facilement séparables, et où $=M_5$ représente une paire d'électrons ou un oxo, $M_6$ représente un groupe protecteur facilement séparable et $M_7$ représente un hydroxy libre ou présent sous forme réactive, et l'autre des radicaux $A_1''''$ et $A_2''''$ représente un hydroxy libre ou éthérifié, un amino, un alcoyle inférieur-amino ou un carboxamido-alcoyle inférieur-amino, avec un composé de formule

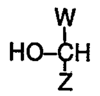 (XXV)

où W et Z ont la signification donnée ci-dessus, où les groupes hydroxy qui y sont éventuellement présents sont protégés avec des groupes protecteurs facilement séparables, si $=M_5$ est une paire d'électrons, on oxyde avec un oxydant faible, et on sépare les groupes protecteurs présents, ou

l) on estérifie un composé de formule

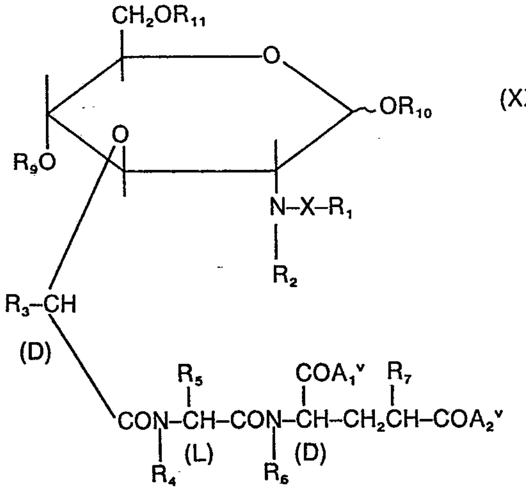 (XXVI)

où X, $R_1$, $R_2$, $R_3$, $R_4$, $R_5$, $R_6$ et $R_7$ ont les significations mentionnées ci-dessus, où les groupes hydroxy qui y sont éventuellement présents sont protégés par des groupes protecteurs facilement séparables, $R_9$, $R_{10}$ et $R_{11}$ représentent des groupes protecteurs facilement séparables et l'un des radicaux $A_1^v$ et $A_2^v$ représente un radical de formule XXVII

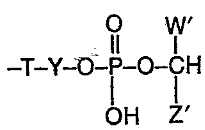 (XXVII)

où T et Y ont les significations données ci-dessus, où les groupes fonctionnels libres présents dans un radical de formule XXVII à l'exception du ou des groupe(s) hydroxy participant à la réaction sont éventuellement protégés par des groupes protecteurs facilement séparables, et W' et Z' ont la signification de W ou Z mentionnée ci-dessus, mais avec la précision qu'au moins un groupe hydroxy dans le radical 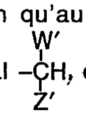, et l'autre des radicaux $A_1^v$ et $A_2^v$ représente un hydroxy libre ou éthérifié, un amino, un alcoyle inférieur-amino ou un carboxamido-alcoyle inférieur-amino, avec un acide carboxylique aliphatique en $C_{12}$ à $C_{90}$ éventuellement insaturé ou un de ses dérivés réactifs, ou on l'éthérifie avec un alcool aliphatique en $C_{10}$ à $C_{22}$ éventuellement insaturé ou un de ses dérivés réactifs et on sépare les groupes protecteurs présents, et, si on le désire, on transforme un composé de formule I obtenu en ses sels, où le préfixe «inférieur» utilisé ci-dessus désigne des radicaux ayant jusqu'à 7 atomes de carbone compris et l'expression «signification mentionnée ci-dessus» employée plus haut donne la signification du radical concerné dans la revendication de produit à laquelle se rapporte cette revendication de procédé.

35. Les composés que l'on peut obtenir selon le procédé de la revendication 34.

**REVENDICATIONS pour l'Etat contractant: AT**

1. Procédé de préparation de muramyl-peptides de formule

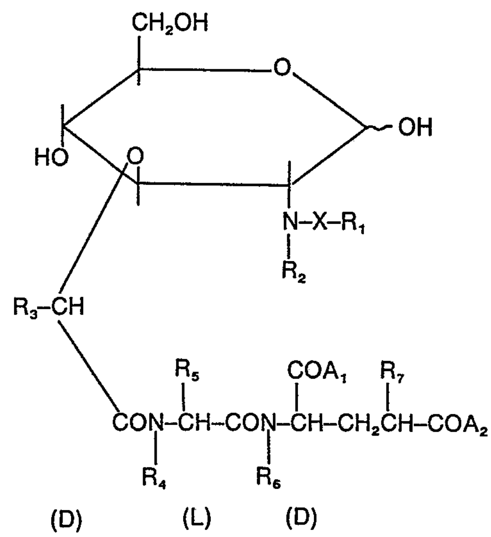 (I)

où X représente un carbonyle ou un carbonyloxy, $R_1$ représente un alcoyle ou un aryle éventuellement substitué, $R_2$, $R_4$ et $R_6$ représentent un hydrogène ou un alcoyle inférieur, $R_3$ représente un hydrogène ou un alcoyle inférieur, $R_5$ représente un hydrogène, un alcoyle inférieur, un hydroxyalcoyle inférieur libre ou fonctionnellement modifié, un mercapto-alcoyle inférieur libre ou fonctionnellement modifié, un aminoalcoyle inférieur éventuellement substitué, un cycloalcoyle, un cycloalcoylalcoyle inférieur, un aryle ou aralcoyle éventuellement substitué, un hétérocyclyle ou hétérocyclyl-alcoyle inférieur contenant de l'azote, ou $R_4$ et $R_5$ représentent également ensemble un alcoylène en $C_3$ à $C_4$, $R_7$ représente un hydrogène ou un carboxyle éventuellement estérifié ou amidé et l'un des radicaux $A_1$ et $A_2$ représente un radical de formule

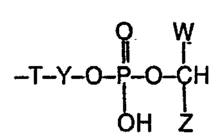 (II)

où T représente NH ou O, Y représente un groupe alcoylène éventuellement substitué, qui peut également être interrompu par un ou deux radicaux oxycarbonyle et/ou iminocarbonyle, W représente un hydrogène et Z un groupe 1,2-dihydroxyéthyle ou 2-hydroxyéthyle, où au moins l'un des groupes

hydroxy est estérifié avec un acide carboxylique aliphatique éventuellement insaturé en $C_{12}$ à $C_{90}$ ou est éthérifié avec un alcool aliphatique éventuellement insaturé en $C_{10}$ à $C_{22}$, ou W et Z représentent chacun un groupe hydroxyméthyle estérifié avec un acide carboxylique aliphatique éventuellement insaturé en $C_{12}$ à $C_{90}$ ou éthérifié avec un alcool aliphatique éventuellement insaturé en $C_{10}$ à $C_{22}$, et l'autre des radicaux $A_1$ et $A_2$ est un hydroxy, un amino, un alcoyle inférieur-amino ou un carboxamido-alcoyle inférieur-amino libre ou éthérifié, ou leurs sels, caractérisé en ce que de façon classique

a) on fait réagir un composé de formule

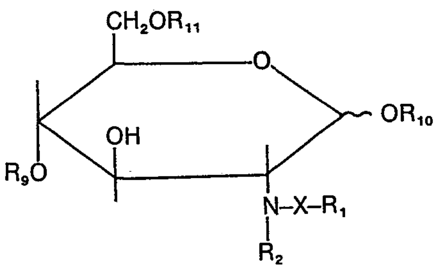 (V)

où X, $R_1$ et $R_2$ ont la signification donnée ci-dessus, où les groupes hydroxy qui y sont éventuellement présents sont protégés avec un groupe protecteur facilement séparable, et $R_9$, $R_{10}$ et $R_{11}$ représentent un groupe protecteur facilement séparable, ou un de ses composés métalliques, avec un composé de formule

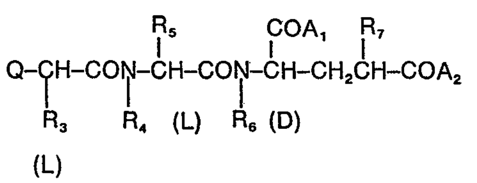 (VI)

oder (D,L)

où Q représente un groupe hydroxy estérifié réactif, $R_3$, $R_4$, $R_5$, $R_6$, $R_7$, $A_1$ et $A_2$ ont la signification donnée ci-dessus et les groupes hydroxy qui y sont éventuellement présents sont protégés par un groupe protecteur facilement séparable, et on sépare les groupes protecteurs présents, ou

b) on condense un composé de formule

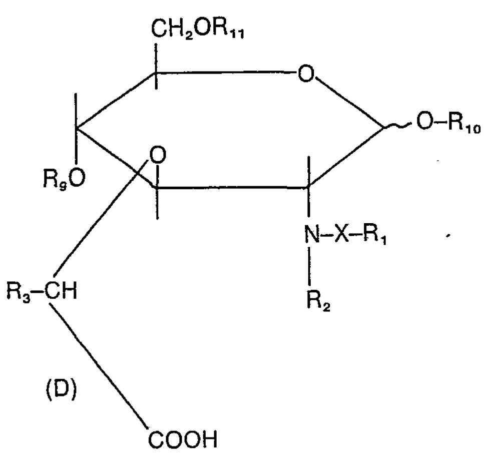 (VII)

où X, $R_1$, $R_2$ et $R_3$ ont la signification mentionnée ci-dessus et $R_9$, $R_{10}$ et $R_{11}$ représentent un hydrogène ou un groupe protecteur facilement séparable, ou un de ses dérivés, avec un composé de formule

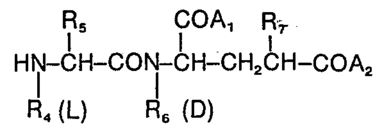 (VIII)

où $R_4$, $R_5$, $R_6$, $R_7$, $A_1$ et $A_2$ ont la signification donnée ci-dessus, avec la précision que les groupes carboxy, et, si on le désire, les groupes hydroxy libres présents dans ces radicaux sont protégés par les groupes protecteurs facilement séparables, ou un de ses dérivés, et on sépare les groupes protecteurs présents, ou

c) on condense un composé de formule

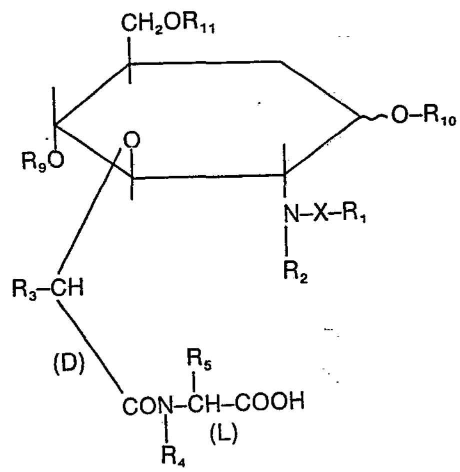 (IX)

où X, $R_1$, $R_2$, $R_3$, $R_4$ et $R_5$ ont la signification donnée ci-dessus, avec la précision que les groupes hydroxy libres qui y sont contenus sont éventuellement protégés avec un groupe protecteur facilement séparable, et $R_9$, $R_{10}$ et $R_{11}$ représentent un hydrogène ou des groupes protecteurs facilement séparables, ou ses dérivés, avec un composé de formule

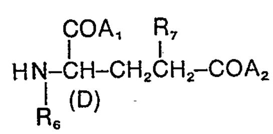 (X)

où $R_6$, $R_7$, $A_1$ et $A_2$ ont la signification donnée ci-dessus, avec la précision que les groupes carboxyle libres présents sont protégés par des groupes protecteurs facilement séparables et on sépare les groupes protecteurs présents, ou

d) on condense un composé de formule

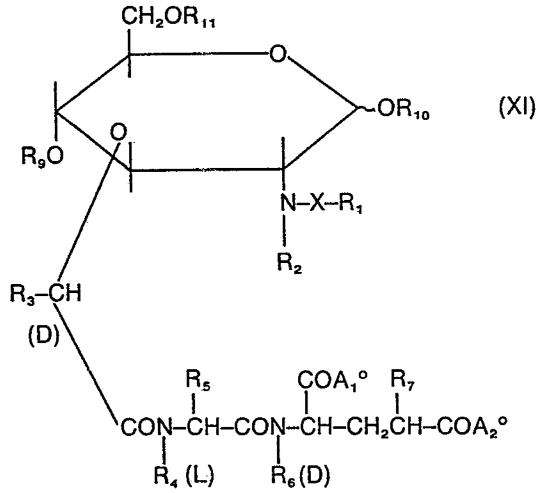

(XI)

où X, $R_1$, $R_2$, $R_3$, $R_4$, $R_5$, $R_6$ et $R_7$ ont la signification donnée ci-dessus, $R_9$, $R_{10}$ et $R_{11}$ représentent un hydrogène ou un groupe protecteur facilement séparable et l'un des radicaux $A_1°$ et $A_2°$ représente un groupe hydroxy activé et l'autre est un hydroxy éthérifié, un amino, un alcoyle inférieur-amino ou un carboxamido-alcoyle inférieur-amino, avec un composé de formule

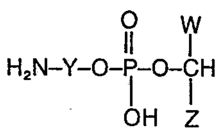

(XII)

où Y, W et Z ont la signification donnée ci-dessus, et on sépare les groupes protecteurs présents ou

e) on estérifie un composé de formule

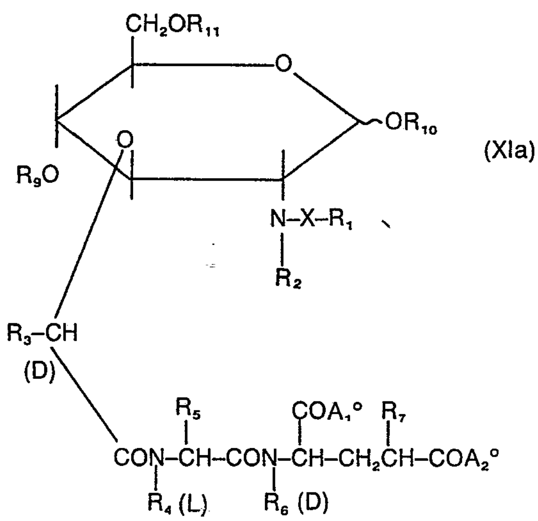

(XIa)

où X, $R_1$, $R_2$, $R_3$, $R_4$, $R_5$, $R_6$ et $R_7$ ont la signification donnée ci-dessus, $R_9$, $R_{10}$ et $R_{11}$ représentent un hydrogène ou un groupe protecteur facilement séparable et l'un des radicaux $A_1°$ et $A_2°$ représente un groupe hydroxy et l'autre est un hydroxy éthérifié, un amino, un alcoyle inférieur-amino ou un carboxamido-alcoyle inférieur-amino, avec un composé de formule

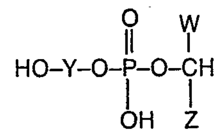

(XIIa)

où Y, W et Z ont la signification donnée ci-dessus, où l'acide XIa ou l'alcool XIIa se présente sous forme réactive, et l'on sépare les groupes protecteurs éventuellement présents, ou

f) dans un composé de formule

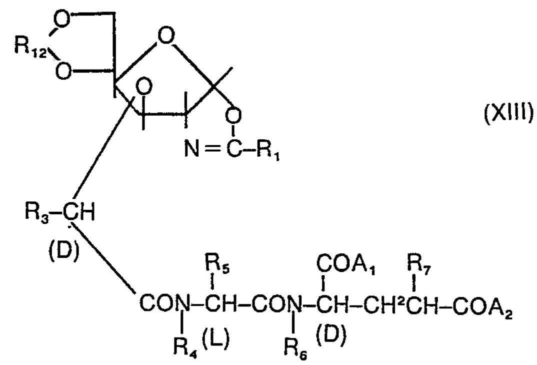

(XIII)

où $R_1$, $R_3$, $R_4$, $R_5$, $R_6$, $R_7$, $A_1$ et $A_2$ ont la signification donnée ci-dessus et $R_{12}$ est un groupe alcoylidène ou cycloalcoylidène, on effectue un clivage acide du noyau oxazoline et du noyau dioxolane, et on sépare les groupes protecteurs eventuellement présents, ou

g) on condense un composé de formule

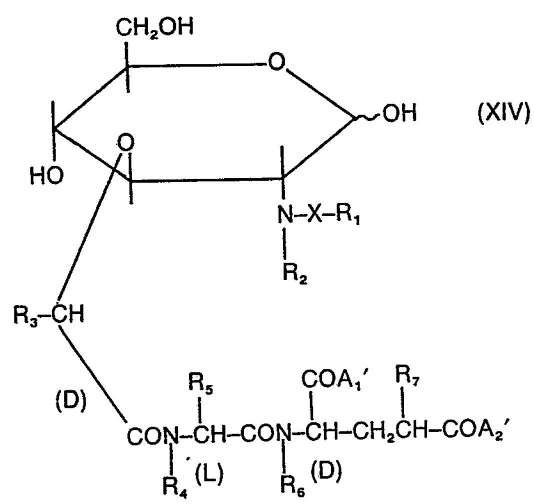

(XIV)

où X, $R_1$, $R_2$, $R_3$, $R_4$, $R_5$, $R_6$ et $R_7$ ont les significations données ci-dessus, où les groupes hydroxy qui y sont présents sont également protégés par des groupes protecteurs facilement séparables, l'un des radicaux $A_1'$ et $A_2'$ reprsésente un radical de formule

$$-T-Y_1-M_1 \qquad (XV)$$

où T a la signification donnée ci-dessus, $Y_1$ représente un alcoylène éventuellement substitué, qui peut être interrompu par un groupe oxycarbonyle ou iminocarbonyle, où les groupes hydroxy qui y

sont présents sont éventuellement protégés par des groupes protecteurs facilement séparables, et $M_1$ représente un groupe amino libre, un groupe acide carboxylique ou un dérivé activé de ces deux groupes, et l'autre des radicaux $A_1'$ et $A_2'$ représente un hydroxy éthérifié ou un amino, un alcoyle inférieur-amino ou un carboxamidoalcoyle inférieur-amino, avec un composé de formule

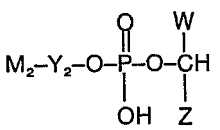

(XVI)

où $M_2$ représente un groupe amino libre ou un de ses dérivés activés si $M_1$ représente un groupe acide carboxylique ou un de ses dérivés activés, ou bien où $M_2$ représente un acide carboxylique ou un de ses dérivés activés si $M_1$ représente un groupe amino libre ou un de ses dérivés activés, $Y_2$ représente un alcoylène éventuellement substitué, qui peut être interrompu par un groupe oxycarbonyle ou iminocarbonyle, avec la précision que les radicaux $Y_1$ et $Y_2$ liés l'un à l'autre par l'intermédiaire d'un groupe iminocarbonyle correspondent au radical Y mentionné ci-dessus, et W et Z ont les significations données ci-dessus, où dans les radicaux $Y_2$, W et Z les groupes hydroxy présents sont éventuellement protégés par des groupes protecteurs facilement séparables, et on sépare les groupes protecteurs éventuellement présents, ou

h) on estérifie un composé de formule

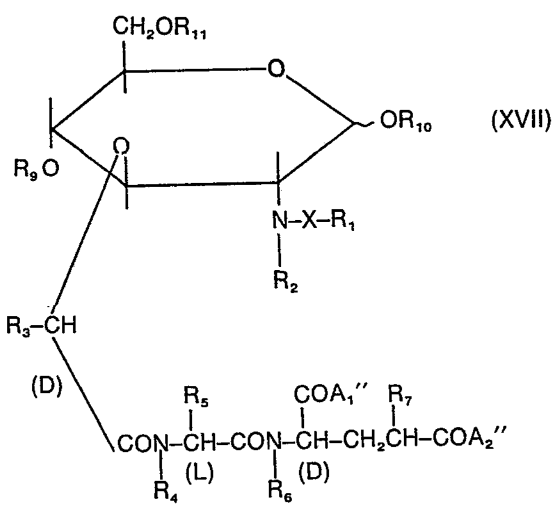

(XVII)

où X, $R_1$, $R_2$, $R_3$, $R_4$, $R_5$, $R_6$ et $R_7$ ont les significations mentionnées ci-dessus, $R_9$, $R_{10}$ et $R_{11}$ représentent des groupes protecteurs facilement séparables, l'un des radicaux $A_1''$ et $A_2''$ représente un radical de formule

$$-T-Y_1-M_3 \qquad (XVIII)$$

où T a la signification donnée ci-dessus, $Y_1$ représente un alcoylène éventuellement substitué qui peut être interrompu par un groupe oxycarbonyle ou iminocarbonyle, où les groupes hydroxy qui y sont présents sont éventuellement protégés par des groupes protecteurs facilement séparables, et $M_3$ représente un groupe hydroxy ou carboxyle libre qui se présente éventuellement sous forme réactive, et l'autre des radicaux $A_1''$ et $A_2''$ est un hydroxy éthérifié, un amino, un alcoyle inférieur-amino ou un carboxamido-alcoyle inférieur-amino, avec un composé de formule

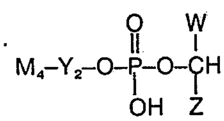

(XIX)

où $M_4$ représente un groupe hydroxy éventuellement présent sous forme réactive si $M_3$ représente un groupe carboxyle éventuellement présent sous forme réactive, ou bien où $M_4$ représente un groupe carboxyle éventuellement présent sous forme réactive si $M_3$ représente un groupe hydroxy éventuellement présent sous forme réactive, $Y_2$ représente un alcoylène éventuellement substitué qui peut être interrompu par un groupe oxycarbonyle ou iminocarbonyle, avec la précision que les radicaux $Y_1$ et $Y_2$ liés l'un à l'autre par l'intermédiaire d'un groupe oxycarbonyle correspondent au radical Y mentionné ci-dessus, et W et Z ont la signification donnée ci-dessus, où dans les radicaux $Y_2$, W et Z sont des groupes hydroxy éventuellement protégés par des groupes protecteurs facilement séparables, et on sépare les groupes protecteurs éventuellement présents, ou

i) on fait réagir un composé de formule

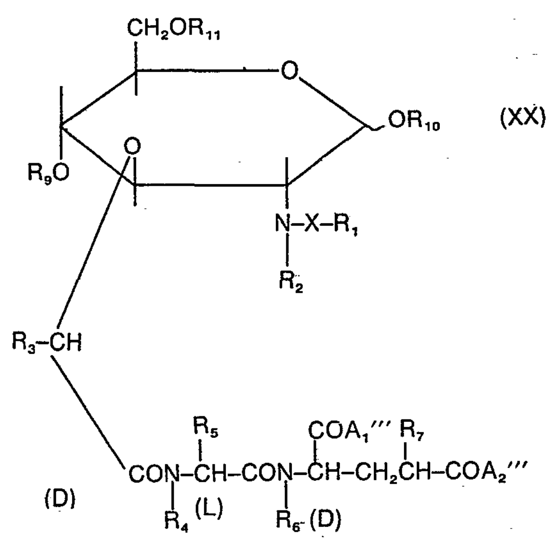

(XX)

où X, $R_1$, $R_2$, $R_3$, $R_4$, $R_5$, $R_6$ et $R_7$ ont les significations données ci-dessus, où les groupes hydroxy qui y sont éventuellement présents sont protégés avec des groupes protecteurs facilement séparables, $R_9$, $R_{10}$ et $R_{11}$ représentent des groupes protecteurs facilement séparables et l'un des radicaux $A_1'''$ et $A_2'''$ représente $-T-Y-OH$, où Y et T ont les significations mentionnées ci-dessus, où

les groupes hydroxy qui y sont éventuellement présents sont protégés avec des groupes protecteurs facilement séparables, et l'autre des radicaux $A_1'''$ et $A_2'''$ représente un hydroxy éthérifié, un amino, un alcoyle inférieur-amino ou un carboxamido-alcoyle inférieur-amino, avec un composé donnant le radical de formule

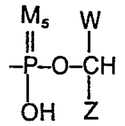 (XXI)

où $=M_5$ représente une paire d'électrons ou un oxo et W et Z ont les significations données ci-dessus, où les groupes hydroxy qui y sont éventuellement présents sont protégés avec des groupes protecteurs facilement séparables si $=M_5$ est une paire d'électrons, on oxyde avec un oxydant faible, et on sépare les groupes protecteurs, ou

k) on fait réagir un composé de formule

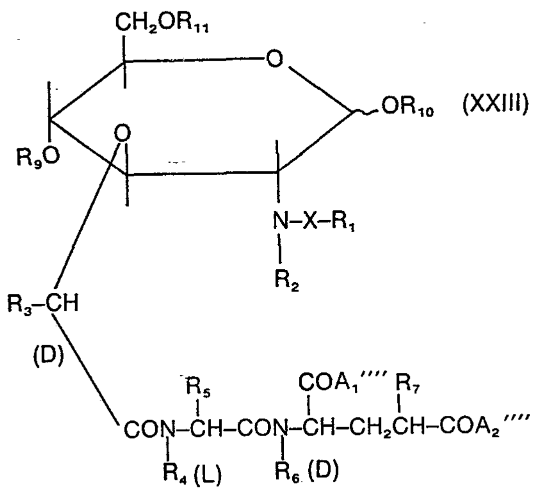

où X, $R_1$, $R_2$, $R_3$, $R_4$, $R_5$, $R_6$ et $R_7$ ont les significations données ci-dessus, où les groupes hydroxy qui y sont éventuellement présents sont protégés avec des groupes protecteurs facilement séparables et l'un des radicaux $A_1''''$ et $A_2''''$ représente

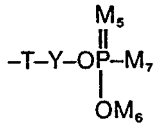 (XXIV)

où T et Y ont la signification donnée ci-dessus, où les groupes hydroxy qui y sont éventuellement présents sont protégés avec des groupes protecteurs facilement séparables, et où $=M_5$ représente une paire d'électrons ou un oxo, $M_6$ représente un groupe protecteur facilement séparable et $M_7$ représente un hydroxy libre ou présent sous forme réactive, et l'autre des radicaux $A_1''''$ et $A_2''''$ représente un hydroxy libre ou éthérifié, un amino, un alcoyle inférieur-amino ou un carboxamido-alcoyle inférieur-amino, avec un composé de formule

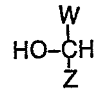 (XXV)

où W et Z ont la signification donnée ci-dessus, où les groupes hydroxy qui y sont éventuellement présents sont protégés avec des groupes protecteurs facilement séparables, si $= M_5$ est une paire d'électrons, on oxyde avec un oxydant faible, et on sépare les groupes protecteurs présents, ou

l) on estérifie un composé de formule

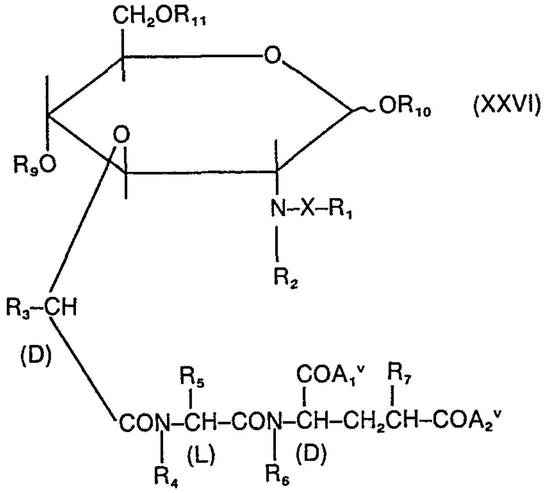

où X, $R_1$, $R_2$, $R_3$, $R_4$, $R_5$, $R_6$ et $R_7$ ont les significations mentionnées ci-dessus, où les groupes hydroxy qui y sont éventuellement présents sont protégés par des groupes protecteurs facilement séparables, $R_9$, $R_{10}$ et $R_{11}$ représentent des groupes protecteurs facilement séparables et l'un des radicaux $A_1^v$ et $A_2^v$ représente un radical de formule XXVII

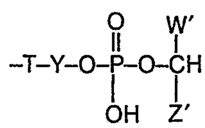 (XXVII)

où T et Y ont les significations données ci-dessus, où les groupes fonctionnels libres présents dans un radical de formule XXVII à l'exception du ou des groupe(s) hydroxy participant à la réaction sont éventuellement protégés par des groupes protecteurs facilement séparables, et W' et Z' ont la signification de W ou Z mentionnée ci-dessus, mais avec la précision qu'au moins un groupe hydroxy dans le radical 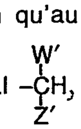, et l'autre des radicaux $A^v_1$ et $A^v_2$ représente un hydroxy libre ou éthérifié, un amino, un alcoyle inférieur-amino ou un carboxamido-alcoyle inférieur-amino, avec un acide carboxylique aliphatique en $C_{12}$ à $C_{90}$ éventuellement insaturé ou un de ses dérivés réactifs, ou on l'éthérifie avec un alcool aliphatique en $C_{10}$ à $C_{22}$ éventuellement insaturé ou un de ses dérivés réactifs et on sépare les groupes protecteurs présents, et, si on le désire, on transforme un

composé de formule I obtenu en ses sels, où le préfixe «inférieur» utilisé ci-dessus désigne des radicaux ayant jusqu'à 7 atomes de carbone compris.

2. Procédé selon la revendication 1, caractérisé en ce qu'on prépare un muramyl-peptide où Y est un radical alcoylène inférieur en $C_2$ à $C_3$ ou ses sels.

3. Procédé selon la revendication 1, caractérisé en ce qu'on prépare des muramyl-peptides où Y représente un radical de formule

$-Y_1-COO-Y_2-$ (IIIa)

$-Y_1-OOC-Y_2-$ (IIIb)

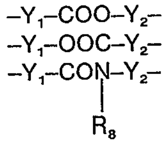 (IIIc)

ou 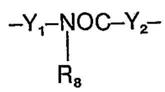 (IIId)

où l'un des radicaux $Y_1$ et $Y_2$ représente un radical alcoylène inférieur éventuellement substitué et l'autre un radical alcoylène inférieur éventuellement substitué, qui peut également être interrompu par un oxycarbonyle ou un N–$R_8$-iminocarbonyle, et $Y_1$ et $Y_2$ présentent ensemble plus de deux atomes de carbone, et $R_8$ est un hydrogène ou un alcoyle inférieur, ou leurs sels.

4. Procédé selon la revendication 1, caractérisé en ce qu'on prépare des muramyl-peptides de formule I où X représente un carbonyle, $R_1$ représente un alcoyle ou aryle éventuellement substitué, $R_2$, $R_3$, $R_4$ et $R_6$ représentent un hydrogène ou un alcoyle inférieur, $R_5$ représente un hydrogène, un alcoyle inférieur, un cycloalcoyle ou un cycloalcoyle-alcoyle inférieur éventuellement substitué par un hydroxy, un alcoxy inférieur, un mercapto ou un alcoyle inférieur-mercapto, ou un halogène, où le radical cycloalcoyle contient de 4 à 6 atomes de carbone, un phényle ou phénylalcoyle inférieur éventuellement substitué, un hétérocyclyle ou hétérocyclyl-alcoyle inférieur contenant un ou deux atomes d'aza, ou bien où $R_4$ et $R_5$ représentent également ensemble un alcoylène en $C_3$ à $C_4$, $R_7$ représente un hydrogène et l'un des radicaux $A_1$ et $A_2$ représente un radical de formule

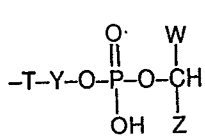 (II)

où T représente NH ou O, Y représente un alcoylène éventuellement substitué, qui peut également être interrompu par un carbonyloxy ou un carbonylimino, W représente un hydrogène et Z représente un groupe 1,2-dihydroxyéthyle ou 2-hydroxyéthyle, où au moins l'un des groupes hydroxy est estérifié ou éthérifié de la manière indiquée dans la revendication 1, et l'autre des radicaux $A_1$ et $A_2$ est un hydroxy, un alcoxy inférieur, un amino, un alcoyle inférieur-amino ou un carboxamido-alcoyle inférieur-amino, ou leurs sels.

5. Procédé selon la revendication 1, caractérisé en ce qu'on prépare des muramyl-peptides de formule I, où X représente un carbonyle, $R_1$ représente un alcoyle inférieur éventuellement substitué par un hydroxy, un alcoxy inférieur ou un halogène, ou un phényle éventuellement substitué par un hydroxy, un alcoxy inférieur, un alcoyle inférieur ou un halogène, $R_2$, $R_4$ et $R_6$ représentent un hydrogène, $R_3$ représente un hydrogène ou un alcoyle inférieur, $R_5$ représente un hydrogène, un alcoyle inférieur en $C_1$ à $C_3$ éventuellement substitué par un hydroxy, un alcoxy inférieur, un mercapto, un alcoyle inférieur-mercapto ou un halogène, un cycloalcoyle ou un cycloalcoyl-alcoyle inférieur, où le radical cycloalcoyle contient de 4 à 6 atomes de carbone et le radical alcoyle inférieur contient de 1 à 3 atomes de carbone, un phényle ou un phényl-alcoyle inférieur avec de 1 à 3 atomes de carbone dans le radical alcoyle inférieur éventuellement substitué par un hydroxy, un alcoxy inférieur ou un halogène, un hétérocyclyle ou hétérocyclyl-alcoyle inférieur avec 5 à 6 chaînons et de 1 à 3 atomes de carbone dans le radical alcoyle inférieur contenant 1 ou 2 atomes d'aza ou bien où $R_4$ et $R_5$ forment également ensemble un alcoylène en $C_3$ à $C_4$, $R_7$ représente un hydrogène, et l'un des radicaux $A_1$ et $A_2$ représente un radical de formule

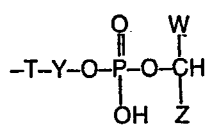 (II)

où T représente HN ou O, Y représente un alcoyle inférieur éventuellement substitué ou un radical de formule

$-Y_1-COO-Y_2-$ (IIIa)

ou $-Y_1-CONH-Y_2-$ (IVc)

où $Y_1$ et $Y_2$ représentent chacun un alcoylène inférieur éventuellement substitué, W représente un hydrogène et Z représente un groupe 1,2-dihydroxyéthyle ou 2-hydroxyéthyle, où au moins l'un des groupes hydroxy est estérifié avec un acide carboxylique aliphatique éventuellement mono- ou bi-insaturé en $C_{16}$ à $C_{22}$ ou avec un acide mycolique naturel ou synthétique, ou éthérifié avec un alcool aliphatique éventuellement mono- ou bi-insaturé en $C_{12}$ à $C_{18}$, et l'autre des radicaux $A_1$ et $A_2$ est un hydroxy, un alcoxy inférieur, un amino, un alcoyle inférieur-amino ou un carboxamido-alcoyle inférieur-amino, ou leurs sels.

6. procédé selon la revendication 1, caractérisé en ce qu'on prépare des muramyl-peptides de formule I, où X représente un carbonyle, $R_1$ représente un alcoyle inférieur en $C_1$ à $C_3$ ou un phényle, $R_2$, $R_4$ et $R_6$ représentent un hydrogène, $R_3$ représente un hydrogène ou un alcoyle inférieur en $C_1$ à $C_3$, $R_5$ représente un hydrogène, un alcoyle inférieur en $C_1$ à $C_3$ éventuellement substitué par

un hydroxy, un méthoxy, un mercapto, un méthylmercapto ou un halogène, un phényle ou phénylméthyle éventuellement substitué par un hydroxy, un méthoxy ou un halogène, un hétérocyclyle ou hétérocyclylméthyle contenant 1 ou 2 atomes d'aza avec 5 chaînons, ou $R_4$ et $R_5$ représentent également ensemble un triméthylène, $R_7$ représente un hydrogène et l'un des radicaux $A_1$ et $A_2$ représente un radical de formule

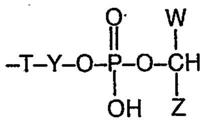

(II)

où T représente HN ou O, Y représente un alcoylène inférieur en $C_2$ à $C_3$ ou un radical de formules (IIIa) ou (IVc), représentées dans la revendication 5, où $Y_1$ et $Y_2$ représentent chacun indépendamment l'un de l'autre un alcoylène inférieur en $C_1$ à $C_3$, qui est non substitué ou substitué par un alcoyle inférieur en $C_1$ à $C_3$ éventuellement substitué par un hydroxy, un alcoxy inférieur, un mercapto ou un alcoyle inférieur-mercapto ou par un phényle ou phényl-alcoyle inférieur éventuellement substitué par un hydroxy, un méthoxy ou un halogène ou par un hétérocyclyle ou hétérocyclylalcoyle inférieur ayant de 5 à 6 chaînons et de 1 à 3 atomes de carbone dans le radical alcoyle inférieur, et contenant 1 ou 2 atomes d'aza, W représente un hydrogène et Z est un groupe 1,2-dihydroxyéthyle ou 2-hydroxyéthyle, où au moins l'un des groupes hydroxy est estérifié avec un acide carboxylique aliphatique en $C_{16}$ à $C_{20}$ éventuellement mono- ou bi-insaturé ou éthérifié avec un alcool aliphatique en $C_{12}$ à $C_{18}$ éventuellement mono- ou bi-insaturé, et l'autre des radicaux $A_1$ et $A_2$ est un hydroxy, un alcoxy inférieur, un amino, un alcoyle inférieur-amino ou un carboxamido-alcoyle inférieur-amino, ou leurs sels.

7. Procédé de préparation de muramyl-peptides de formule I représentée dans la revendication 1, où X représente un carbonyle ou un carbonyloxy, $R_1$ représente un alcoyle inférieur en $C_1$ à $C_3$ ou un phényle, $R_2$, $R_4$ et $R_6$ représentent un hydrogène, $R_3$ représente un hydrogène ou un alcoyle inférieur en $C_1$ à $C_3$, $R_5$ représente un hydrogène ou un alcoyle inférieur, $R_7$ représente un hydrogène, $A_1$ représente un amino, un alcoyle inférieur-amino, un hydroxy ou un alcoxy inférieur et $A_2$ représente un radical de la formule (II) représentée dans la revendication 1, ou T représente NH ou O, Y représente un alcoylène inférieur en $C_2$ à $C_3$ ou un radical de formule

$$-CH_2-CO-NH-CH_2-CH_2-$$

W représente un hydrogène et Z représente un groupe 1,2-dihydroxyéthyle ou 2-hydroxyéthyle, où 1 ou 2 groupes hydroxy sont estérifiés avec des acides alcanecarboxyliques en $C_{16}$ à $C_{22}$ semblables ou différents éventuellement mono- ou bi-insaturés ou éthérifiés avec un alcanol en $C_{12}$ à $C_{18}$ éventuellement mono- ou bi-insaturé, ou bien où deux groupes hydroxy voisins sont cétalisés formellement avec une bis-alcoyle inférieur-cétone avec formation d'un noyau dioxolane ou acétalisés avec un aldéhyde aliphatique non substitué ayant jusqu'à 20 atomes de carbone, ou bien où W et Z représentent chacun un groupe hydroxyméthyle, qui est estérifié avec un acide alcanecarboxylique en $C_{16}$ à $C_{22}$ éventuellement mono- ou bi-insaturé ou éthérifié avec un alcool insaturé en $C_{12}$ à $C_{18}$ éventuellement mono- ou bi-insaturé, ou de leurs sels de manière analogue à l'un des procédés mentionnés dans la revendication 1.

8. Procédé selon la revendication 7, caractérisé en ce qu'on prépare des composés où les significations de $A_1$ et $A_2$ sont échangées, ou leurs sels.

9. Procédé selon la revendication 1, caractérisé en ce qu'on prépare le
N-acétyl-muramyl-L-alanyl-D-isoglutamine-2-(1',2'-dipalmitoyl-sn-glycéro-3'-hydroxyphosphoryloxy)-éthylamide
ou un de ses sels.

10. Procédé selon la revendication 1, caractérisé en ce qu'on prépare le
N-acétyl-desméthylmuramyl-L-alanyl-D-isoglutamine-2-(1',2'-dipalmitoyl-sn-glycéro-3'-hydroxyphosphoryloxy)-éthylamide
ou un de ses sels.

11. Procédé selon la revendication 1, caractérisé en ce qu'on prépare le
2-(1',2'-dipalmitoyl-sn-glycéro-3'-hydroxyphosphoryloxy)-éthylamide
de l'acide
N-acétyl-muramyl-L-alanyl-D-isoglutaminyl-γ-oxy-acétique
ou un de ses sels.

12. Procédé selon la revendication 1, caractérisé en ce qu'on prépare le
N-acétyl-muramyl-L-alanyl-D-isoglutaminyl-L-alanine-2-(1',2'-dipalmitoyl-sn-glycéro-3'-hydroxyphosphoryloxy)-éthylamide
ou un de ses sels.

13. Procédé selon la revendication 1, caractérisé en ce qu'on prépare le
N-acétyl-desméthylmuramyl-L-alanyl-D-isoglutamine-2-(1',2'-dihexadécyl-sn-glycéro-3'-hydroxyphosphoryloxy)-éthylamide
ou un de ses sels.

14. Procédé selon la revendication 1, caractérisé en ce qu'on prépare le
N-acétyl-desméthylmuramyl-L-alanyl-D-isoglutamine-2-(1'-hexadécyl-sn-glycéro-3'-hydroxyphosphoryloxy)-éthylamide
ou un de ses sels.

15. Procédé selon la revendication 1, caractérisé en ce qu'on prépare le
N-acétyl-desméthylmuramyl-L-alanyl-D-isoglutamine-2-(3'-palmitoyl-rac-glycéro-1'-hydroxyphosphoryloxy)-éthylamide
ou un de ses sels.

16. Procédé selon la revendication 1, caractérisé en ce qu'on prépare le
N-acétyl-desméthylmuramyl-L-alanyl-D-isoglutamine-2-(1'-palmitoyl-2'-oléoyl-

sn-glycéro-3′-hydroxyphosphoryloxy)-éthylamide
ou un de ses sels.

17. Procédé selon la revendication 1, caractérisé en ce qu'on prépare le N-acétyl-desméthylmuramyl-L-alanyl-D-isoglutamine-2-(1′-O-palmitoyl-propanediol-3′-O-hydroxyphosphoryloxy)-éthylamide
ou un de ses sels.

18. Procédé selon la revendication 1, caractérisé en ce qu'on prépare le N-acétyl-muramyl-L-α-aminobutyryl-D-isoglutaminyl-L-alanine-2-(1′,2′-dipalmitoyl-sn-glycéro-3′-hydroxyphosphoryloxy)-éthylamide
ou un de ses sels.

19. Procédé selon la revendication 1, caractérisé en ce qu'on prépare le N-acétyl-muramyl-L-α-aminobutyryl-D-isoglutaminyl-L-alanine-2-(1′,2′-dipalmitoyl-rac-glycéro-3′-hydroxyphosphoryloxy)-éthylamide
ou un de ses sels.

20. Procédé selon la revendication 1, caractérisé en ce qu'on prépare le N-benzoyl-muramyl-L-α-aminobutyryl-D-isoglutaminyl-L-alanine-2-(1′,2′-dipalmitoyl-sn-glycéro-3′-hydroxyphosphoryloxy)-éthylamide
ou un de ses sels.

21. Procédé selon la revendication 1, caractérisé en ce qu'on prépare le N-propionyl-desméthylmuramyl-L-alanyl-D-isoglutaminyl-L-alanine-2-(1′,2′-dipalmitoyl-sn-glycéro-3′-hydroxyphosphoryloxy)-éthylamide
ou un de ses sels.

22. Procédé selon la revendication 1, caractérisé en ce qu'on prépare le N-acétyl-muramyl-L-valyl-D-isoglutaminyl-L-alanine-2-(1′,2′-dipalmitoyl-sn-glycéro-3′-hydroxyphosphoryloxy)-éthylamide
ou un de ses sels.

23. Procédé selon la revendication 1, caractérisé en ce qu'on prépare le N-benzoyl-desméthylmuramyl-L-α-aminobutyryl-D-isoglutaminyl-L-alanine-2-(1′,2′-dipalmitoyl-sn-glycéro-3′-hydroxyphosphoryloxy)-éthylamide
ou un de ses sels.

24. Procédé selon la revendication 1, caractérisé en ce qu'on prépare le N-acétyl-desméthylmuramyl-L-alanyl-D-glutamyl-($C_\alpha$)-glycinamide-($C_\gamma$)-L-alanine-2-(1′,2′-dipalmitoyl-sn-glycéro-3′-hydroxyphosphoryloxy)-éthylamide
ou un de ses sels.

25. Procédé selon la revendication 1, caractérisé en ce qu'on prépare le N-acétyl-muramyl-L-alanyl-D-isoglutaminyl-L-alanine-2-(3′-palmitoyl-rac-glycéro-1′-hydroxyphosphoryloxy)-éthylamide
ou un de ses sels.

26. Procédé selon la revendication 1, caractérisé en ce qu'on prépare le N-acétyl-muramyl-L-alanyl-D-isoglutaminyl-L-alanine-2-(1′,2′-hexadécylidène-sn-glycéro-3′-hydroxyphosphoryloxy)-éthylamide
ou un de ses sels.

27. Procédé selon la revendication 1, caractérisé en ce qu'on prépare le N-acétyl-desméthylmuramyl-L-alanyl-D-isoglutaminyl-L-alanine-2-(1′,2′-hexadécyclidène-sn-glycéro-3′-hydroxyphosphoryloxy)-éthylamide
ou un de ses sels.

28. Procédé selon la revendication 1, caractérisé en ce qu'on prépare un sel pharmaceutiquement acceptable d'un composé selon l'une des revendications 1–27.

29. Procédé selon la revendication 28, caractérisé en ce qu'on prépare un sel de sodium.

30. Procédé selon l'une des revendications 1–29, caractérisé en ce que dans un composé de formule I où les groupes hydroxyle en position 1, 4 et/ou 6 de la partie sucrée sont protégés par des groupes protecteurs, on sépare ces groupes protecteurs.

31. Procédé selon l'une des revendications 1–29, caractérisé en ce que dans un composé de formule I où un ou plusieurs groupes carboxyle sont protégés par des groupes protecteurs, on sépare ces groupes protecteurs.

32. Procédé selon l'une des revendications 1–29, caractérisé en ce qu'on traite avec un acide pour séparer les groupes protecteurs.

33. Procédé selon l'une des revendications 1–29, caractérisé en ce qu'on utilise comme composés avec un ou plusieurs groupes hydroxy estérifiés réactifs des composés où le ou les groupes hydroxy est (sont) estérifié(s) avec un acide inorganique fort ou un acide sulfonique.

34. Procédé selon l'une des revendications 1–29, caractérisé en ce qu'on utilise comme composés avec un ou plusieurs groupes hydroxy estérifiés réactifs des composés où le(s) groupe(s) hydroxy est (sont) estérifié(s) avec un acide halohydrique.

35. Procédé selon l'une des revendications 1–29, caractérisé en ce qu'on utilise comme composés avec un ou plusieurs groupes protecteurs d'hydroxy des composés où le(s) groupe(s) hydroxy est (sont) protégé(s) avec des radicaux acyle, des radicaux aroyle, ou des radicaux provenant de dérivés d'acide carbonique ou avec des radicaux alcoyle ramifiés en position alpha, ou avec des radicaux alpha-mono-, -di- ou -triarylalcoyle inférieur ou avec des radicaux formateurs d'acétal.

36. Procédé selon l'une des revendications 1–29, caractérisé en ce qu'on utilise comme composés avec un ou plusieurs groupes protecteurs d'hydroxy des composés où le(s) groupe(s) hydroxy est (sont) protégé(s) avec des radicaux alcanoyle inférieur, benzoyle, benzyloxycarbonyle, alcoxy inférieur-carbonyle, tert-butyle ou avec des

radicaux benzyle, triphénylméthyle ou tétrahydropyranyle éventuellement substitués.

37. Procédé selon l'une des revendications 1–29, caractérisé en ce qu'on utilise comme composés avec un ou plusieurs groupes protecteurs de carboxy des composés où le(s) groupe(s) carboxy est (sont) protégé(s) avec des radicaux tert-butyle, benzyle ou avec des radicaux triphénylméthyle ou benzhydryle éventuellement substitués par un halogène ou un alcoxy inférieur.

38. Procédé selon l'une des revendications 1–29, caractérisé en ce qu'on utilise comme composés avec un ou plusieurs groupes acide carboxylique activés des composés où le(s) groupe(s) acide carboxylique se présente(nt) sous forme d'anhydride, d'azide, d'amide activé ou sous forme d'ester activé.

39. Procédé selon la revendication 38, caractérisé en ce qu'on utilise comme anhydrides d'acide de tels corps formés avec des alcoyle inférieur-esters de l'acide carbonique, comme amides d'acide des imidazolides ou isooxazolides ou comme esters activés des cyano- ou carboxyméthylesters, acétylaminoéthylthicester, p-nitro- ou 2,4,5-trichloro-phénylester,
N-hydroxy-succinimide-,
N-hydroxy-phtalimide- ou
N-hydroxy-pipéridine-ester,
8-hydroxy-quinoléine-ester,
méthoxyéthoxy-thioester ou par réaction avec du carbodiimide avec addition de N-hydroxy-succinimide ou d'un 1-hydroxybenzotriazole ou 3-hydroxy-4-oxo-3,4-dihydro-benzo-[d]-1,2,-3-triazine.

40. Procédé mixte de préparation d'une préparation pharmaceutique, caractérisé en ce qu'on mélange un composé obtenu selon l'une des revendications 1 à 39 avec un support pharmaceutique.